(19) Europäisches Patentamt
European Patent Office
Office européen des brevets

(11) Numéro de publication: **0 053 540**
**B1**

(12) **FASCICULE DE BREVET EUROPEEN**

(45) Date de publication du fascicule du brevet: **14.08.85**

(51) Int. Cl.[4]: **C 07 D 501/24**

(21) Numéro de dépôt: **81401827.1**

(22) Date de dépôt: **19.11.81**

(54) ***Nouveaux dérivés de la céphalosporine et leur préparation.***

(30) Priorité: **20.11.80 FR 8024638**

(43) Date de publication de la demande: **09.06.82 Bulletin 82/23**

(45) Mention de la délivrance du brevet: **14.08.85 Bulletin 85/33**

(84) Etats contractants désignés: **AT BE CH DE FR GB IT LI LU NL SE**

(56) Documents cités:
**FR - A - 2 081 451**
**FR - A - 2 137 899**
**FR - A - 2 457 296**

(73) Titulaire: **RHONE-POULENC SANTE, Les Miroirs 18 Avenue d'Alsace, F-92400 Courbevoie Cedex (FR)**

(72) Inventeur: **Berger, Christian, 26 A rue Paul Rivet, F-92350 Le Plessis-Robinson (FR)**
Inventeur: **Farge, Daniel, 30 rue des Pins Sylvestres, F-94320 Thiais (FR)**
Inventeur: **Moutonnier, Claude, 3 rue Auguste Rodin, F-92350 Le Plessis-Robinson (FR)**
Inventeur: **Wolff, Gérard, 7 rue Jeanne d'Arc, F-94320 Thiais (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 053 540 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne de nouveaux dérivés de vinyl-3 céphalosporines de formule générale:

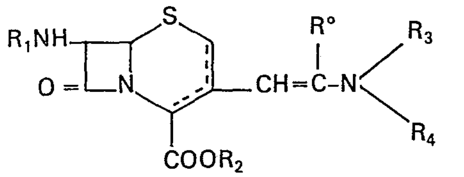

(I)

et leur préparation.

Le produit de formule générale (I) se présente sous forme bicyclooctène-2 ou -3 (selon la nomenclature des Chemical Abstracts), les substituants R° et $-NR_3R_4$ de la chaîne en position -3 peuvent se trouver en position cis ou trans du bicyclooctène, et les symboles $R_3$ et $R_4$, qui sont identiques ou différents, représentent des radicaux alcoyle (éventuellement substitués par un radical alcoyloxy ou dialcoylamino) ou phényle ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, et

a) le symbole $R_1$ représente un radical de formule générale:

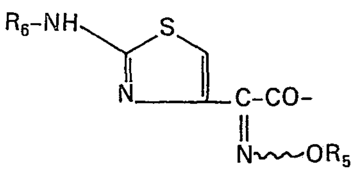

(II)

[(dans laquelle $R_5$ est un atome d'hydrogène, un radical alcoyle, vinyle, cyanométhyle, un groupement protecteur d'oxime tel que trityle, tétrahydropyranyle ou méthoxy-2 propyle-2 ou un radical de formule générale:

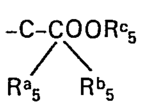

(II')

(dans laquelle $R^a_5$ et $R^b_5$, qui sont identiques ou différents, sont des atomes d'hydrogène, des radicaux alcoyle ou forment ensemble un radical alcoylène contenant 2 ou 3 atomes de carbone et $R^c_5$ est un radical protecteur d'acide par exemple tel que cité ci-après pour $R_2$) et $R_6$ est un radical protecteur d'amine tel que t.butoxycarbonyle, trityle, benzyle, dibenzyle, benzyloxycarbonyle, p.nitrobenzyloxycarbonyle ou p.méthoxybenzyloxycarbonyle, ou le radical de formule générale (V) défini ci-après)],

un radical benzhydryle, trityle,

un radical acyle de formule générale:

$R_7-CO-$ (III)

[(dans laquelle $R_7$ est un radical alcoyle (substitué par un radical phényle ou phénoxy))]

un radical de formule générale:

$R_8O\,Co-$ (IV)

[(dans laquelle $R_8$ est un radical alcoyle ramifié non substitué ou alcoyle ramifié ou droit portant 1 ou plusieurs substituants [choisis parmi phényle et phényle substitué (par un radical alcoyloxy, nitro ou phényle)], ou vinyle)],

un radical de formule générale:

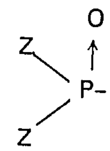

(V)

(dans laquelle les symboles Z représentent des radicaux phényle ou des radicaux OZ' dans lesquels les symboles Z' représentent des radicaux alcoyle, trichloro-2,2,2 éthyle, phényle ou benzyle, ces 2 derniers radicaux pouvant être substitués par un atome d'halogène ou un radical alcoyle, alcoyloxy ou nitro ou bien les symboles Z' des 2 substituants Z forment ensemble un radical alcoylène contenant 2 ou 3 atomes de carbone), ou bien $R_1NH$ est remplacé par un radical méthylène-amino dans lequel le radical méthylène est substitué par un groupement dialcoylamino ou aryle (lui-même éventuellement substitué par un ou plusieurs radicaux méthoxy ou nitro), le symbole $R_2$ représente un radical protecteur d'acide tel que méthoxyméthyle, t.butyle, benzhydryle, benzyle, nitrobenzyle ou p.méthoxybenzyle, et le symbole R° est un radical d'un premier groupe constitué par un radical phényle éventuellement substitué (par un radical alcoyle, trifluorométhyle, dialcoylaminométhyle, alcoyloxy, alcoylthio, dialcoylamino ou par un atome d'halogène tel que le fluor ou le chlore ou un radical hétérocyclyle à 5 ou 6 chaînons contenant un seul hétéroatome (tel que pyridyle-2 ou -3, thiényle-2 ou -3 ou furyle-2 ou -3) éventuellement substitué par un radical alcoyle, alcoyloxy ou diméthylaminométhyle, ou bien un radical d'un second groupe (qui sera désigné comme «radical $-CH\,R_9R_{10}$»), constitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons tel que pyridyle-2 ou -3, thiényle-2 ou -3 ou furyle-2 ou -3, un radical cycloalcoyle contenant 3 à 6 chaînons ou un radical dans lequel $R_9$ et $R_{10}$ forment, avec l'atome de carbone auquel ils sont rattachés, un hétérocycle à 5 ou 6 chaînons contenant un atome d'oxygène ou de soufre, ou bien

b) le symbole $R_1$ représente un radical aminoprotecteur tel que trityle, silyle, un radical alcoyloxycarbonyle dont la partie alcoyle contient 1 à 5 atomes de carbone, de préférence ramifié sur

l'atome de carbone en α de l'atome d'oxygène (et/ou éventuellement substitué par un ou plusieurs radicaux phényle ou furyle, par exemple α-phénylalcoyloxycarbonyle, diphénylméthoxycarbonyle, α-furylalcoyloxycarbonyle), un radical (biphénylyl-4)-2 propyloxycarbonyle, ou un radical de formule générale:

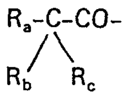

(VI)

[dans laquelle
$R_a$ est un radical phényle, thiényle, furyle, cyclohexadiényle ou cyclohexényle éventuellement substitués, $R_b$ est un atome d'hydrogène et $R_c$ est un atome d'hydrogène, un radical hydroxy éventuellement protégé, un radical amino protégé ou un radical sulfo éventuellement protégé, ou $R_a$ est un radical phénoxy éventuellement substitué ou pyridylthio et $R_b$ et $R_c$ sont des atomes d'hydrogène, ou bien $R_a$ est un radical phényle, thiényle, furyle et $R_b$ et $R_c$ forment ensemble un radical alcoyloxyimino, cycloalcoyloxyimino ou phénylalcoyloxyimino de forme syn, les radicaux phényle et phénoxy peuvent être substitués par des groupes hydroxy, alcoyloxy, acyloxy, benzoyloxy, 4-carbamoyloxy, amino ou alcoylamino protégés, dialcoylamino, alcoylsulfonylamino, aminométhyle, t.butoxycarbonylaminométhyle ou par un atome d'halogène, les radicaux thiényle, furyle, cyclohexadiényle et cyclohexényle peuvent être substitués par des radicaux aminométhyle protégés, notamment en position -5 sur les radicaux thiényle et furyle et en position -2 ou -3 sur les radicaux cylohexadiényle et cyclohexényle, les radicaux hydroxy, amino et sulfo que représente $R_c$ peuvent être protégés par tout groupement connu dont la mise en place et l'élimination n'altèrent pas le reste de la molécule. Les groupements protecteurs d'hydroxy sont notamment ceux cités dans la demande de brevet français 2 321 294, exceptés les groupements halogénés ou le groupement stannyle];
le symbole $R_2$ représente un radical protecteur tel qu'un groupe aryle polysubstitué par des radicaux aliphatiques ou aromatiques comme alcoyle ou phényle, un groupe hétérocyclique aromatique contenant de l'oxygène ou du soufre, un groupe méthyle substitué comme t.alcoyle (contenant 4 ou 5 atomes de carbone), diphénylméthyle éventuellement substitué (par exemple 4,4'-diméthoxydiphénylméthyle), alcoyloxyphénylalcoyle (par exemple méthoxybenzyle, diméthoxybenzyle), nitrobenzyle, 4,5-diméthoxy 2-nitrobenzyle, furfuryle, ou un groupe 2-oxa (ou 2-thia) cycloalcoyle ou 2-oxa (ou 2-thia) cycloalcényle à 5 à 7 chaînons, ou un groupe nitrophényle ou 2,4-dinitrophényle, ou encore un radical silyle subsitué (par exemple par des radicaux alcoyloxy, cycloalcoyle, phényle ou phénylalcoyle, notamment trialcoylsilyle) et le symbole R° représente
un radical alcoyle (contenant 1 à 7 atomes de carbone),
un radical cycloalcoyle (contenant 3 à 7 atomes de carbone),
un radical cycloalcoyl-alcoyle (contenant 4 à 7 atomes de carbone),
un radical aryle mono- ou bicyclique comme phényle ou naphtyle,
un radical arylalcoyle comme phénylalcoyle,
un radical hétérocyclyle ou hétérocyclylalcoyle pour lesquels l'hétérocycle et de préférence aromatique à 5 ou 6 chaînons et contient 1 à 4 atomes d'azote et/ou un atome d'oxygène ou de soufre, comme pyrrolyle, pyridyle-2 (ou -3), thiényle, furyle, imidazolyle, tétrazolyle, thiazolyle ou isothiazolyle, tous ces radicaux R° pouvant être éventuellement substitués par des radicaux alcoyle ou alcoyloxy.

Il est entendu que, sauf mention spéciale, les portions ou radicaux alcoyles ou acyles cités ci-dessus ou qui seront cités ci-après sont droits ou ramifiés et contiennent 1 à 4 atomes de carbone.

Dans la formule générale (I) les radicaux R° et $-NR_3R_4$ peuvent se trouver respectivement en position cis et trans ou inversement.

Dans ce qui suit la stéréoisomérie trans sera désignée par E et la stéréoisomérie cis sera désignée par Z.

Il est entendu que les mélanges de isomères bicyclooctène-2 et -3 et/ou cis et trans entrent dans le cadre de la présente invention.

Par ailleurs, il est également entendu que le groupement $-OR_5$ du radical de formule générale (II) peut se trouver dans l'une des positions syn ou anti et que ces isomères et leurs mélanges entrent aussi dans le cadre de la présente invention.

La forme syn peut être représentée par la formule

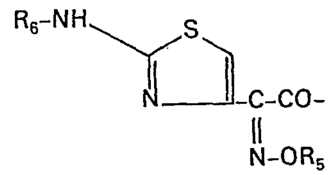

(II a)

La forme anti peut être représentée par la formule

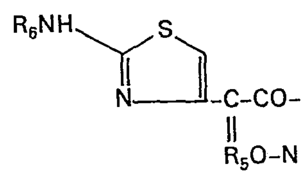

(II b)

Parmi les significations de $R_1$ définies ci-dessus en (a) peuvent être citées notamment:
méthoxyimino-2 (tritylamino-2 thiazolyl-4)-2 acétyle, méthoxyimino-2 (t.butoxycarbonylamino-2 thiazolyl-4)-2 acétyle, trityloxyimino-2 (tritylamino-2 thiazolyl-4)-2 acétyle, tétrahydropyranyloxyimino-2 (tritylamino-2 thiazolyl-4)-2 acétyle, t.butoxycarbonylméthoxyimino-2 (tritylamino-2 thiazolyl-4)-2 acétyle, trityle, phénylacétyle, phénoxyacétyle, t.butoxycarbonyle, benzyloxycarbonyle, p.méthoxybenzyloxycarbonyle, p.nitrobenzyloxycarbonyle, diphénylméthoxycarbonyle, (biphénylyl-4)-2 isopropyloxycarbonyle, vinyloxycarbonyle, diphénylphosphinoyle, diméthoxyphosphoryle, diéthoxyphosphoryle, diphénoxyphosphoryle.

Comme exemples de radicaux méthylèneamino on peut citer:
diméthylaminomtéhylèneamino
diméthoxy-3,4 benzylidèneamino
nitro-4 benzylidèneamino

1. Selon l'invention, les produits de formule générale (I) pour lesquels $R_1$, $R_2$, $R_3$ et $R_4$ ont les définitions données précédemment, et R° est un radical du premier groupe défini précédemment en a) ou représente un radical défini en b) choisi parmi les radicaux aryle ou hétérocyclyle, peuvent être obtenus par action d'un réactif, éventuellement préparé in situ, de formule générale:

$$R°\text{-}C(NR_3R_4)_3 \qquad (VII)$$

dans laquelle R°, $R_3$ et $R_4$ sont définis comme ci-dessus, sur un déribé de céphalosporine de formule générale:

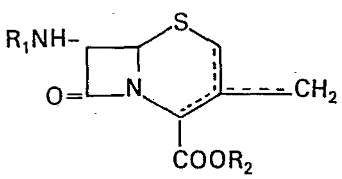

(VIII)

dans laquelle, $R_1$ et $R_2$ étant définis comme précédemment, le dérivé se présente sous forme méthyl-3 bicyclooctène-2 ou -3 ou méthylène-3 bicyclooctane.

On opère généralement dans un solvant organique tel que le diméthylformamide, l'hexaméthylphosphorotriamide, les glymes, le dioxanne, le diméthylacétamide, l'acétate d'éthyle, ou dans un mélange de tels solvants à une température comprise entre 20°C et la température de reflux du mélange réactionnel.

2. Selon l'invention, les produits de formule générale (I) dans laquelle $R_1$, $R_2$, $R_3$ et $R_4$ sont définis comme précédemment et R° est un radical du second groupe défini précédemment en a) ou représente un radical défini en b) choisi parmi les radicaux alcoyle, cycloalcoyle, cycloalcoylalcoyle, arylalcoyle ou hétérocyclylalcoyle, peuvent être obtenus par action d'un réactif, éventuellement préparé in situ, de formule générale:

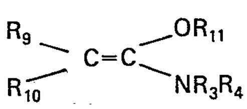

(IX a)

(dans laquelle $R_9$ et $R_{10}$ sont tels que $R_9R_{10}CH-$ représente le radical R° défini ci-dessus, $R_3$ et $R_4$ sont définis comme précédemment et $R_{11}$ est un radical alcoyle contenant 1 à 5 atomes de carbone), sur un dérivé de céphalosporine de formule générale (VIII).

La réaction s'effectue généralement dans les conditions décrites précédemment pour la préparation des produits de formule générale (I) à partir des produits (VII) et (VIII).

Les réactifs de formule générale (VII) peuvent être obtenus selon ou par application de la méthode décrite par C.F. Hobbs et coll., J. Org. Chem. 36, 2885 (1971).

Les réactifs de formule générale (IXa) peuvent être obtenus selon ou par application de la méthode décrite par H. Bredereck et coll., Chem. Ber., 97 3076 (1964) et Chem. Ber., 97 3081 (1964).

Les dérivés de la céphalosporine de formule générale (VIII) dans laquelle $R_1$ représente un radical de formule générale (II) peuvent être préparés à partir des produits de formule générale:

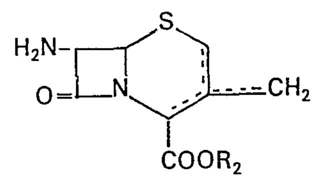

(X)

[dans laquelle, $R_2$ étant défini comme précédemment, la position de la double liaison est définie comme pour le produit de formule générale (VIII)] par action d'un acide de formule générale

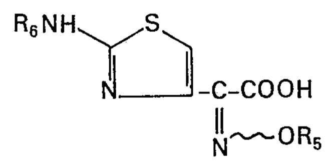

(XI)

[dans laquelle $R_5$ et $R_6$ sont définis comme précédemment à l'exception pour $R_5$ de représenter l'atome d'hydrogène] ou d'un dérivé réactif de cet acide suivie le cas échéant de l'élimination du radical protecteur de l'oxime. Il est entendu que l'acide de formule générale (XI) sous forme syn, anti ou leurs mélanges, conduit respectivement aux produits de formule générale (VIII) de forme syn, anti ou leurs mélanges.

Généralement on effectue la condensation du produit de formule générale (XI), dont la fonction acide est libre, sur l'amino-7 céphalosporine de formule générale (X) dans un solvant organique tel que le diméthylformamide, l'acétonitrile, le tétrahydrofuranne, le chlorure de méthylène ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléeine, à une température comprise entre −20 et 40°C.

Lorsque l'on utilise un dérivé réactif de l'acide de formule générale (XI), il est possible de mettre en œuvre l'anhydride, un anhydride mixte ou un ester réactif de formule générale

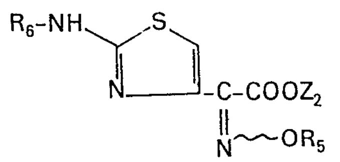

(XII)

[(dans laquelle, $R_5$ et $R_6$ étant définis comme précédemment, $Z_2$ représente un radical succinimido, benzotriazolyle-1,nitro-4 phényle, dinitro-2,4 phényle, pentachlorophényle ou phtalimido)] ou bien un halogénure d'acide, par exemple le chlorure d'acide.

Lorsque l'on met en œuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple l'acétone), ainsi que des mélanges de tels solvants [en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine)] ou dans un milieu hydroorganique en présence d'un agent alcalin de condensation tel que le bicarbonate de sodium, et l'on opère à une température comprise entre −40 et +40°C.

Lorsque l'on met en œuvre un ester réactif de formule générale (XII) on opère généralement en présence d'une trialcoylamine (par exemple triéthylamine) dans un solvant organique tel que le diméthylformamide, à une température comprise entre 0 et 40°C.

L'introduction des groupements protecteurs $R_1$ et/ou $R_2$ des produits de formule générale (VIII), pour lesquels $R_1$ et $R_2$ sont définis comme précédemment en a) [à l'exception pour $R_1$ de représenter un radical de formule générale (II)], et des produits de formule générale (X), pour lesquels $R_2$ est défini comme précédemment en a), peut être effectuée sur une céphalosporine respectivement de formule générale (X) ou de formule générale:

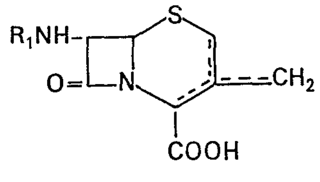

(XIII)

ou

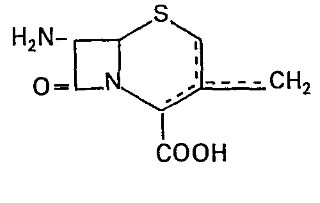

(XIV)

Par application des méthodes décrites dans les références suivantes:

- lorsque $R_1$ est un radical trityle: par analogie avec la méthode décrite par J.C. Sheehan et coll., J. Amer. Chem. Soc., 84, 2983 (1962).
- lorsque $R_1$ est phénylacétyle ou phénoxyacétyle: selon E.H. Flynn, Cephalosporins and Penincillins, Ac. Press (1972).
- lorsque $R_1$ est un radical t.butoxycarbonyle: selon L. Moroder et coll., Hoppe Seyler's Z. Physiol. Chem. 357, 1651 (1976),
- lorsque $R_1$ est benzyloxycarbonyle, p.méthoxybenzyloxycarbonyle, p.nitrobenzyloxycarbonyle ou vinyloxycarbonyle: par action d'un chloroformiate en milieu hydroorganique en présence d'un bicarbonate alcalin, ou selon le brevet belge 788 885,
- lorsque $R_1$ est diphénylméthoxycarbonyle: par action de l'azidoformiate correspondant en milieu hydroorganique, en présence d'un bicarbonate alcalin,
- lorsque $R_1$ est (biphénylyl-4)-2 isopropyloxycarbonyle: par analogie avec la méthode décrite dans Helv. Chim. Acta, 51, 924 (1968),
- lorsque $R_1$ est un radical de formule générale (V): par application de la méthode décrite par A. Morimoto et coll., J.C.S. Perkin I, 1109 (1980) à partir des halogénures correspondants [qui peuvent être eux mêmes obtenus selon Houben Weyl, Methoden der Organischen Chemie, vol. 12 part 2 page 274, Georg Thieme Verlag Stuttgart (1964)],
- lorsque $R_1NH$ est remplacé par diméthylaminométhylèneamino: par analogie avec la méthode décrite par J.F. Fitt, J. Org. Chem. 42(15), 2639 (1977),
- lorsque $R_1NH$ est remplacé par nitro-4 benzylidèneamino ou diméthoxy-3,4 benzylidèneamino: selon la méthode décrite par R.A. Firestone, Tetrahedron Lett., 375 (1972),
- lorsque $R_2$ est méthoxyméthyle: selon S. Seki et coll., Tetrahedron Lett., 33, 2915 (1977),
- lorsque $R_2$ est t.butyle: selon R.J. Stedman, J. Med. Chem., 9, 444 (1966),
- lorsque $R_2$ est benzhydryle: selon la demande de brevet néerlandais 73 03263,
- lorsque $R_2$ est benzyle, nitrobenzyle ou p.méthoxybenzyle: selon R.R. Chauvette et coll., J. Org. Chem., 38(17), 2994 (1973).

Les dérivés de céphalosporine de formule générale (VIII) dans laquelle $R_1$ et $R_2$ sont définis comme précédemment en b) peuvent être obtenus par axylation d'un produit de formule générale (X) dans laquelle $R_2$ est défini comme en b) par application des méthodes décrites dans la demande de brevet français 2 321 294.

Les amino-7 céphalosporines de formule générale (X) dans laquelle $R_2$ est défini comme en b) peuvent être obtenues par application ou par analogie avec les méthodes décrites par E.H. Flynn, Cephalosporins and Penicillins, Ac. Press (1972).

Les acides de formule générale (XI), dans laquelle $R_5$ est hydrogène ou alcoyle, peuvent être

préparés selon la méthode décrite dans le brevet belge 850 662.

Les produits de formule générale (XI) dans laquelle $R_5$ est un radical vinyle peuvent être préparés selon la méthode décrite dans le brevet belge 869 079.

Les produits de formule générale (XI) dans laquelle $R_5$ est un radical cyanométhyle peuvent être préparés selon la méthode décrite dans la demande de brevet allemand 2 812 625.

Les acides de formule générale (XI), dans laquelle $R_5$ est un radical protecteur, peuvent être préparés par protection de l'oxime d'un tel acide dans lequel $R_5$ est hydrogène, par toute méthode connue qui n'altère pas le reste de la molécule. La protection s'effectue notamment par les groupements trityle ou tétrahydropyrannyle qui sont éliminables par acidolyse, par exemple par l'acide trifluoracétique, l'acide formique (aqueux ou non) ou l'acide p.toluènesulfonique. La protection peut également s'effectuer par le groupement méthoxy-2 propyl-2 qui peut être éliminé par application de la méthode décrite dans le brevet belge 875 379.

Les acides de formule générale (XI) dans laquelle $R_5$ est un radical de formule générale (II') peuvent être préparés selon les méthodes décrites dans les brevets belges 864 810, 865 298, 876 541 et 876 542.

Les nouveaux produits de formule générale (I) sont utiles comme intermédiaires pour la préparation de céphalosporines de formule générale:

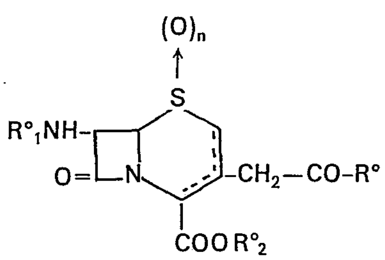

(XV)

dans laquelle $R°_1$ est défini comme $R_1$ en a) dans la formule générale (I), ou représente un atome d'hydrogène ou un radical de formule générale (II) dans laquelle le radical $R^c_5$ contenu dans $R_5$ est un atome d'hydrogène, et $R°_2$ est défini comme $R_2$ en a) dans la formule générale (I) ou représente un atome d'hydrogène, ou bien $R°_1$ et $R°_2$ sont définis comme précédemment $R_1$ et $R_2$ en b) ou représentent des atomes d'hydrogène, R° est défini comme précédemment en a) ou b) et n est égal à 0 ou 1 et qui se présentent sous forme (oxo-2 éthyl)-3 bicyclooctène-2 ou -3 lorsque n = 0 et sous forme (oxo-2 éthyl)-3 bicyclooctène-2 lorsque n = 1.

Les produits de formule générale (XV) peuvent être obtenus à partir des produits de formule générale (I) en opérant comme suit : 1) Lorsque l'on veut préparer un produit de formule générale (XV) pour lequel $R°_1$ est autre qu'un atome d'hydrogène et n = 0, on hydrolyse en milieu acide ou neutre l'énamine de formule générale (I) ou un mélange de ses isomères, puis élimine éventuellement les radicaux protecteurs d'acide $R^c_5$ et/ou $R_2$ (pour obtenir un produit pour lequel $R°_1$ contient un radical carboxy libre et/ou $R°_2$ est un atome d'hydrogène), ainsi que le cas échéant les radicaux protecteurs contenus par $R°_1$ tel que défini pour $R_1$ en b).

On opère de préférence dans un acide organique (par exemple acide formique, acide acétique) ou minéral (par exemple acide chlorhydrique, acide sulfurique) en présence ou non d'un solvant, en milieu aqueux ou organique, à une température comprise entre $-20°C$ et la température de reflux du mélange réactionnel, puis traite éventuellement par un base minérale (bicarbonate alcalin) ou organique (amine tertiaire ou pyridine).

Lorsque l'on opère en milieu organique, l'hydrolyse est réalisée par addition d'eau au mélange réactionnel.

Lorsque l'on opère en présence d'un solvant, il n'est pas nécessaire que le solvant soit miscible à la phase aqueuse acide. Le contact est alors réalisé par agitation vive.

Parmi les solvants utilisables peuvent être cités les solvants chlorés, l'acétate d'éthyle, le tétrahydrofuranne, l'acétonitrile, le diméthylformamide, les alcools.

Il n'est pas absolument nécessaire d'avoir purifié l'intermédiaire de formule générale (I) pour mettre en œuvre cette réaction.

Lorsque l'on veut obtenir un produit de formule générale (XV) pour lequel $R°_2$ est un atome d'hydrogène, l'élimination des groupements protecteurs du radical carboxy s'effectue:

- lorsqu'il s'agit d'un groupement t.butyle, p.méthoxybenzyle ou benzhydryle: par traitement en milieu acide. Dans le cas du radical benzhydryle, on peut opérer en présente d'anisole,
- lorsqu'il s'agit d'un groupement méthoxyméthyle: par traitement en milieu acide dilué,
- lorsqu'il s'agit d'un groupement nitrobenzyle: par réduction (notamment traitement par le zinc dans l'acide acétique ou hydrogénolyse).

Lorsque l'on veut préparer un produit de formule générale (XV) pour lequel $R°_2$ est un atome d'hydrogène et $R°_1$ est un radical de formule générale (II) dans laquelle $R_5$ est un groupement de formule générale (II') dont la fonction acide est protégée, il est nécessaire d'utiliser une énamine de formule générale (I) dans laquelle $R_2$ et $R^c_5$ sont différents et éliminables sélectivement. Il en est de même si l'on veut inversement préparer une cétone de formule générale (XV) dans laquelle $R°_1$ contient un groupement carboxy libre et $R°_2$ est autre qu'un atome d'hydrogène.

Lorsque l'on veut éliminer les groupements protecteurs contenus par $R°_1$ tel qu'il est défini précédemment pour $R_1$ en b), on opère comme décrit dans la demande de brevet français 2 321 294.

2) Lorsque l'on veut préparer un produit de formule générale (XV) dans laquelle $R°_1$ est autre qu'un atome d'hydrogène et n = 1, on oxyde le produit de formule générale (XV) correspondant pour lequel n = 0. On opère par application des méthodes décrites dans la demande de brevet allemand 2 637 176.

3) Lorsque l'on veut préparer une cétone de formule générale (XV) dans laquelle $R°_1$ est un atome d'hydrogène, on élimine le radical $R°_1$ d'un produit de formule générale (XV) [dans laquelle soit $R°_1$ est défini comme $R_1$ en a) à l'exception de représenter un radical de formule générale (II), soit $R°_1$ représente un radical trityle, alcoyloxycarbonyle (dont la partie alcoyle contient 1 à 5 atomes de carbone) de préférence ramifié sur l'atome de carbone en $\alpha$ de l'atome d'oxygène et/ou éventuellement substitué, phénoxyacétyle ou phénacétyle, et $R°_2$ a l'une des définitions correspondantes données pour $R_2$ en a) ou en b) ou représente un atome d'hydrogène], ou bien on élimine simultanément les radicaux $R°_1$ et $R°_2$ d'un produit de formule générale (XV) [dans laquelle $R°_1$ est défini comme ci-dessus et $R°_2$ a l'une des définitions correspondantes données pour $R_2$ en a) ou la définition d'un groupement facilement éliminable donnée pour $R_2$ en b), par exemple p.nitrobenzyle ou un groupe méthyle substitué tel que t.butyle ou diphénylméthyle].

L'élimination des radicaux protecteurs s'effectue par toute méthode connue pour la libération d'une fonction amine et/ou acide qui n'altère pas le reste de la molécule.

On opère notamment dans les conditions décrites dans la demande de brevet français 2 321 294.

A titre d'exemple:

L'élimination des groupements protecteurs d'amines s'effectue

- lorsque $R°_1$ représente t.butoxycarbonyle, trityle ou p.méthoxybenzyloxycarbonyle: par traitement en milieu acide. De préférence on utilise l'acide trifluoroacétique en opérant à une température comprise entre 0 et 20°C, ou bien on utilise l'acide formique, phosphorique ou polyphosphorique purs ou en présence d'eau, à une température comprise entre 20 et 60°C ou encore l'acide paratoluènesulfonique ou méthanesulfonique dans l'acétone ou l'acétonitrile à une température comprise entre 20°C et la température de reflux du mélange réactionnel
- lorsque $R°_1$ représente phénylacétyle ou phénoxyacétyle: selon la méthode décrite dans le brevet BE 758 800,
- lorsque $R°_1$ représente benzyloxycarbonyle: par hydrogénation catalytique
- lorsque $R°_1$ représente diphénylméthoxycarbonyle, (biphénylyl-4)-2 isopropyloxycarbonyle ou vinyloxycarbonyle et lorsque $R'_1NH-$ est remplacé par diméthylaminométhylèneamino, diméthoxy-3,4 benzylidèneamino ou nitro-4 benzylidèneamino: par hydrolyse en milieu acide
- lorsque $R°_1$ représente un radical diphénylphosphinoyle: selon la méthode décrite par P. Haake et coll., J. Am. Chem. Soc., 95, 8073 (1973)
- lorsque $R°_1$ représente un radical $(Z'O)_2P(O)-$: selon la méthode décrite dans le brevet belge 833 619 ou, lorsque Z' est trichloro-2,2,2 éthyle ou p.nitrobenzyle, par réduction (notamment par le zinc dans l'acide acétique).

4. Lorsque l'on veut préparer un produit de formule générale (XV) pour lequel $R°_1$ est un radical de formule générale (II) contenant le cas échéant une fonction acide libre ou protégée, et $R°_2$ a une définition donnée précédemment pour $R_2$ en a) ou représente un atome d'hydrogène, ou bien $R°_1$ et $R°_2$ ont les définitions données en b) ou $R°_2$ représente un atome d'hydrogène, et R° a une définition correspondante donnée pour $R_2$ en a ) ou b) et n est 0 ou 1, on peut aussi acyler une amino-7 céphalosporine (ou le cas échéant un mélange de ses isomères) de formule générale (XV) dans laquelle, R°, $R°_2$ et n étant définis comme ci-dessus, $R°_1$ est un atome d'hydrogène, au moyen d'un acide représenté par la formule générale:

$R°_1$–OH (XVI)

dans laquelle $R°_1$ est défini comme ci-dessus, ou par action d'un dérivé réactif de cet acide, puis éventuellement éliminer les radicaux protecteurs.

On opère par analogie avec la méthode décrite précédemment pour la préparation des produits de formule générale (VIII) à partir des produits de formules générales (X) et (XI), ou dans les conditions citées dans la demande de brevet français 2 321 294.

Il est entendu que lorsque $R°_1$ contient un radical amino ou acide, celui-ci est préalablement protégé.

Lorsque $R°_1$ contient un groupement hydroxyimino, hydroxy ou sulfo celui-ci est libre ou protégé.

Ces radicaux sont ou peuvent être protégés selon les méthodes décrites dans la demande de brevet français 2 321 294.

A titre d'exemple

les fonctions amines sont protégées par un groupement protecteur tel que cité précédemment pour $R_6$,

les fonctions acides sont protégées par un groupement protecteur tel que défini précédemment pour $R_2$,

l'oxime peut être protégée par un groupement protecteur tel que cité précédemment pour $R_5$.

l'élimination de ces radicaux s'effectue dans les conditions décrites précédemment; en particulier pour les groupements protecteurs d'amines

- lorsqu'il s'agit d'un radical benzyle ou dibenzyle: par hydrogénation catalytique
- losque'il s'agit d'un radical p.nitrobenzyloxycarbonyle: par réduction, notamment traitement par le zinc dans l'acide acétique, ou par hydrogénolyse.

L'élimination des radicaux protecteurs d'oxime

s'effectue
- lorsque'il s'agit du radical trityle ou tétrahydropyrannyle: par acidolyse par exemple par l'acide trifluoracétique, l'acide formique aqueux ou non ou l'acide paratoluènesulfonique,
- lorsqu'il s'agit du radical méthoxy-2 propyle-2: selon la méthode décrite dans le brevet belge 875 379.

Les dérivés de la céphalosporine de formule générale (XV) dans laquelle $R°_1$ et $R°_2$ sont définis comme en b) ou représentent des atomes d'hydrogène, R° est défini comme en b) et n est défini comme précédemment sont décrits pour leurs propriétés antibactériennes, ou à titre d'intermédiaires pour la préparation de produits doués d'activité antibactérienne dans la demande de brevet français 2 321 294.

Les produits de formule générale (XV) pour lesquels $R°_1$ qui sera appelé ci-après $R°_{1a}$ est défini comme $R_1$ en a) ou représente un radical de formule générale (II) dans laquelle le symbole $R^c_5$ contenu par $R_5$, est un atome d'hydrogène, $R°_2$ qui sera appelé ci-après $R°_{2a}$ est défini comme $R_2$ en a) ou représente un atome d'hydrogène, n est défini comme précédemment et R° est défini comme en a) sont des intermédiaires pour la préparation de produits de formule générale:

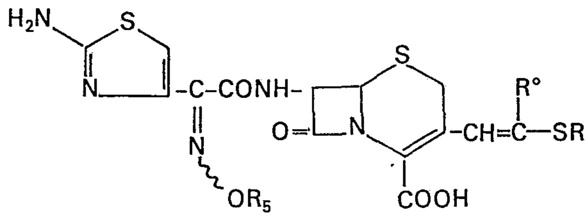

(XVII)

doués d'activité antibactérienne.

Dans la formule générale (XVII), R° est défini comme précédemment en a), $R_5$ est un atome d'hydrogène, un radical alcoyle, vinyle, cyanométhyle ou représente un groupement de formule générale (II') dans laquelle $R^a_5$ et $R^b_5$ étant définis comme en a), $R^c_5$ est un atome d'hydrogène et R est choisi parmi les significations:

1) pyridyle-2, -3 ou -4 éventuellement N-oxydés
2) pyrimidinyle-2
3) méthyl-6 oxyde-1 pyridazinyle-3
4) dioxo-5,6 tétrahydro-1,4,5,6 triazine-1,2,4 yle-3 substitué en position -4 par
a) un radical alcoyle contenant 1 ou 2 atomes de carbone éventuellement substitué par un radical alcoyloxy, alcoylthio, formyle,
b) un radical allyle, dihydroxy-2,3 propyle, dihydroxy-1,3 propyle-2 ou formyl-2 hydroxy-2 éthyle,
c) un radical alcoyle contenant 2 ou 3 atomes de carbone substitué par hydroxy, carbamoyloxy, acyloxy ou acylamino (dont les portions acyles sont non substituées ou substituées par amino), alcoylsulfonylamino, uréido, alcoyluréido ou dialcoyluréido,
5) dioxo-5,6 tétrahydro-1,4,5,6 triazine-1,2,4 yle-3 substitué en position -1 ou dioxo-5,6 tétrahydro-1,2,5,6 triazine-1,2,4 yle-3 substitué en position -2 par un radical alcoyle contenant 1 ou 2 atomes de carbone éventuellement substitué par un radical formyle
6) alcoyl-2 dihydro-2,5 oxo-5 triazine-1,2,4 yle-3 éventuellement substitué en position -6 par un radical alcoyle ou alcoyloxy dont les portions et radicaux alcoyles contiennent 1 ou 2 atomes de carbone,
7) amino-1 dihydro-1,2 oxo-2 pyrimidinyle-4
8) thiadiazol-1,3,4 yle-5 substitué par alcoyle, dialcoylaminoalcoyle ou acylaminoalcoyle,
9) tétrazolyle-5 substitué en position -1 par
a) un radical alcoyle contenant 1 ou 2 atomes de carbone éventuellement substitué par un radical formyle,
b) un radical alcoyle contenant 2 ou 3 atomes de carbone substitué par hydroxy, acylamino, ou dialcoylamino, ou
10) a) alcoyl-1 triazol-1,2,4 yle-5 éventuellement substitué en position -3 par un radical alcoyloxycarbonyle dont les radicaux alcoyle et alcoyloxy contiennent 1 ou 2 atomes de carbone
b) alcoyl-1 triazol-1,3,4 yle-5

Il est entendu que les substituants R° et -SR peuvent être situés respectivement en position E et Z et inversement par rapport au noyau céphalosporine et que ces isomères et leurs mélanges entrent dans la définition de la formule générale (XVII).

De même le radical $-OR_5$ peut se trouver dans les positions syn ou anti et ces isomères et leurs mélanges entrent aussi dans la définition de la formule générale (XVII).

Lorsque le radical R est un radical tétrahydro-1,4,5,6 triazinyle substitué en position -1 ou -4 ou tétrahydro-1,2,5,6 triazinyle substitué en position -2, il peut être représenté par les formes tautomères:

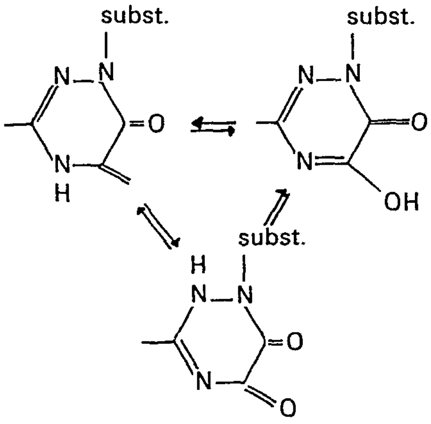

(XVIII a)

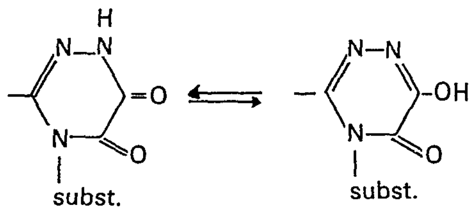

(XVIII b)

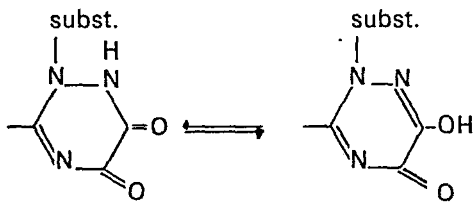

(XVIII c)

Lorsque le radical R contient un substituant formylalcoyle, il peut se présenter sous sa forme aldéhyde libre ou hydrate d'aldéhyde. On observe notamment ces formes dans les conditions décrites ci-dessous.

Les études de résonance magnétique nucléaire montrent en particulier que lorsque R est dioxo-5,6 formylméthyl-4 tétrahydro-1,4,5,6 triazine-1,2,4 yle-3:

- en solvant acide, tel que l'acide formique ou trifluoroacétique (deutérés), en présence ou non-d'eau (lourde) le produit se présente principalement sous la forme d'aldéhyde libre.
- en solvant basique tel que l'eau (lourde) additionnée de bicarbonate de sodium, il se présente principalement sous la forme d'hydrate d'aldéhyde.
- en solvant neutre tel que diméthylsulfoxyde ($d_6$), les formes aldéhyde libre et hydrate d'aldéhyde sont présentes, l'addition d'eau déplaçant l'équilibre en faveur de la forme hydrate d'aldéhyde.

Les produits de formule générale (XVII) peuvent être obtenus à partir des cétones de formule générale (XV) en opérant comme suit.

Les céphalosporines de formule générale:

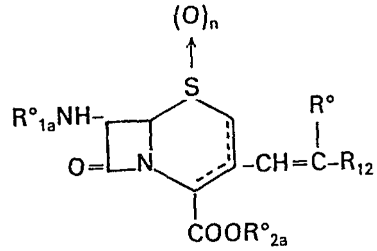

(XIX)

[dans laquelle $R°_{1a}$, $R°_{2a}$ et n sont définis comme précédemment ou $R°_{1a}$ est un radical de formule générale (II) dans laquelle $R^c_5$ et/ou $R^6$ sont des atomes d'hydrogène, R° est défini comme précédemment en a), étant entendu que lorsque n = 0 le produit se présente sous forme bicyclooctène-2 et/ou lorsque n = 1 le produit se présente sous forme bicyclooctène-2 et le symbole $R_{12}$ représente un radical de formule générale:

$R'_{12}SO_2O-$ (XX)

dans laquelle $R'_{12}$ représente un radical alcoyle, trihalogénométhyle (par exemple trifluorométhyle) ou phényle éventuellement substitué par un atome d'halogène ou par un ou plusieurs radicaux alcoyle ou nitro], peuvent être préparées par action d'une forme activée d'un acide $R'_{12}SO_3H$ du type:

$(R'_{12}SO_2)_2O$ (XXa)

$R'_{12}SO_2Hal$ (XXb)

[$R'_{12}$ étant défini comme ci-dessus et Hal étant un atome d'halogène] sur une cétone de formule générale (XV) telle que définie pour les intermédiaires des produits de formule générale (XVII), ou sur un mélange de ses isomères, suivie éventuellement de la réduction du sulfoxyde obtenu lorsque n = 1 et éventuellement de l'élimination des radicaux protecteurs de la fonction amine du radical de formule générale (II) et/ou des fonctions acides.

Il est entendu que, lorsque $R°_{1a}$ est un radical de formule générale (II) dans laquelle $R_5$ est un atome d'hydrogène, il est nécessaire que l'oxime soit protégée. La protection et l'élimination s'effectuent selon les méthodes décrites précédemment.

On opère généralement en présence
- d'une base tertiaire de formule générale:

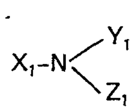

(XXI a)

dans laquelle $X_1$, $Y_1$ et $Z_1$ représentent des radicaux alcoyle ou phényle, ou éventuellement deux d'entre eux forment un cycle avec l'atome d'azote auquel ils sont rattachés (par exemple la triéthylamine ou la diméthylaniline)
- ou d'une pyridine substituée par des radicaux alcoyle ou dialcoylamino (par exemple la di-t.butyl-2,6 méthyl-4 pyridine)
- ou d'un amidure de lithium obtenu par réaction

d'un alcoyllithium (par exemple le n.butyllithium) sur une amine secondaire de formule générale:

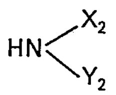

(XXI B)

dans laquelles $X_2$ et $Y_2$ représentent des radicaux alcoyle ou phényle, ou éventuellement $X_2$ et $Y_2$ forment un cycle avec l'atome d'azote auquel ils sont rattachés (par exemple la diisopropylamine ou la tétraméthyl-2,2,6,6 pipéridine)
- ou d'un alcoolate de métal alcalin (par exemple le t.butylate de potassium)
- ou d'un hydrure de métal alcalin (par exemple l'hydrure de potassium)
- ou d'un alcoyllithium ou d'un aryllithium (par exemple le n-butyllithium ou le mésityllithium), dans un solvant organique tel qu'un éther (par exemple le dioxanne, le tétrahydrofuranne ou le diméthoxy-1,2 éthane) ou dans un mélange de solvants organiques comprenant un éther (tel que cité ci-dessus) et un solvant tel que l'hexaméthylphosphorotriamide, la N-méthylpyrrolidone ou la diméthyl-1,3 imidazolidinone-2, à une température comprise entre −78°C et la température de reflux du mélange réactionnel, éventuellement sous atmosphère inerte (azote ou argon).

Le cas échéant la réduction du S-oxyde s'effectue selon les méthodes décrites dans la demande de brevet allemand 2 637 176.

L'élimination des radicaux protecteurs d'amine et d'acides s'effectue dans les conditions décrites précédemment.

Il est également possible d'obtenir les produits de formule générale:

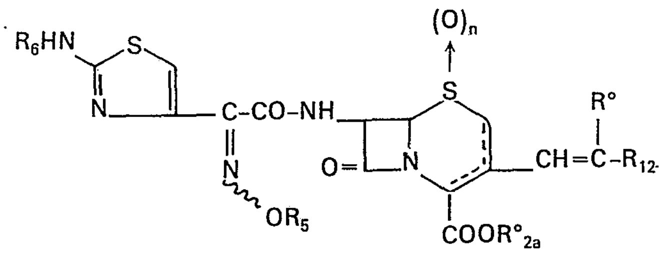

(XXII)

[dans laquelle, $R_5$, $R°_{2a}$, R°, $R_{12}$ et n étant définis comme précédemment, lorsque n = 0 le produit se présente sous forme bicyclooctène-2 ou -3 et, lorsque n = 1, le produit se présente sous forme bicyclooctène-2, et $R_6$ et $R^c_5$ (contenu dans $R_5$) sont définis comme précédemment en a) ou représentent des atomes d'hydrogène], en opérant de la manière suivante:

On prépare une amino-7 céphalosporine de formule générale:

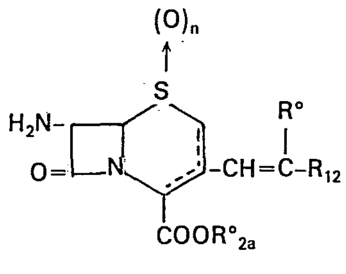

(XXIII)

[dans laquelle $R°_{2a}$, $R_{12}$ et n sont définis comme précédemment et la position de la double liaison ainsi que la configuration du substituant en -3 est la même que pour le produit de formule générale (XIX)], par élimination du radical $R°_{1a}$ ou éventuellement élimination simultanée des radicaux protecteurs $R°_{1a}$ et $R°_{2a}$ d'un produit de formule générale (XIX) [dans laquelle $R°_{1a}$ est défini comme $R_1$ précédemment en a) à l'exception de représenter un radical de formule générale (II)].

On opère dans les conditions décrites précédemment pour la préparation d'un produit de formule générale (XV) dans laquelle $R°_1$ est un atome d'hydrogène.

On peut alors préparer les produits de formule générale (XXII) par axylation de l'amino-7 céphalosporine de formule générale (XXIII) (ou le cas échéant d'un mélange de ses isomères), au moyen d'un acide de formule générale (XI) ou par action d'un dérivé réactif de cet acide, puis éventuellement réduction du sulfoxyde obtenu et éventuellement élimination des radicaux protecteurs.

On opère dans les conditions décrites précédemment pour la préparation d'une amino-7 céphalosporine de formule générale (VIII), les conditions de blocage étant les mêmes.

Le cas échéant la réduction du sulfoxyde et l'élimination des radicaux protecteurs d'acide et/ou d'amine s'effectuent dans les conditions décrites précédemment.

I. Les thiovinyl-3 céphalosporines de formule générale (XVII) peuvent être préparées par action d'un thiol (ou d'un de ses sels alcalins ou alcalino-terreux) de formule générale:

R-SH (XXIV)

(dans laquelle R, qui est défini comme précédemment, est éventuellement protégé), sur un dérivé de la céphalosporine (ou un mélange des isomères) de formule générale (XXII) tel que défini précédemment, suivie de la réduction du sulfoxyde obtenu lorsque n = 1 et le cas échéant de l'élimination des radicaux protecteurs.

Lorsque l'on veut obtenir un produit de formule générale (XVII) dans laquelle R contient un radical formyle, on met en œuvre un thiol de formule générale (XXIV) dans laquelle ce radical est protégé à l'état d'acétal de formule générale:

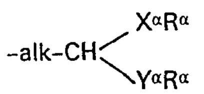
(XXV a)

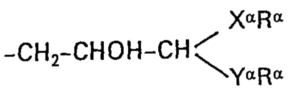
(XXV b)

dans lesquelles alk est un radical alcoylène contenant 1 ou 2 atomes de carbone, $X^\alpha$ et $Y^\alpha$ sont identiques et représentent des atomes d'oxygène ou de soufre et $R^\alpha$ représente un radical alcoyle, ou bien $X^\alpha$ et $Y^\alpha$ sont identiques ou différents et représentent des atomes d'oxygène ou de soufre et les radicaux $R^\alpha$ forment ensemble un radical alcoylène contenant 2 ou 3 atomes de carbone.

Il est entendu que, lorsque le radical R du produit de formule générale (XXIV) est susceptible d'interférer avec la réaction, il est préférable de protéger ce groupement, par toute méthode connue en soi et qui n'altère pas le reste de la molécule.

S'il s'agit d'un groupement amino ou alcoylamino, la protection s'effectue par un radical tel que $R_6$ défini précédemment.

S'il s'agit de groupements hydroxy la protection s'effectue par des radicaux trityle, tétrahydropyrannyle, méthoxy-2 propyle-2 ou bien, lorsqu'il s'agit d'un radical dihydroxy-2,3 propyle ou dihydroxy-1,3 propyle-2, à l'état d'acétal cyclique sous forme d'un radical diméthyl-2,2 dioxolannyl-4 méthyle ou diméthyl-2,2 dioxannyle-5.

Par ailleurs il est entendu que, lorsque le radical R du produit de formule générale (XXIV) comporte un radical hydroxy ou sulfonyle, il est préférable de mettre en œuvre un produit de formule générale (XXII) dans laquelle n = 0.

Il est également entendu que, lorsque dans la formule générale (XXII), le radical $R_5$ est un atome d'hydrogène, il est préférable de protéger l'oxime dans les conditions décrites précédemment. La réaction des produits de formules générales (XXIV) et (XXII) s'effectue généralement en présence d'une base telle qu'une pyridine ou une base organique tertiaire de formule générale (XXIa). On utilise par exemple la diisopropyléthylamine ou la diéthylphénylamine.

Lorsque l'on utilise un sel du thiol de formule générale (XXIV) il n'est pas nécessaire d'opérer en présence d'une base organique telle que définie ci-dessus.

On opère avantageusement dans un solvant organique tel que le diméthylformamide, le tétrahydrofuranne ou l'acétonitrile, le méthanol, l'éthanol ou un mélange de tels solvants.

Il est également possible d'opérer en présence de bicarbonate alcalin dans un solvant tel que cité ci-dessus, éventuellement en présence d'eau. On opère à un température comprise entre −20°C et la température de reflux du mélange réactionnel, la température choisie étant variable selon le thiol employé. De même, selon le thiol employé, le temps de réaction peut varier de 5 minutes à 48 heures.

Eventuellement on opère sous azote.

La réduction de l'oxyde et l'élimination des groupements protecteurs d'amine, d'acide ou de l'oxime s'effectuent selon les méthodes décrites précédemment.

L'élimination des radicaux protecteurs de groupements hydroxy s'effectue dans les conditions décrites précédemment pour les radicaux protecteurs de l'oxime, c'est à dire:

- par acidolyse par exemple par l'acide trifluoracétique, l'acide formique aqueux ou non ou l'acide de paratoluènesulfonique lorsqu'il s'agit du radical trityle, téthrahydropyrannyle, diméthyl-2,2 dioxolannyl-4 méthyle ou diméthyl-2,2 dioxannyle-5. Lorsqu'on utilise l'acide formique, aqueux ou non, la libération des radicaux hydroxy protégés à l'état d'acétal cyclique peut conduire au moins partiellement aux mono ou diesters formiques correspondants, qui peuvent être séparés le cas échéant par chromatographie.
- selon la méthode décrite dans le brevet belge 875 379 lorsqu'il s'agit du radical méthoxy-2 propyle-2.

L'élimination des groupements de formule générale (XXV a) ou (XXV b) (lorsque l'on veut obtenir un produit de formule générale (XVII) dans laquelle R contient un radical formyle) s'effectue
- en présence d'un acide sulfonique (par exemple acide méthanesulfonique ou acide p.toluènesulfonique) dans un solvant organique (par exemple acétonitrile ou acétone), éventuellement en présence d'eau et éventuellement en présence d'un réactif acétalisable tel que l'acétone, l'acide glyoxylique, le benzaldéhyde ou l'acide pyruvique, à une température comprise entre 20°C et la température de reflux du mélange réactionnel,
- ou bien, lorsque le radical R est un radical dioxo-5,6 tétrahydro-1,4,5,6 triazine-1,2,4 yle-3, par action d'acide formique pur ou aqueux (contenant de préférence moins de 10% d'eau), soit en présence ou non de silice, soit par transacétalisation en présence d'un réactif acétalisable tel que défini ci-dessus.

Il est souvent préférable d'opérer au moyen du sulfoxyde de la céphalosporine de formule générale (XXII). Dans le cas contraire, lorsque l'on obtient un mélange d'isomères bicyclooctène-2 et bicyclooctène-3 de formule générale (XVII), ce mélange peut être oxydé pour obtenir l'oxyde du produit correspondant de formule générale (XVII) qui se présente sous forme bicyclooctène-2. Cet oxyde peut alors être réduit pour obtenir l'isomère bicyclooctène-2 de formule générale (XVII).

Les thiols de formule générale (XXIV) (qui peuvent être mis en œuvre sous leur forme tautomère), peuvent être préparés par application de

l'une des méthodes suivantes selon la signification du radical R:

- lorsque R est un radical pyridyle-3: selon la méthode décrite par H.M. Wuest et E.H. Sakal, J. Am. Chem. Soc., 73, 1210 (1951),
- lorsque R est un radical oxyde-1 pyridyle-3: selon la méthode décrite par B. Blank et coll., J. Med. Chem. 17, 1065 (1974),
- lorsque R est un radical oxyde-1 pyridyle-4: selon la méthode décrite par R.A.Y. Jones et coll., J. Chem. Soc. 2937 (1960),
- lorsque R est un radical méthyl-6 oxyde-1 pyridazinyle-3: selon la méthode décrite dans le brevet belge 787 635,
- lorsque R est

1°/- un radical dioxo-5,6 tétrahydro-1,4,5,6 triazine-1,2,4 yle-3 substitué en position -4 par un radical $R^{\gamma}$ choisi parmi:

a) un radical allyle, alcoyle (1 ou 2 atomes de carbone, lui-même éventuellement substitué par un radical alcoyloxy ou alcoylthio),

b) un radical dihydroxy-2,3 propyle ou dihydroxy-1,3 propyle-2 (éventuellement protégés sous forme d'acétal cyclique)

c) un radical alcoyle [2 à 3 atomes de carbone, lui-même substitué par hydroxy, carbamoyloxy, alcoylsulfonylamino, acylamino (éventuellement substitué), uréido, alcoyluréido ou dialcoyluréido],

d) un radical de formule générale (XXVa) ou (XXVb),

2°/- un radical dioxo-5,6 tétrahydro-1,4,5,6 triazine-1,2,4 yle-3 substitué en position -1, ou dioxo-5,6 tétrahydro-1,2,5,6 triazine-1,2,4 yle-3 substitué en position -2 par un radical alcoyle contenant 1 ou 2 atomes de carbone ou par un radical de formule générale (XXVa): en faisant agir un oxalate d'alcoyle sur une thiosemicarbazide de formule générale:

$R\gamma$ NH CS NH–$NH_2$ (XXVI a)

$H_2NCS$ NH NH–$R\gamma$ (XXVI b)

ou $H_2N$ CS N–$NH_2$ (XXVI c)

$\quad\quad\quad |$

$\quad\quad\quad R\gamma'$

dans lesquelles $R^{\gamma}$ a la définition donnée ci-dessus en 1°/- et $R^{\gamma'}$ est un substituant défini ci-dessus en 2°/-, en présence d'un alcoolate alcalin, par exemple l'éthylate ou le méthylate de sodium ou le t.butylate de potassium, par application de la méthode décrite par M. Pesson et M. Antoine, Bull. Soc. Chim. France 1590 (1970).

Il n'est pas absolument nécessaire de purifier le produit obtenu (ni de libérer les radicaux protégés) pour le mettre en œuvre pour la préparation des produits de formule générale (XVII).

La thiosemicarbazide de formule générale (XXVIa), (XXVIb) ou (XXVIc) peut être préparée selon l'une des méthodes décrites par K. A. Jensen et coll., Acta Chem. Scand., 22, 1 (1968), ou par application de la méthode décrite par Y. Kazakov et J.Y. Potovskii, Doklady Acad. Nauk. SSSR 134, 824 (1960), étant entendu que, lorsque $R^{\gamma}$ contient un radical amino, ce dernier est protégé.

La protection du radical amino et l'élimination du radical protecteur s'effectuent selon les méthodes habituelles qui n'altèrent pas le reste de la molécule. On utilise notamment le groupement t.butoxycarbonyle, qui peut être éliminé par hydrolyse acide.

- Lorsque R est un radical alcoyl-1 triazol-1,3,4 yle-5: par application de l'une des méthodes décrites par M. Pesson et M. Antoine, Bull. Soc. Chim. France 1590 (1970);
- Lorsque R est un radical dioxo-5,6 tétrahydro-1,4,5,6 triazine-1,2,4 yle-3 substitué en position -4 par acyloxyalcoyle (éventuellement substitué): par acylation respectivement de la dioxo-5,6 hydroxyalcoyl-4 mercapto-3 tétrahydro-1,4,5,6 triazine-1,2,4, dont le radical mercapto a été préalablement protégé (par exemple selon C.G. Kruse et coll., Tet. Lett. 1725 (1976), par toute méthode connue pour acyler un alcool sans toucher au reste de la molécule, puis libération du groupement mercapto en milieu acide.
- Lorsque R est un radical alcoyl-2 dioxo-5,6 tétrahydro-1,2,5,6 triazine-1,2,4 yle-3, alcoyl-1 triazol-1,2,4 yle-5 ou alcoyl-1 alcoyloxycarbonyl-3 triazol-1,2,4 yle-5: selon la méthode décrite par M. Pesson et M. Antoine, C.R. Acad. Sci., Ser C, 267, 25, 1726 (1968).
- Lorsque R est un radical alcoyl-2 dihydro-2,5 oxo-5 triazine-1,2,4 yle-3 substitué en position -6 par un radical alcoyle ou alcoyloxy: selon la méthode décrite dans J. Antibiotics, 33, 783 (1980).
- Lorsque R est un radical amino-1 dihydro-1,2 oxo-2 pyrimidinyle-4: selon la méthode décrite dans la demande de brevet européen EP 00005.
- Lorsque R est un radical thiadiazol-1,3,4 yle-5 éventuellement substitué par alcoyle: selon les méthodes décrites dans le brevet belge 830 821,
- Lorsque R est un radical thiadiazol-1,3,4 yle-5 substitué par dialcoylaminoalcoyle: selon la méthode décrite dans la demande de brevet allemand 2 446 254.
- Lorsque R est un radical thiadiazol-1,3,4 yle-5 substitué par un radical acylaminoalcoyle: selon la méthode décrite dans la demande de brevet japonais 7 680 857,
- Lorsque R est un radical tétrazolyle-5 éventuellement substitué en position -1 par alcoyle ou hydroxyalcoyle: selon les méthodes décrites dans le brevet belge 830 821.
- Lorsque R est un radical tétrazolyle-5 substitué en position -1 par un radical dialcoylaminoalcoyle: par application de la méthode décrite dans la demande de brevet allemand 2 738 711.

- Lorsque R est un radical tétrazolyle-5 substitué par un radical acylaminoalcoyle: selon la méthode décrite dans le brevet US 4 117 123.

- Lorsque R est un radical tétrazolyle-5 substitué en position -1 par un radical de formule générale (XXVa): par action d'azoture de sodium sur l'isothiocyanate correspondant, par analogie avec la méthode décrite par R.E. Orth, J. Pharm. Sci. 52 (9), 909 (1963). II – Les thiovinylcéphalosporines

de formule générale (XVII) peuvent également être obtenues de la manière suivante.

On fait agir un thiol de formule générale (XXIV) (ou un de ses sels alcalins ou alcalino-terreux) sur un produit de formule générale (XIX) (ou sur un mélange de ses isomères) [dans laquelle $R°_{1a}$ est défini comme $R_1$ en a) à l'exception de représenter un radical de formule générale (II)] puis on réduit éventuellement le sulfoxyde obtenu (lorsque n = 1) et élimine éventuellement les radicaux protecteurs de R pour préparer un produit de formule générale:

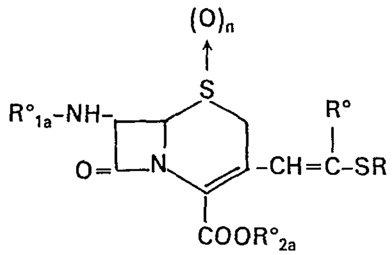
(XXVII)

dans laquelle, n étant défini comme précédemment, R°, $R°_{1a}$ et $R°_{2a}$ sont définis comme ci-dessus et R prend une définition correspondante.

La réaction s'effectue dans les conditions décrites précédemment pour l'obtention d'un produit de formule générale (XVII) à partir d'un produit de formule générale (XXII) et d'un thiol de formule générale (XXIV).

Il est entendu que le radical R du thiol est (le cas échéant) protégé comme décrit précédemment et que l'élimination des radicaux protecteurs peut être effectuée dans les conditions décrites précédemment. Il est cependant préférable de conserver les groupements protecteurs jusqu'à l'obtention du produit de formule générale (XVII).

Il est souvent préférable d'employer un produit de formule générale (XIX) dans laquelle n = 1. Dans le cas contraire lorsque l'on obtient un mélange d'isomères bicyclooctène-2 et bicyclooctène-3, il est nécessaire de transformer le produit en sulfoxyde pour la suite des opérations, ce qui a pour effet de stabiliser la double liaison de telle manière que l'on ait un bicyclooctène-2, puis de réduire le sulfoxyde du produit final obtenu.

On prépare un produit de formule générale:

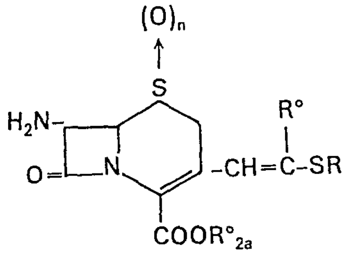
(XXVIII)

dans laquelle R, R°, $R°_{2a}$ et n sont définis comme ci-dessus, par élimination du radical $R°_{1a}$ d'un produit de formule générale (XXVII) tel que défini précédemment ou éventuellement élimination simultanée du radical $R°_{1a}$ et des autres radicaux protecteurs de ce produit.

On opère dans les conditions décrites précédemment pour la préparation d'un produit de formule générale (XXIII).

*Il n'est pas nécessaire d'isoler le produit de formule générale (XXVIII) pour l'utiliser dans la suite de la synthèse.*

On prépare alors la thiovinyl-3 céphalosporine de formule générale (XVII) par acylation d'une amino-7 céphalosporine de formule générale (XXVIII) dans laquelle R, R° et $R°_{2a}$ sont définis comme précédemment, au moyen d'un acide représenté par la formule générale (XI) [dans laquelle $R_5$ et $R_6$ sont définis comme précédemment], ou d'un dérivé réactif de cet acide, dans les conditions décrites précédemment pour la préparation des produits de formule générale (VIII), puis on réduit l'oxyde obtenu (lorsque n = 1) et élimine les radicaux protecteurs.

Il est entendu que:

- les radicaux amino ou alcoylamino, qui existent dans certains radicaux R, doivent être protégés, et
- les radicaux carboxy, hydroxy ou formyle contenus dans les radicaux R peuvent être protégés.

La protection, et l'élimination des radicaux protecteurs, ainsi que la réduction de l'oxyde s'effectuent dans les conditions décrites précédemment.

Il est également entendu que, lorsque R contient un substituant hydroxy ou sulfonyle, on préfère mettre en œuvre un produit de formule générale (XXVIII) dans laquelle n = 0.

Les produits de formules générales (XIX), (XXII) ou (XXVII), dans lesquelles n = 1, peuvent être obtenus par oxydation des produits correspondants dans lesquels n = 0 selon la méthode décrite dans la demande de brevet DE 2 637 176.

Les isomères des produits de formules générales (I), (XV), (XVII), (XIX), (XXII), (XXIII), (XXVII), (XXVIII) peuvent être séparés par chromatographie ou par cristallisation.

Plus particulièrement, les isomères bicyclooctène-2 des produits de formule générale (XV), (XVII), (XIX), (XXII) ou (XXVII) peuvent être obtenus à partir des mélanges bicyclooctène-2+bicyclooctène-3, par oxydation puis réduction.

Les produits de formule générale (XVII) peuvent être transformés en sels d'addition avec les acides. Ils peuvent également être transformés en sels métalliques ou en sels d'addition avec les bases azotées selon les méthodes connues en soi.

Les nouveaux produits selon l'invention et les produits de formules générales (XV) et (XVII) peuvent être éventuellement purifiés par des méthodes physiques telles que la cristallisation ou la chromatographie.

Les dérivés de la céphalosporine de formule générale (XVII) et leurs sels pharmaceutique-

ment acceptables présentent des propriétés antibactériennes particulièrement intéressantes. Ils manifestent une activité remarquable in vitro et in vivo sur les germes Gram-positifs et Gram-négatifs.

In vitro, ils se sont montrés actifs à une concentration comprise entre 2 et 20 µg/cm³ sur des souches de staphylocoques sensibles à la pénicilline G (Staphylococcus aureus Smith), et à une concentration comprise entre 1 et 10 µg/cm³ sur Escherichia coli souche NIHJ.

In vivo, ils se sont montrés actifs sur les infections expériementales de la souris à Staphylococcus aureus Smith (sensible à la pénicilline G) à une dose comprise entre 1 et 20 mg/kg par jour par voie sous-cutanée, et à Escherichia coli (souche Monod) à des doses comprises entre 0,01 et 10 mg/kg par jour par voie sous-cutanée.

Par ailleurs la $DL_{50}$ des produits de formule générale (XVII) est comprise entre 1,5 et des doses supérieures à 2,5 g/kg par voie sous-cutanée chez la souris.

D'un intérêt particulier sont les produits de formule générale (I) dans laquelle le symbole $R_1$ est un radical trityle, un radical de formule générale (III), un radical de formule générale (IV), ou un radical de formule générale (V), et le symbole $R_2$ est un radical protecteur tel que défini en a) ou bien le symbole $R_1$ est un radical de formule générale (VI), et le symbole $R_2$ est un radical protecteur tel que défini en b), le symbole R° représente un radical alcoyle ou phényle et les symboles $R_3$ et $R_4$ représentent des radicaux alcoyle, et parmi ces produits, plus spécialement les produits de formule générale (I) dans laquelle le symbole $R_1$ est un radical trityle, un radical de formule générale (III) dans laquelle $R_7$ est phénoxyméthyle, un radical de formule générale (IV) dans laquelle $R_8$ est un radical alcoyle ramifié, un radical de formule générale (V) dans laquelle Z est un radical alcoyloxy ou un radical α-amino α-phénylacétamido dont la fonction amine est protégée, le symbole $R_2$ est un radical benzhydryle ou p.méthoxybenzyle le symbole R° est méthyle ou phényle et les symboles $R_3$ et $R_4$ sont des radicaux méthyle.

Les exemples suivants, donnés à titre non limitatif, illustrent la présente invention.

Exemple 1

A une solution de 2,4 g de benzhydryloxycarbonyl-2 t.butoxycarbonylamino-7 méthyl-3 oxo-8 thia-5 aza-1 bicyclo[4.2.0] octène-2 dans 10 cm³ de diméthylformamide à 80°C on ajoute goutte à goutte 2,02 g de diméthylamino-1 méthoxy-1 éthylène en 50 minutes. On concentre à sec sous pression réduite (1 mm de mercure; 0,13 kPa) à 30°C. Le résidu est dissous dans 10 cm³ d'acétate d'éthyle et la solution est filtrée sur 50 g d'alumine basique. On élue avec 200 cm³ d'acétate d'éthyle et concentre l'éluat à sec sous pression réduite (20 mm de mercure; 2,7 kPa) à 20°C. On obtient ainsi 1 g de benzhydryloxycarbonyl-2 t.butoxycarbonylamino-7 (diméthylamino-2 propène-1 yl-1)-3 oxo-8 thia-5 aza-1 bicyclo[4.2.0] octène-2, mélange 50/50 des formes Z et E, sous forme d'une huile rouge.

Spectre infra-rouge ($CCl_4$), bandes caractéristiques ($cm^{-1}$) 3440, 1770, 1720, 1505, 1370, 1160, 700.

Spectre de RMN du proton (350 MHz, $CDCl_3$, δ en ppm. J en Hz) Mélanges des isomères Z et E: 1,45 (s, $-C(CH_3)_3$); 1,88 et 1,94 (2s, $=\underset{\|}{C}-CH_3$); 2,85 et 2,86 (2s, $-N(CH_3)_2$); 3,05 à 3,35 (m, $-S-CH_2-$); 5,05 (d, J = 4, -H en 6); 5,32 (m, -H en 7); 5,50 (d, J = 9, -CONH-); 5,83 et 6,03 (2s, -CH=); 6,89 (s, $-COOC\underline{H}(C_6H_5)_2$).

Exemple 2

A une solution de 1,08 g de benzhydryloxycarbonyl-2 t.butoxycarbonylamino-7 méthyl-3 oxo-8 thia-5 aza-1 bicyclo[4.2.0] octène-2 dans 15 cm³ de dioxanne à 80°C,on ajoute en 3 minutes une solution de 1,9 g de tris-diméthylaminométhylbenzène dans 15 cm³ de dioxanne en maintenant la température à 80°C, puis poursuit l'agitation à cette température pendant 15 minutes. Le mélange réactionnel est versé dans un mélange agité de 100 cm³ d'acétate d'éthyle et 100 cm³ d'eau et de glace pilée. La phase organique est séparée, lavée deux fois par 50 cm³ d'eau, séchée sur sulfate de magnésium et le solvant est évaporé à sec sous pression réduite (20 mm de mercure; 2,7 kPa) à 20°C. Le résidu et chromatographié sur une colonne de 200 g de gel de silice Merck (0,04–0,06) (diamètre de la colonne: 4 cm, hauteur: 32 cm). On élue par 1,5 litre de mélange acétate d'éthylecyclohexane 20–80 (volumes) sous une pression de 40 kPa en recueillant des fractions de 50 cm³. Les fractions 19 à 26 sont réunies et concentrées à sec sous pression réduite (20 mm de mercure; 2,7 kPa) à 20°C. On obtien 0,4 g de benzhydryloxycarbonyl-2 t.butoxycarbonylamino-7 [α-(β-diméthylaminostyryl]-3 oxo-8 thia-5 aza-1 bicyclo [4.2.0] octène-2, forme E sous forme d'un solide jaune.

Spectre infra-rouge ($CHCl_3$), bandes caractéristiques ($cm^{-1}$), 3430, 1765, 1715, 1575, 1540, 1500, 1495, 1450, 1390, 1370, 1150, 690.

Spectre de RMN du proton (350 MHz, $CDCl_3$, δ en ppm, J en Hz) 1,48 (s, 9H, $(CH_3)_3C-$); 2,30 et 2,40 (2d, J = 14, 2H, $-CH_2-S-$); 2,83 (s, 6H, $-N(CH_3)_2$); 4,97 (d, J = 4, 1H, H en 6); 5,20 (d, J = 9, 1H,-CONH-); 5,27 (dd, J = 4 et 9, 1H, H en 7); 6,61 (s, 1H, -CH=); 6,92 (s, 1H, -COOCH<).

Exemple 3

A une solution de 3,75 g de t.butoxycarbonylamino-7 (méthoxy-4 benzyloxycarbonyl)-2 méthyl-3 oxo-8 thia-5 aza-1 bicyclo[4.2.0] octène-2 dans 25 cm³ de diméthylformamide portée à 80°C on ajoute en 55 minutes 4 cm³ de diméthylamino-1 méthoxy-1 éthylène en maintenant la température à 80°C. On obtient ainsi une solution de t.butoxycarbonylamino-7 (diméthylamino-2 propène-1 yl-1)-3 (méthoxy-4 benzyloxycarbonyl)-2 oxo-8 thia-5 aza-1 bicyclo[4.2.0] octène-2 que l'on verse dans un mélange de 20 cm³ d'eau glacée et

150 $cm^3$ d'acétate d'éthyle. La phase organique est lavée par 2 fois 200 $cm^3$ d'eau, séchée sur sulfate de sodium, filtrée en présence de noir décolorant et évaporée à sec sous pression réduite (20 mm de mercure; 2,7 kPa). Le résidu est chromatographié sur une colonne de 45 g de gel de silice Merck (0,06-0,2) (diamètre de la colonne: 3 cm, hauteur: 15 cm). On élue avec 500 $cm^3$ d'un mèlange acétate d'éthyle/cyclohexane 20-80 (en volumes) et recueille des fractions de 40 $cm^3$. Les fractions 7 à 10 sont concentrées à sec sous pression réduite (20 mm de mercure; 2,7 kPa) et on obtient ainsi 0,25 g d'un mélange d'acétonyl-3 t.butoxycarbonylamino-7 (méthoxy-4 benzyloxycarbonyl)-2 oxo-8 thia-5 aza-1 bicyclo[4.2.0] octène-2 et octène-3 (70-30) sous forme d'un solide ocre.

Rf = 0,27 Plaque de silice Merck $F_{254}$, épaisseur 0,25 mm, éluant: acétate d'éthyle/cyclohexane 40-60 (en volumes).

Spectre de masse: M = 476.

Spectre de RMN du proton (350 MHz, $CDCl_3$, δ en ppm, J en Hz) 1,47 [s, 9H, $-C(CH_3)_3$ de l'octène-2 et de l'octène-3]; 2,03 (s, $-CO-CH_3$ de l'octène-3); 2,12 (s, $-CO-CH_3$ de l'octène-2); 3,18 et 3,33 (2d, J = 17,5, $-CH_2$-CO- de l'octène-3); 3,30 et 3,55 (2d, J = 18, $-CH_2$-S- de l'octène-2); 3,51 et 3,73 (2d, J = 17,5, $-CH_2$-CO- de l'octène-2); 3,80 (s, $-OCH_3$ de l'octène-2); 3,82 (s, $-OCH_3$ de l'octène-3); 4,95 (d, J = 4,5, -H en 6 de l'octène-2 et s, -H en 2 de l'octène-3); 5,07 (s, $-COO-CH_2-$ de l'octène-3); 5,13 et 5,20 (2d, J = 12, $-COO-CH_2-$ de l'octène-2); 5,21 (d, J = 4, -H en 6 de l'octène-3); 5,33 (d, J = 9, -CO-NH- de l'octène-2); 5,42 (mf, -CO-NH- et -H en 7 de l'octène-3); 5,59 (dd, J = 9 et 4,5, -H en 7 de l'octène-2); 6,06 (s, -H en 4 de l'octène-3); 6,89 (mt, -H aromatiques en ortho du $-OCH_3$ de l'octène-2 et de l'octène-3); 7,28 (d, J = 7,5, -H aromatiques en méta du $-OCH_3$ de l'octène-3); 7,34 (d, J = 7,5, -H aromatiques en ortho du $-OCH_3$ de l'octène-2).

Exemple 4

A une solution de 5,38 g d'un mélange d'environ 50/50 de benzhydryloxycarbonyl-2 méthyl-3 oxo-8 tritylamino-7 thia-5 aza-1 bicyclo[4.2.0] octène-2 et octène-3 dans 25 $cm^3$ de diméthylformamide portée à 80°C, on ajoute, en 56 minutes, en maintenant la température à 80°C, 4 $cm^3$ de diméthylamino-1 méthoxy-1 éthylène. On obtient ainsi une solution d'un mélange de benzhydryloxycarbonyl-2 (diméthylamino-2 propène-1 yl-1)-3 oxo-8 tritylamino-7 thia-5 aza-1 bicyclo[4.2.0] octène-2 et octène-3 que l'on verse dans un mélange de 200 $cm^3$ d'eau glacée et 200 $cm^3$ d'acétate d'éthyle. La phase organique est lavée par 2 fois 200 $cm^3$ d'eau, séchée sur sulfate de sodium, filtrée en présence de noir décolorant et évaporée à sec sous pression réduite (20 mm de mercure; 2,7 kPa). Le résidu est chromatographié sur une colonne de 60 g de gel de silice Merck (0,06-0,2) (diamètre de la colonne: 3 cm, hauteur: 20 cm). On élue avec 1000 $cm^3$ d'un mélange acétate d'éthyle/cyclohexane 15-85 (en volumes) en recueillant des fractions de 60 $cm^3$. Les fractions 9 à 15 sont réunies et évaporées à sec sous pression réduite (20 mm de mercure; 2,7 kPa). On obtient ainsi 0,51 g d'un mélange environ 50/50 d'acétonyl-3 benzhydryloxycarbonyl-2 oxo-8 tritylamino-7 thia-5 aza-1 bicyclo[4.2.0] octène-2 et 3 sous forme d'un solide jaune.

Rf = 0,18 Plaque de silice Merck $F_{254}$, épaisseur 0,25 mm; éluant: acétate d'éthyle/cyclohexane 20-80 (en volumes).

Spectre de masse M = 664.

Spectre de RMN du proton (350 MHz, $CDCl_3$, δ en ppm, J en Hz) 1,88 (s, $-CO-CH_3$ de l'octène-3); 2 (s, $-CO-CH_3$ de l'octène-2); 3 et 3,18 (2d, J = 16, $-CH_2$-CO- de l'octène-3); 3,02 (d, J = 11, >NH de l'octène-3); 3,11 et 3,34 (2d, J = 18, $-CH_2$-S- de l'octène-2); 3,14 (d, J = 8, >NH de l'octène-2); 3,44 et 3,66 (2d, J = 17, $-CH_2$-CO- de l'octène-2); 4,23 (d, J = 3,5, -H en 6 de l'octène-3); 4,31 (d, J = 4,5, -H en 6 de l'octène-2); 4,58 (dd, J = 3,5 et 11, -H en 7 de l'octène-3); 4,72 (dd, J = 8 et 4,5, -H en 7 de l'octène-2); 5 (s, -H en 2 de l'octène-3); 5,89 (s, -H en 4 de l'octène-3); 6,70 [s, $-COOC\underline{H}(C_6H_5)_2$ de l'octène-3]; 6,85 (s, $-COOC\underline{H}(C_6H_5)_2$ de l'octène-2); 7,15 à 7,60 (mt, 25H aromatiques).

Exemple 5

A une solution de 2,58 g de benzhydryloxycarbonyl-2 diéthoxyphosphoramido-7 méthyl-3 oxo-8 thia-5 aza-1 bicyclo[4.2.0] octène-2 dans 10 $cm^3$ de diméthylformamide portée à 80°C, on ajoute, en 55 minutes en maintenant la température à 80°C, une solution de 2,3 $cm^3$ de diméthylamino-1 méthoxy-1 éthylène dans 4 $cm^3$ de diméthylformamide. On obtient ainsi une solution de benzhydryloxycarbonyl-2 diéthoxyphosphoramido-7 (diméthylamino-2 propène-1 yl-1)-3 oxo-8 thia-5 aza-1 bicyclo[4.2.0] octène-2 que l'on verse dans un mélange de 150 $cm^3$ d'eau glacée et 100 $cm^3$ d'acétate d'éthyle. La phase organique est lavée par 250 $cm^3$ d'eau, séchée sur sulfate de sodium, filtrée en présence de noir décolorant et évaporée à sec sous pression réduite (20 mm de mercure; 2,7 kPa). Le résidu est chromatographié sur une colonne de 30 g de gel de silice Merck (0,06-0,2) (diamètre de la colonne: 2,2 cm, hauteur: 18 cm). On élue avec 100 $cm^3$ d'un mélange acétate d'éthyle/cyclohexane 50-50 (en volumes) puis 300 $cm^3$ d'un mélange acétate d'éthyle/cyclohexane 70-30 (en volumes) en recueillant des fractions de 30 $cm^3$. Les fractions 9 à 12 sont réunies et évaporées à sec sous pression réduite (20 mm de mercure; 2,7 kPa). On obtient ainsi 0,31 g d'un mélange 75/25 d'acétonyl-3 benzhydryloxycarbonyl-2 diéthoxyphosphoramido-7 oxo-8 thia-5 aza-1 bicyclo[4.2.0] octène-2 et octène-3 sous forme d'un solide jaune.

Rf = 0,36 [plaque de silice Merck $F_{254}$, épaisseur 0,25 mm; éluant: acétate d'éthyle/cyclohexane 80-20 (en volumes)].

Spectre de RMN du proton (350 MHz, DMSO $d_6$, δ en ppm, J en Hz) 1,15 à 1,30 (mt, 6H, $-CH_3$ de l'octène-2 et de l'octène-3); 1,88 (s, $-CO-CH_3$ de l'octène-3); 1,96 (s, $-CO-CH_3$ de l'octène-2); 3,24 et 3,44 (2d, J = 17,5, $-CH_2$-CO- de l'octène-3);

3,40 et 3,60 (2d, J = 18,5 $-CH_2-S-$ de l'octène-2); 3,53 et 3,65 (2d, J = 17,5, $-CH_2-CO-$ de l'octène-2); 3,90 à 4,05 (mt, 4H, $-OCH_2-$ de l'octène-2 et de l'octène-3); 4,97 (dt, J = 11 et 4,5, -H en 7 de l'octène-3); 5,06 (d, J = 4,5, -H en 6 de l'octène-3); 5,08 (d, J = 4,5, -H en 6 de l'octène-2); 5,11 (s large, -H en 2 de l'octène-3); 5,16 (dt, J = 11 et 4,5, -H en 7 de l'octène-2); 6,24 (t, J = 11, >N-H de l'octène-2); 6,27 (t, J = 11, >N-H de l'octène-3); 6,40 (s large, -H en 4 de l'octène-3); 6,77 [s, $-COOC\underline{H}(C_6H_5)_2$ de l'octène-3]; 6,84 [s, $-COO-C\underline{H}(C_6H_5)_2$ de l'octène-2]; 7,25 à 7,55 (mt, 10H, aromatique de l'octène-2 et de l'octène-3).

Les exemples suivants montrent comment les produits de formule générale (I) (éventuellement préparés in situ) peuvent être utilisés pour la préparation des produits de formule générale (XV) ou (XVII).

Exemple de référence 1

A une solution de 345 g de benzhydryloxycarbonyl-2 t.butoxycarbonylamino-7 méthyl-3 oxo-8 thia-5 aza-1 bicyclo[4.2.0] octène-2 dans 1600 $cm^3$ de diméthylformamide anhydre à 80°C on ajoute goutte à goutte en 55 minutes 218 g de diméthylamino-1 méthoxy-1 éthylène. On obtient ainsi une solution de benzhydryloxycarbonyl-2 t.butoxycarbonylamino-7 (diméthylamino-2 propène-1 yl-1)-3 oxo-8 thia-5 aza-1 bicyclo[4.2.0] octène-2 que l'on verse dans un mélange agité de 2 litres d'eau distillée, 2 kg de glace et 2,5 litres d'acétate d'éthyle. La phase organique est décantée, lavée avec 3 fois 1 litre d'eau distillée, séchée sur sulfate de sodium et concentrée à sec sous pression réduite (20 mm de mercure; 2,7 kPa) à 30°C. Le résidu est partagé en 2 parties égales que l'on chromatographie séparément chacune sur une colonne de 1500 g de gel de silice Merck (0,06–0,2) (diamètre de la colonne: 8,5 cm; hauteur: 70 cm). On élue chaque colonne avec 20 litres de mélange acétate d'éthylecyclohexane 23–77 (volumes) en recueillant des fractions de 1 litre. On concentre à sec à 30°C sous pression réduite (20 mm de mercure; 2,7 kPa) les fractions 7 à 15 de chaque colonne. On obtient ainsi 149 g d'acétonyl-3 benzhydryloxycarbonyl-2 t.butoxycarbonylamino-7 oxo-8 thia-5 aza-1 bicyclo[4.2.0] octène-2 sous la forme d'une meringue jaune.

Spectre infra-rouge ($CHCl_3$), bandes caractéristiques ($cm^{-1}$) 3440, 1780, 1720, 1505, 1455, 1395, 1370, 1160, 695.

Spectre de RMN du proton (350 MHz, $CDCl_3$, $\delta$ en ppm, J en Hz) 1,48 [s, 9H, $-C(CH_3)_3$]; 2,10 (s, 3H, $CH_3CO-$); 3,29 et 3,62 (2d, J = 19, 2H, $-SCH_2-$); 3,57 et 3,67 (2d, J = 16, 2H, $-CH_2CO-$); 5,00 (d, J = 4, 1H, $H_6$); 5,23 (d, J = 9, 1H, -CONH-); 5,62 (dd, J = 4 et 9, 1H, $H_7$); 6,97 (s, 1H, -COOCH<).

L'acétonyl-3 benzhydryloxycarbonyl-2 t.butoxycarbonylamino-7 oxo-8 thia-5 aza-1 bicyclo[4,2.0] octène-2 peut être utilisé selon l'une ou l'autre des méthodes suivantes:

A. A une solution de 6,6 g de tétraméthyl-2,2,6,6 pipéridine dans 50 $cm^3$ de tétrahydrofuranne à 20°C on ajoute en 1 minute 26,5 $cm^3$ de solution 1,6 M de n.butyllithium dans l'hexane. On agite pendant 30 minutes à 20°C puis refroidit le mélange à −70°C. On ajoute goutte à goutte en 20 minutes une solution de 22,2 g d'acétonyl-3 benzhydryloxycarbonyl-2 t.butoxycarbonylamino-7 oxo-8 thia-5 aza-1 bicyclo[4.2.0] octène-2 dans 50 $cm^3$ de tétrahydrofuranne. On agite pendant 30 minutes à −70°C et ajoute 35 $cm^3$ d'hexaméthylphosphorotriamide en 3 minutes puis 10,7 $cm^3$ d'anhydride trifluorométhanesulfonique en 5 minutes. On agite pendant 45 minutes à −70°C puis laisse revenir à 0°C en 1 heure. Le mélange est alors dilué avec 500 $cm^3$ d'acétate d'éthyle et lavé successivement avec 650 $cm^3$ d'acide chlorhydrique 0,2N et 300 $cm^3$ d'eau distillée. La solution est séchée sur sulfate de sodium et concentrée à sec sous pression réduite (20 mm de mercure; 2,7 kPa) à 20°C. Le résidu est chromatographié sur une colonne de 300 g de gel de silice Merck (0,06–0,2) (diamètre de la colonne: 3,5 cm, hauteur: 70 cm). On élue avec 2 litres de mélange acétate d'éthyle/cyclohexane 20–80 (volumes) et recueille des fractions de 100 $cm^3$. On concentre à sec sous pression réduite (20 mm de mercure; 2,7 kPa) à 20°C les fractions 3 à 9. On obtient ainsi 6,3 g du mélange 66–34 (moles) des benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z sous forme d'une huile jaune.

Spectre infra-rouge ($CHCl_3$), bandes caractéristiques ($cm^{-1}$) 3440, 1790, 1720, 1505, 1455, 1420, 1370, 1155, 1140, 910, 700.

Spectre de RMN du proton (350 MHz, $CDCl_3$, $\delta$ en ppm, J en Hz) 1,47 [s, 9H, $-C(CH_3)_3$]; 1,94 (s, 3H, $CH_3-C=$ de l'octène-2); 1,99 (s, 3H, $CH_3-C=$ de l'octène-3); 3,43 et 3,72 (2d, J = 18, 2H, $-SCH_2-$); 5,00 (d, J = 4, $H_6$ octène-2); 5,04 (s, $H_2$ de l'octène-3); 5,26 (d, J = 4, $H_6$ octène-3); 5,27 (d, J = 9, -CONH- octène-2); 5,35 à 5,48 (m, 2H, $H_7$ et -CONH- de l'octène-3); 5,56 (s, $H_4$ de l'octène-3); 5,65 (dd, J = 4 et 9, $H_7$ de l'octène-2); 6,17 (s, 1H, -CH=C- de l'octène-2); 6,65 (s, 1H, -CH=C- de l'octène-3); 6,87 (s, 1H, -COOCH< octène-3); 6,95 (s, 1H, -COOCH< de l'octène-2).

Le mélange des benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z peut être utilisé selon l'une ou l'autre des méthodes suivantes:

$A_1$. On ajoute 1,53 g de sel de sodium de mercapto-5 méthyl-2 thiadiazole-1,3,4 à une solution de 5,9 g de mélange 66–34 des benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z dans 100 $cm^3$ de diméthylformamide à 20°C. On

agite pendant 1 heure 10 minutes à 20°C puis dilue la solution avec 500 $cm^3$ d'eau. On extrait avec 300 $cm^3$ d'acétate d'éthyle, sèche la phase organique sur sulfate de sodium, concentre à sec à 20°C sous pression réduite (20 mm de mercure; 2,7 kPa) et chromatographie le résidu sur une colonne de 100 g de gel de silice Merck (0,06–0,2) (diamètre de la colonne: 3,5 cm; hauteur: 25 cm). On élue avec 1 litre de mélange acétate d'éthyle/cyclohexane 35–65 (volumes) et recueille des fractions de 60 $cm^3$. On concentre à sec à 20°C les fractions 8 à 14 sous pression réduite (20 mm de mercure; 2,7 kPa). On obtient ainsi 1,35 g de mélange 40–60 de benzhydryloxycarbonyl-2 t.butoxycarbonylamino-7 {[(méthyl-2 thiadiazol-1,3,4 yl-5) thio]-2 propène-1 yl-1}-3 oxo-8 thia-5 aza-1 bicyclo[4.2.0] octène-2 et octène-3, forme Z sous forme d'une meringue jaune.

Spectre infra-rouge ($CHCl_3$), bandes caractéristiques ($cm^{-1}$) 3440, 1780, 1720, 1505, 1455, 1390, 1370, 1160.

Spectre de RMN du proton (350 MHz, $CDCl_3$, δ en ppm, J en Hz) 1,48 [s, 9H, $-C(CH_3)_3$]; 2,06 (s, large, $CH_3-C=$, octène-2 et -3); 2,74 (s, $CH_3$-Het de l'octène-2); 2,76 (s, $CH_3$-H et de l'octène-3); 3,66 et 3,76 (2d, J = 18, $-SCH_2-$ octène-3); 5,04 (d, J = 4, $H_6$ octène-2); 5,19 (s, $H_2$ octène-3); 5,25 (d, J = 4, $H_6$ octène-3); 5,31 (d, J = 8, -CONH- octène-2); 5,36 à 5,46 (m, $H_7$ et -CONH- octène-3); 5,66 (dd, J = 4 et 8, $H_7$ octène-2); 6,18 (s, $H_4$ octène-3); 6,58 (s, $-CH=C$ octène-3); 6,64 (s, $-CH=C$ octène-2); 6,90 (s, -COOCH octène-3); 6,97 (s, $-COOCH<$ octène-2).

On refroidit à 0°C une solution de 0,22 g de benzhydryloxy-carbonyl-2 t.butoxycarbonylamino-7{[(methyl-2 thiadiazol-1,3,4 yl-5) thio]-2 propène-1 yl-1}-3 oxo-8 thia-5 aza-1 bicyclo [4.2.0] octène-2 et octène-3, forme Z dans 10 $cm^3$ de dichlorométhane. On ajoute goutte à goutte en 20 minutes une solution de 0,07 g d'acide m.chloroperbenzoïque à 85% dans 5 $cm^3$ de dichlorométhane. On agite ensuite la solution pendant 5 minutes à 0°C, lave avec 20 $cm^3$ de solution saturée de bicarbonate de sodium, sèche sur sulfate de sodium et concentre à sec sous pression réduite (20 mm de mercure; 2,7 kPa) à 20°C. Le résidu est chromatographié sur une colonne de 4 g de gel de silice Merck (0,06–0,2) (diamètre de la colonne:1 cm; hauteur: 10 cm). On élue avec 100 $cm^3$ de mélange acétate d'éthyle/cyclohexane 60–40 (volumes) et receuille des fractions de 3 $cm^3$. On concentre à sec sous pression réduite (20 mm de mercure; 2,7 kPa) à 20°C les fractions 11 à 22. On obtient ainsi 0,12 g de benzydryloxycarbonyl-2 t.butoxycarbonylamino-7 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z sous forme d'une meringue blanche.

Spectre infra-rouge ($CHCl_3$), bandes caractéristiques ($cm^{-1}$) 3420, 1800, 1720, 1505, 1455, 1370, 1160, 1045, 695.

Spectre de RMN du proton (350 MHz, $CDCl_3$, δ en ppm, J en Hz) 1,48 [s, 9H, $-C(CH_3)_3$]; 2,05 (s, 3H, $-CH_3$); 2,72 (s, 3H, $CH_3$-Hétérocycle); 3,70 et 4,03 (2d, J = 18, 2H, $-SCH_2-$); 4,58 (d, J = 4, 1H, $H_6$); 5,77 (d, J = 8, 1H, -CONH-); 5,85 (dd, J = 4 et 9, 1H, $H_7$); 6,75 (s, 1H, -CH=); 6,98 (s, $-COOCH<$).

On chauffe à 40°C une solution de 4,45 g de benzhydryloxycarbonyl-2 t.butoxycarbonylamino-7 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z dans 150 $cm^3$ d'acétonitrile. On ajoute goutte à goutte en 5 minutes une solution de 2,59 g d'acide p.toluènesulfonique (monohydraté) dans 75 $cm^3$ d'acétonitrile. Le mélange est agité à 40°C pendant 35 minutes puis versé dans 200 $cm^3$ de solution aqueuse saturée de bicarbonate de sodium. On ajoute 500 $cm^3$ d'eau distillée et extrait avec 500 $cm^3$ de dichlorométhane. La phase organique est lavée avec 500 $cm^3$ d'eau distillée, séchée sur sulfate de magnésium et concentrée à sec sous pression réduite (20 mm de mercure; 2,7 kPa) à 20°C. On obtient ainsi 3,53 g d'amino-7 benzhydryloxycarbonyl-2 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z sous la forme d'une meringue brune.

Spectre infra-rouge ($CHCl_3$), bandes caractéristiques ($cm^{-1}$) 3420, 1800, 1725, 1500, 1455, 1370, 1160, 1050, 695.

On refroidit à 4°C une solution de 3,53 g d'amino-7 benzhydryloxycarbonyl-2 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z dans 250 $cm^3$ de dichlorométhane. On ajoute successivement 3,28 g d'acide méthoxyimino-2 (tritylamino-2 thiazolyl-4)-2 acétique et 1,54 g de dicyclohexylcarbodiimide. On agite le mélange pendant 1 heure ½ à 4°C puis concentre à sec sous pression réduite (20 mm de mercure; 2,7 kPa) à 20°C. Le résidu est repris avec 50 $cm^3$ d'acétate d'éthyle et l'insoluble est séparé par filtration. La solution est lavée avec 100 $cm^3$ de solution aqueuse saturée de bicarbonate de sodium puis avec 100 $cm^3$ d'acide chlorhydrique 0,1N. On sèche sur sulfate de sodium et concentre à sec sous pression réduite (20 mm de mercure; 2,7 kPa) à 20°C. Le résidu est chromatographié sur une colonne de 80 g de gel de silice Merck (0,06–0,2) (diamètre de la colonne: 3 cm; hauteur: 64 cm). On élue avec 300 $cm^3$ d'un mélange acétate d'éthyle/cyclohexane 35–65 (en volumes) puis avec 1200 $cm^3$ du mélange acétate d'éthyle/cyclohexane 70–30 (en volumes) en recueillant des fractions de 60 $cm^3$. On concentre à sec les fractions 12 à 20 sous pression réduite (20 mm de mercure; 2,7 kPa) à 20°C. On obtient ainsi 2 g de benzhydryloxycarbonyl-2 {[(méthyl-2 thiadiazol-1,3,4 yl-5 thio]-2 propène-1 yl-1}-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme Z sous la forme d'une meringue blanche.

Spectre infra-rouge (KBr), bandes caractéristiques ($cm^{-1}$) 3420,1800,1730, 1685, 1530, 1495, 1450, 1040, 755, 705.

Spectre de RMN du proton (350 MHz, $CDCl_3$, δ en ppm, J en Hz) 2,05 (s, 3H, $CH_3$-C=); 2,72 (s, 3H, $CH_3$-hétérocycle); 3,71 et 4,06 (2d, J = 18, 2H, $-SCH_2-$); 4,12 (s, 3H, $=NOCH_3$); 4,61 (d, J = 5, 1H, $H_6$); 6,21 (dd, J = 5 et 9, 1H, $H_7$); 6,73 (s, 1H, -CH=); 6,76 (s, 1H, H sur thiazole); 6,97 (s, 1H,-COOCH ); 7,14 (s large, 1H, -NH trityle); 7,44 (d, J = 9, 1H, -CONH-).

Une solution de 2,52 g de benzyhydryloxycarbonyl-2{[(méthyl-2 thiadiazol-1,3,4 yl-5 thio]-2 propène-1 yl-1}-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme Z dans 40 cm³ de dichlorométhane est refroidie à −10°C. On ajoute successivement 0,95 cm³ de N,N-diméthylacétamide et 0,7 g de trichlorure de phosphore. La solution est agitée pendant 20 minutes à −10°C puis versée dans 50 cm³ de solution aqueuse saturée de bicarbonate de sodium. On dilue avec 200 cm³ d'eau distillée et extrait avec 300 cm³ d'acétate d'éthyle. La phase organique est séchée sur sulfate de sodium et concentrée à sec sous pression réduite (20 mm de mercure; 2,7 kPa) à 20°C. Le résidu est chromatographié sur une colonne de 25 g de gel de silice Merck (0,06–0,20) (diamètre de la colonne: 2 cm; hauteur: 15 cm). On élue avec 500 cm³ de mélange acétate d'éthyle/cyclohexane 50–50 (en volumes) en recueillant des fractions de 30 cm³. On concentre à sec les fractions 4 à 13 sous pression réduite (20 mm de mercure; 2,7 kPa) à 20°C. On obtient ainsi 0,77 g de benzhydryloxycarbonyl-2 {[(méthyl-2 thiadiazol-1,3,4 yl-5) thio]-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z sous la forme d'une meringue blanche.

Spectre infra-rouge (KBr), bandes caractéristiques ($cm^{-1}$)1790, 1725, 1685, 1525, 1495, 1450, 1050, 750, 700.

Spectre de RMN du proton (350 MHz, $CDCl_3$, δ en ppm, J en Hz) 2,02 (s, 3H, $CH_3$-C=); 2,7 (s, 3H, $CH_3$- hétérocycle); 3,63 et 3,74 (2d, J = 18, 2H, $-SCH_2-$); 4,07 (s, 3H, $=NOCH_3$); 5,09 (d, J = 4, 1H, $H_6$); 5,94 (dd, J = 4 et 9, 1H, $H_7$); 6,6 (s, 1H, -CH=); 6,76 (s, 1H, H thiazole); 6,84 (d, J = 9, 1H, -CONH-); 6,94 (s, 1H, -COOCH<); 7,04 (s large, 1H, -NH- trityle).

On dissout 0,76 g de benzhydryloxycarbonyl-2 {[(méthyl-2thiadiazol-1,3,4 yl-5) thio]-2 propène-1 yl-1}-3 [méthoxyimino-2(tritylamino-2 thiazolyl-4)-2 acétamido]-7 oxo-8 thia-5 aza-1 bicyclo[4.2.0] octène-2, isomère syn, forme Z dans 8 cm³ d'acide formique. On chauffe la solution à 50°C et ajoute goutte à goutte 4 cm³ d'eau distillée en 10 minutes. On agite pendant 30 minutes à 50°C puis ajoute 4 cm³ d'eau distillée, refroidit, filtre et concentre la solution à sec sous pression réduite (1 mm de mercure; 0,13 kPa) à 40°C. On ajoute 40 cm³ d'éthanol qu'on évapore sous pression réduite (1 mm de mercure; 0,13 kPa) à 30°C et on répète encore 2 fois cette opération. On reprend le résidu avec 10 cm³ d'éthanol, filtre, lave le gâteau avec 20 cm³ d'éther diéthylique, sèche sur sulfate de sodium et évapore le solvant sous pression réduite (1 mm de mercure; 0,13 kPa) à 20°C. On isole ainsi 60 mg d'[(amino-2 thiazolyl-4)-2 méthoxyimino-2 acétamido]-7 carboxy-2 {[(méthyl-2 thiadiazol-1,3,4 yl-5) thio]-2 propène-1 yl-1}-3 oxo-8 thia-5 aza-1 bicyclo[4.2.0] octène-2, isomère syn, forme Z sous forme d'une poudre jaune.

Spectre infra-rouge (KBr), bandes caractéristiques ($cm^{-1}$) 3600, 2600, 2700, 1670, 1625, 1535, 1365, 1035

Spectre de RMN du proton (350 MHz, DMSO $d_6$, δ en pm, J en Hz) 2,24 (s large, =C-$CH_3$); 2,75 (s, -$CH_3$ thiadiazole); 3,87 (s, $=N-OCH_3$); 4,51 (d, J = 14, 1H des -H du $-S-CH_2-$); 5,44 (dd, J = 5 et 9, -H en 7); 5,62 (d, J = 5, -H en 6); 6,75 (s, -H thiazole); 7,07 (s, -CH=); 7,20 (s large, $-NH_2$); 9,55 (d, J = 9, -CO-NH-).

$A_2$. A une solution de 0,42 g de benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z dans 10 cm³ de dichlorométhane à 0°C on ajoute en 20 minutes une solution de 0,13 g d'acide m.chloroperbenzoïque à 85% dans 5 cm³ de dichlorométhane. On agite à 20°C pendant 10 heures puis lave la solution avec 20 cm³ de solution saturée de bicarbonate de sodium et sèche sur sulfate de sodium. On concentre à sec sous pression réduite (20 mm de mercure; 2,7 kPa) à 20°C et chromatographie le résidu sur une colonne de 6 g de gel de silice Merck 0,06–0,20) (diamètre de la colonne: 1 cm; hauteur: 12 cm). On élue avec 100 cm³ de mélange acétate d'éthyle/cyclohexane 30–70 (en volumes) en recueillant des fractions de 3 cm³. On concentre à sec les fractions 8 à 18 sous pression réduite (20 mm de mercure; 2,7 kPa) à 20°C. On obtient ainsi 0,14 g de benzhydryloxycarbonyl-2 t.butoxycarbonylamino-7 oxo-8 oxyde-5 (trifluorométhanesulfonyl-oxy-2 propène-1 yl-1)-3 thia-5 aza-1 bicyclo[4.2.0] octène-2 sous forme de meringue blanche.

Spectre infra-rouge ($CHBr_3$), bandes caractéristiques ($cm^{-1}$) 3420, 1810, 1720, 1505, 1455, 1420, 1220, 1040.

Spectre de RMN du proton (350 MHz, $CDCl_3$, δ en ppm, J en Hz) 1,50 [s, 9H, $-C(CH_3)_3$]; 1,98 (s, 3H, $CH_3$-C=); 3,52 et 3,82 (2d, J = 18, 2H, $-SCH_2-$); 4,59 (d, J = 4, 1H, $H_6$); 5,73 (d, J = 8, 1H, -CONH-); 5,86 (dd, J = 4 et 8, 1H, $H_7$); 6,44 (s, 1H, -CH=); 6,98 (s, 1H, -COOCH<).

B. A une solution de 1,28 g de tétraméthyl-2,2,6,6 pipéridine dans 10 cm³ de tétrahydrofuranne à 20°C on ajoute en 2 minutes 5,37 cm³ d'une solution 1,6M de n.butyllithium dans l'hexane. Le mélange est agité pendant 1 heure à 20°C puis refroidi à −70°C. On ajoute alors goutte à goutte en 20 minutes une solution de 4,5 g

d'acétonyl-3 benzhydryloxycarbonyl-2 t.butoxycarbonylamino-7 oxo-8 thia-5 aza-1 bicyclo[4.2.0] octène-2 dans 10 $cm^3$ de tétrahydrofuranne puis agite la solution pendant 30 minutes à −70°C. On ajoute alors 7 $cm^3$ d'hexaméthylphosphorotriamide en 3 minutes puis 4,34 $cm^3$ d'anhydride trifluorométhanesulfonique en 3 minutes. On agite encore pendant 40 minutes à −70°C puis laisse revenir à 0°C en 40 minutes. Le mélange est alors versé dans un mélange agité de 125 $cm^3$ d'acide chlorhydrique 0,25N et de 50 $cm^3$ d'acétate d'éthyle. La phase organique est décantée, lavée avec 50 $cm^3$ de solution aqueuse saturée de bicarbonate de sodium, séchée sur sulfate de sodium et concentrée à sec sous pression réduite (20 mm de mercure; 2,7 kPa) à 20°C. Le résidu est chromatographié sur une colonne de 90 g de gel de silice Merck (0,06–0,20) (diamètre de la colonne: 2,4 cm; hauteur: 45 cm). On élue avec 1 litre de mélange acétate d'éthyle/cyclohexane 20–80 (en volumes) et recueille des fractions de 100 $cm^3$. On concentre à sec les fractions 2 à 7 sous pression réduite (20 mm de mercure; 2,7 kPa) à 20°C. On obtient ainsi 2 g de benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2, forme Z sous forme d'une meringue jaune.

Spectre infra-rouge ($CHCl_3$), bandes caractéristiques ($cm^{-1}$) 3440, 1790, 1720, 1505, 1455, 1420, 1370, 1155, 1140, 910, 700.

Spectre de RMN du proton (350 MHz, $CDCl_3$, δ en ppm, J en Hz) 1,47 [s, 9H, $-C(CH_3)_3$]; 1,94 (s, 3H, $CH_3$-C=C-);

3,43 et 3,72 (2d, J = 18, 2H, $-SCH_2-$); 5,00 (d, J = 4, $H_6$); 5,27 (d, J = 9, -CONH-); 5,65 (dd, J = 4 et 9, $H_7$); 6,17 (s, 1H, -CH=C-);

6,95 (s, 1H, -COOCH<).

C. A une solution de 0,78 g de tétraméthyl-2,2,6,6 pipéridine dans 5 $cm^3$ de tétrahydrofuranne à 20°C on ajoute en 10 minutes 3,44 $cm^3$ de solution 1,6 M de butyllithium dans l'hexane. On agite la solution pendant 10 minutes à 20°C puis on refroidit à −70°C. On ajoute alors en 20 minutes une solution de 2,54 g d'acétonyl-3 benzhydryloxycarbonyl-2 t.butoxycarbonylamino-7 oxo-8 thia-5 aza-1 bicyclo[4.2.0] octène-2 dans 6 $cm^3$ de tétrahydrofuranne puis on agite pendant 15 minutes à −70°C. On ajoute successivement 7 $cm^3$ d'hexaméthylphosphorotriamide en 3 minutes et une solution de 5,23 g de chlorure de p.toluènesulfonyle dans 7 $cm^3$ de tétrahydrofuranne en 3 minutes. On agite pendant 45 minutes à −70°C puis laisse remonter la température jusqu'à 0°C, dilue la solution avec 100 $cm^3$ d'acétate d'éthyle et lave successivement avec 50 $cm^3$ de solution 0,1N d'acide citrique, 50 $cm^3$ de solution aqueuse saturée de bicarbonate de sodium et 100 $cm^3$ d'eau distillée. La phase organique est séchée sur sulfate de sodium et concentrée à sec sous pression réduite (20 mm de mercure; 2,7 kPa) à 20°C. Le résidu est chromatographié sur une colonne de 110 g de gel de silice Merck (0,06–0,20) (diamètre de la colonne: 3 cm; hauteur: 26 cm). On élue avec 1200 $cm^3$ d'un mélange acétate d'éthyle/cyclohexane 15–80 (en volumes) en recueillant des fractions de 60 $cm^3$. On concentre à sec les fractions 3 à 15 sous pression réduite (20 mm de mercure; 2,7 kPa) à 20°C. On obtient ainsi 0,94 g de benzhydryloxycarbonyl-2 t. butoxycarbonylamino-7 (tosyloxy-2 propène-1 yl-1)-3 oxo-8 thia-5 aza-1 bicyclo[4.2.0] octène-2, forme Z sous forme d'une meringue jaune pâle.

Spectre infra-rouge ($CHBr_3$), bandes caractéristiques ($cm^{-1}$) 3420, 1785, 1720, 1595, 1500, 1455, 1370, 815, 530.

Spectre de RMN du proton (350 MHz, $CDCl_3$, δ en ppm, J en Hz) 1,5 [s, 9H, $-C(CH_3)_3$]; 2,0 (s, 3H, $CH_3$-C=);

2,35 (s, 3H, $-CH_3$ tosyle); 3,51 (s large, 2H, $-SCH_2-$); 4,78 (d, J = 4, 1H, $H_6$); 5,92 (dd, J = 4 et 9, 1H, $H_7$); 6,08 (s, 1H, -CH=); 6,93 (s, 1H, -COOCH<).

D. A une solution de 2,33 g de tétraméthyl-2,2,6,6 pipéridine dans 15 $cm^3$ de tétrahydrofuranne à 20°C, on ajoute en 10 minutes 10,3 $cm^3$ de solution 1,6 M de butyllithium dans l'hexane. On agite pendant 20 minutes à 20°C puis on refroidit à −70°C. On ajoute alors en 15 minutes une solution de 7,61 g d'acétonyl-3 benzhydryloxycarbonyl-2 t.butoxycarbonylamino-7 oxo-8 thia-5 aza-1 bicyclo[4.2.0] octène-2 dans 20 $cm^3$ de tétrahydrofuranne. On agite encore pendant 20 minutes à −70°C puis on ajoute 15 $cm^3$ d'hexaméthylphosphorotriamide en 3 minutes et ensuite une solution de 4,5 $cm^3$ de chlorure de méthanesulfonyle dans 10 $cm^3$ de tétrahydrofuranne en 3 minutes. On agite pendant 45 minutes à −70°C puis laisse remonter la température à 0°C et dilue le mélange avec 300 $cm^3$ d'acétate d'éthyle. La solution est lavée successivement avec 100 $cm^3$ de solution saturée de bicarbonate de sodium et 100 $cm^3$ d'eau distillée, séchée sur sulfate de sodium et concentrée à sec sous pression réduite (20 mm de mercure; 2,7 kPa) à 20°C. Le résidu est chromatographié sur une colonne de 150 g de gel de silice Merck (0,06–0,20) (diamètre de la colonne: 3,7 cm; hauteur: 30 cm). On élue avec 2,5 litres de mélange acétate d'éthyle/cyclohexane 22–78 (en volumes) en recueillant des fractions de 100 $cm^3$. On concentre à sec sous pression réduite (20 mm de mercure; 2,7 kPa) à 20°C les fractions 12 à 20. On obtient ainsi 3,2 g de benzhydryloxycarbonyl-2 t.butoxycarbonylamino-7 (mésyloxy-2 propène-1 yl-1)-3 oxo-8 thia-5 aza-1 bicyclo[4.2.0] octène-2, forme Z sous forme d'une meringue jaune pâle.

Spectre infra-rouge ($CHCl_3$), bandes caractéristiques ($cm^{-1}$) 3430, 1790, 1720, 1610, 1500, 1455, 1375, 1155, 970.

Spectre de RMN du proton (350 MHz, $CDCl_3$, δ en ppm, J en Hz) 1,50 [s, 9H, $-C(CH_3)_3$]; 1,80 (s, 3H, $CH_3$-C=);

3,15 (s, 3H, $-OSO_2CH_3$); 3,43 et 3,77 (2d, J = 18, 2H, $-SCH_2-$); 5,06 (s, J = 4, 1H, $H_6$); 5,38 (d, J = 9, 1H, -CONH-); 5,74 (dd, J = 4 et 9, 1H, $H_7$); 6,97 (s, 1H, -COOCH<).

Une solution de 1 g de benzhydryloxycarbonyl-2 t.butoxycarbonylamino-7 (mésyloxy-2 propène-1 yl-1)-3 oxo-8 thia-5 aza-1 bicyclo[4.2.0] octène-2, forme Z dans 12 $cm^3$ d'acétonitrile est chauffée à 38°C. On ajoute en 20 minutes une solution de 0,63 g de monohydrate d'acide p.toluènesulfonique dans 10 $cm^3$ d'acétonitrile puis on maintient le mélange à 38°C pendant 1 heure. On dilue avec 30 $cm^3$ de dichlorométhane, lave la solution obtenue avec 20 $cm^3$ de solution saturée de bicarbonate de sodium puis avec 50 $cm^3$ d'eau distillée, sèche sur sulfate de sodium et concentre jusqu'à un volume de 15 $cm^3$ sous pression réduite (20 mm de mercure; 2,7 kPa) à 20°C. On obtient ainsi une solution d'amino-7 benzhydryloxycarbonyl-2 (mésyloxy-2 propène-1 yl-1)-3 oxo-8 thia-5 aza-1 bicyclo[4.2.0] octène-2, forme Z dans laquelle on ajoute 0,7 $cm^3$ de triéthylamine (Solution A).

A une suspension de 0,8 g d'acide méthoxyimino-2 (tritylamino-2 thiazolyl-4)-2 acétique, isomère syn dans 12 $cm^3$ de dichlorométhane refroidie à −10°C, on ajoute successivement 0,16 $cm^3$ de N,N-diméthylacétamide et 0,81 $cm^3$ de solution benzénique de phosgène (2,5 M) en 20 minutes. On agite encore pendant 16 heures à −10°C puis on ajoute la solution A goutte à goutte en 25 minutes. On agite la solution pendant 2 heures à −10°C puis on dilue avec 50 $cm^3$ d'acétate d'éthyle et lave avec 50 $cm^3$ d'acide chlorhydrique 0,1 N, 50 $cm^3$ de solution saturée de bicarbonate de sodium puis 50 $cm^3$ d'eau distillée. On sèche sur sulfate de sodium et concentre à sec sous pression réduite (20 mm de mercure; 2,7 kPa) à 20°C. Le résidu est chromatographié sur une colonne de 10 g de gel de silice Merck (0,06–0,2) (diamètre de la colonne: 1,5 cm; hauteur: 12 cm). On élue avec 700 $cm^3$ de mélange acétate d'éthyle/cyclohexane 25–75 (en volumes) en recueillant des fractions de 25 $cm^3$. On concentre à sec les fractions 10 à 21 sous pression réduite (20 mm de mercure; 2,7 kPa) à 20°C. On obtient ainsi 0,43 g de benzhydryloxycarbonyl-2 (mésyloxy-2 propène-1 yl-1)-3 [méthoxyimino-2 (tritylamino-2 thiazolyl-4)-2 acétamido]-7 oxo-8 thia-5 aza-1 bicyclo[4.2.0] octène-2, isomère syn, forme E sous forme d'une meringue jaune.

Spectre de RMN du proton (350 MHz, $CDCl_3$, δ en ppm, J en Hz) 1,8 (s, $=\underset{|}{C}-CH_3$);

3,18 (s, $-OSO_2-CH_3$); 3,42 et 3,78 (2d, J = 18, $-S-CH_2-$); 4,08 (s, $=N-OCH_3$); 5,13 (d, J = 5, -H en 6); 6,09 (dd, J = 5 et 9, -H en 7); 6,86 (d, J = 9, -CO-NH-); 6,95 [s, $-COO-\underline{H}C(C_6H_5)_2$]; 7,05 [mf, $-N\underline{H}-C(C_6H_5)_3$]; 7,2 à 7,5 (mt aromatiques).

Les produits obtenus en $A_2$, B, C et D peuvent être utilisés pour préparer un produit de formule générale (XVII), par analogie avec la méthode décrite en $A_1$.

Exemple de référence 2

A une solution de 20,9 g d'acétonyl-3 benzhydryloxycarbonyl-2 t.butoxycarbonylamino-7 oxo-8 thia-5 aza-1 bicyclo[4.2.0] octène-2 dans 400 $cm^3$ d'acétonitrile à 38°C on ajoute en 20 minutes une solution de 15,2 g de monohydrate d'acide p.toluènesulfonique dans 200 $cm^3$ d'acétonitrile. Le mélange est maintenu à 40°C pendant 25 minutes puis concentré à sec sous pression réduite (20 mm de mercure; 2,7 kPa) à 40°C. Le résidu est repris avec 500 $cm^3$ d'acétate d'éthyle, 500 $cm^3$ d'eau distillée et 150 $cm^3$ de solution aqueuse saturée de bicarbonate de sodium. La phase organique est séparée par décantation, lavée avec 200 $cm^3$ d'eau distillée, séchée sur sulfate de sodium puis concentrée à sec sous pression réduite (20 mm de mercure; 2,7 kPa) à 30°C. On obtient ainsi 12,5 g d'acétonyl-3 amino-7 benzhydryloxycarbonyl-2 oxo-8 thia-5 aza-1 bicyclo[4.2.0] octène-2 sous la forme d'une huile orangée.

Spectre infra-rouge ($CHCl_3$), bandes caractéristiques ($cm^{-1}$) 3430, 1770, 1720, 1495, 1450, 1245, 755, 740.

Spectre de RMN du proton (350 MHz, $CDCl_3$, δ en ppm, J en Hz) 2,02 (s, 3H, $-CH_3$); 3,25 et 3,50 (2d, J = 18, 2H, $-SCH_2-$); 3,55 et 3,66 (2d, J = 17, 2H, $-CH_2-$); 4,74 (d, J = 4, 1H, $H_7$); 4,96 (d, J = 4, 1H, $H_6$); 6,86 (s, 1H, -COCH<).

L'acétonyl-3 amino-7 benzhydryloxycarbonyl-2 oxo-8 thia-5 aza-1 bicyclo[4.2.0] octène-2 peut être utilisé selon l'une ou l'autre des méthodes suivantes:

A. A une solution de 11,5 g d'acétonyl-3 amino-7 benzhydryloxycarbonyl-2 oxo-8 thia-5 aza-1 bicyclo[4.2.0] octène-2 dans 115 $cm^3$ de dichlorométhane à 20°C, on ajoute successivement 12 g d'acide méthoxyimino-2 (tritylamino-2 thiazolyl-4)-2 acétique, isomère syn, 30 mg de diméthylamino-4 pyridine et 6,72 g de dicyclohexylcarbodiimide. On agite le mélange pendant 3 heures à 20°C puis concentre à sec sous pression réduite (20 mm de mercure; 2,7 kPa) à 20°C. Le résidu est repris avec 300 $cm^3$ d'acétate d'éthyle et l'insoluble est éliminé par filtration. La solution est lavée avec 100 $cm^3$ d'eau distillée puis avec 50 $cm^3$ de solution saturée de bicarbonate de sodium. On sèche sur sulfate de sodium et concentre à sec sous pression réduite (20 mm de mercure; 2,7 kPa) à 30°C. Le résidu est chromatographié sur une colonne de 250 g de gel de silice Merck (0,06–0,20) (diamètre de la colonne: 4,5 cm); hauteur: 32 cm). On élue avec 3 litres de mélange acétate d'éthyle/cyclohexane 40–60 (en volumes) en recueillant des fractions de 200 $cm^3$. On concentre à sec les fractions 9 à 11 sous pression réduite (20 mm de mercure; 2,7 kPa) à 20°C. On obtient ainsi 7 g de mélange 80–20 des isomères acétonyl-3 benzhydryloxycarbonyl-2 [méthoxyimino-2 (tritylamino-2 thiazolyl-4)-2 acétamido]-7 oxo-8 thia-5 aza-1 bicyclo[4.2.0] octène-2 et -3, isomère syn sous forme d'une meringue beige.

Spectre infra-rouge ($CHCl_3$), bandes caractéristiques ($cm^{-1}$) 3410, 1785, 1725, 1685, 1595, 1585, 1495, 1450, 695.

Spectre de RMN du proton (350 MHz, $CDCl_3$, δ en ppm, J en Hz) 2,06 (s, $CH_3CO$- de l'octène-3); 2,09 (s, $CH_3CO$- de l'octène-2); 3,30 et 3,52 (2d, J = 18, $-SCH_2-$); 3,6 (s large, $-CH_2CO-$ de l'octène-2 et −3); 4,07 (s, $CH_3ON$= octène-3); 4,08 (s, $CH_3ON$= octène-2); 5,10 (d, J = 4, $H_6$ octène-2); 5,15 (s, $H_2$ octène-3); 5,30 (d, J = 4, $H_6$ octène-3); 5,75 (dd, J = 4 et 9, $H_7$ octène-3); 5,95 (dd, J = 4 et 9, $H_7$ octène-2); 6,74 (s, H du thiazole, octène-3); 6,77 (s, H du thiazole, octène-2); 6,83 (d, J = 9, -CONH- octène-2); 6,84 (s, -COOCH< octène-3); 6,87 (s, -COOCH< octène-2); 6,88 (d, J = 4, -CONH- octène-3); 7,01 (s large, -NH- trityle octènes-2 et -3).

A une solution refroidie à 0°C de 6,7 g du mélange des isomères de l'acétonyl-3 benzhydryloxycarbonyl-2 [méthoxyimino-2 (tritylamino-2 thiazolyl-4)-2 acétamido]-7 oxo-8 thia-5 aza-1 bicyclo[4.2.0] octène-2 et -3 dans 100 cm³ de dichlorométhane, on ajoute en 20 minutes une solution de 1,6 g d'acide m.chloroperbenzoïque à 85% dans 50 cm³ de dichlorométhane. On agite pendant 1 heure ½ à 0°C puis ajoute 50 cm³ de solution saturée de bicarbonate de sodium. La phase organique est décantée, séchée sur sulfate de sodium et concentrée à sec sous pression réduite (20 mm de mercure; 2,7 kPa) à 20°C. Le résidu est chromatographié sur une colonne de 75 g de gel de silice Merck (0,06–0,20) (diamètre de la colonne: 3 cm; hauteur: 21 cm). On élue avec 1,5 litre de mélange acétate d'éthyle/cyclohexane 70–30 (en volumes) en recueillant des fractions de 100 cm³. On concentre à sec les fractions 7 à 10 sous pression réduite (20 mm de mercure; 2,7 kPa) à 20°C. On obtient ainsi 5 g d'acétonyl-3 benzhydryloxycarbonyl-2 [méthoxyimino-2 (tritylamino-2 thiazolyl-4)-2 acétamido]-7 oxo-8 oxyde-5 thia-5 aza-1 bicyclo[4.2.0] octène-2, isomère syn sous forme d'une meringue jaune.

Une solution de 5 g d'acétonyl-3 benzhydryloxycarbonyl-2 [méthoxyimino-2 (tritylamino-2 thiazolyl-4)-2 acétamido]-7 oxo-8 oxyde-5 thia-5 aza-1 bicyclo[4.2.0] octène-2 dans un mélange de 60 cm³ de dichlorométhane et de 2,3 cm³ de N,N-diméthylacétamide est refroidie à −10°C. On ajoute alors 1,01 cm³ de trichlorure de phosphore et agite pendant une heure à −10°C. Le mélange réactionnel est dilué avec 300 cm³ d'acétate d'éthyle, lavé successivement avec 50 cm³ de solution saturée de bicarbonate de sodium et 300 cm³ d'eau distillée, séché sur sulfate de sodium et concentré à sec sous pression réduite (20 mm de mercure; 2,7 kPa) à 20°C. On obtient ainsi 4,5 g d'acétonyl-3 benzhydryloxycarbonyl-2 [méthoxyimino-2 (tritylamino-2 thiazolyl-4)-2 acétamido]-7 oxo-8 thia-5 aza-1 bicyclo[4.2.0] octène-2, isomère syn, sous la forme d'une meringue jaune.

Spectre infra-rouge ($CHCl_3$), bandes caractéristiques ($cm^{-1}$) 3410, 1785, 1725, 1685, 1515, 1450, 1370, 1040, 695.

Spectre de RMN du proton (350 MHz, $CDCl_3$, δ en ppm, J en Jz) 2,09 (s, 3H, $CH_3CO-$); 3,30 et 3,52 (2d, J = 18, 2H, $-SCH_2-$); 3,62 (s, 2H, $-CH_2CO-$); 4,08 (s, 3H =$NOCH_3$); 5,10 (d, J = 4, $H_6$); 5,95 (dd, J = 4 et 9, $H_7$); 6,77 (s, H du thiazole); 6,83 (d, J = 9, -CONH-); 6,87 (s, -COOCH<); 7,01 (s, -NH-trityle).

B. A une suspension de 5,84 g de l'acide méthoxyimino-2 (tritylamino-2 thiazolyl-4)-2 acétique (isomère syn) dans un mélange de 50 cm³ de dichlorométhane et de 1,23 cm³ de N,N-diméthylacétamide à −10°C on ajoute en 20 minutes 6,47 cm³ de solution 2,5 M de phosgène dans le benzène. On laisse réagir pendant 16 heures à −10°C puis on ajoute en 20 minutes une solution de 5,57 g d'acétonyl-3 amino-7 benzhydryloxycarbonyl-2 oxo-8 thia-5 aza-1 bicyclo[4.2.0] octène-2 dans un mélange de 50 cm³ de dichlorométhane et de 5,6 cm³ de triéthylamine. Le mélange est agité pendant 2 heures à −10°C. On ajoute 100 cm³ d'acide chlorhydrique 0,5 N et sépare la phase organique par décantation. On sèche sur sulfate de sodium et concentre à sec sous pression réduite (20 mm de mercure; 2,7 kPa) à 20°C. Le résidu est chromatographié sur une colonne de 130 g de gel de silice Merck (0,06–0,2) (diamètre de la colonne: 3 cm; hauteur: 37 cm). On élue avec 2 litres de mélange acétate d'éthyle/cyclohexane 40–60 (en volumes) en recueillant des fractions de 100 cm³. On concentre à sec sous pression réduite (20 mm de mercure ; 2,7 kPa) à 20°C les fractions 8 à 14. On obtient ainsi 4 g d'acétonyl-3 benzhydryloxycarbonyl-2 [méthoxyimino-2 (tritylamino-2 thiazolyl-4)-2 acétamido]-7 oxo-8 thia-5 aza-1 bicyclo[4.2.0] octène-2, isomère syn identique au produit préparé précédemment en A/.

Une solution de 4,5 g d'acétonyl-3 benzhydryloxycarbonyl-2 [méthoxyimino-2 (tritylamino-2 thiazolyl-4)-2 acétamido]-7 oxo-8 thia-5 aza-1 bicyclo[4.2.0[octène-2, isomère syn dans 42 cm³ d'acide formique est chauffée à 50°C. On ajoute, en 15 minutes, 15 cm³ d'eau distillée et agite le mélange pendant 15 minutes à 50°C. On filtre et concentre à sec sous pression réduite (1 mm de mercure; 0,13 kPa) à 20°C. Le résidu est repris 3 fois de suite avec 50 cm³ d'éthanol que l'on évapore à chaque fois à sec sous pression réduite (1 mm de mercure; 0,13 kPa) à 20°C. Le résidu solide est recristallisé dans 60 cm³ de propanol-2 et les cristaux séparés par filtration, lavés avec 20 cm³ d'éther diéthylique et séchés sous pression réduite (1 mm de mercure; 0,13 kPa) à 30°C. On obtient ainsi 1,2 g d'acétonyl-3 [(amino-2 thiazolyl-4)-2 méthoxyimino-2 acétamido]-7 carboxy-2 oxo-8 thia-5 aza-1 bicyclo[4.2.0] octène-2, isomère syn sous la forme de cristaux beiges.

Spectre infra-rouge (KBr), bandes caractéristiques ($cm^{-1}$) 3330, 1770, 1710, 1670, 1625, 1530, 1360, 1035.

Spectre de RMN du proton (350 MHz, DMSO $d_6$, δ en ppm, J en Hz) 2,2 (s, 3H, $CH_3CO-$); 3,3 à 3,6 (m, 4H, $-SCH_2-$ et $-CH_2CO-$); 4,0 (s, 3H, $CH_3ON$=); 5,10 (d, J = 4, 1H, $H_6$); 5,82 (dd, J = 4 et

9, 1H, $H_7$); 6,60 (s large, 2H, $-NH_2$); 6,80 (s, 1H, H du thiazole); 9,35 (d, J = 9, 1H, -CONH-).

Exemple de référence 3

On ajoute goutte à goutte en 2 minutes une solution de 2,2 g de benzhydryloxycarbonyl-2 t.butoxycarbonylamino-7 méthyl-3 oxo-8 thia-5 aza-1 bicyclo[4.2.0] octène-2 dans 7 $cm^3$ d'hexaméthylphosphorotriamide à une solution de 1,85 g de diméthylamino-1 méthoxy-1 éthylène dans 10 $cm^3$ d'hexaméthylphosphorotriamide à 80°C. On agite la solution à 80°C pendant 2 heures. La solution est versée progressivement dans un mélange agité de 100 $cm^3$ d'eau distillée et de 100 $cm^3$ d'acétate d'éthyle. La phase organique est séparée par décantation, lavée avec 50 $cm^3$ de solution 1N d'acide citrique puis avec 2 fois 50 $cm^3$ de solution aqueuse saturée de chlorure de sodium, séchée sur sulfate de sodium et concentrée à sec sous pression réduite (20 mm de mercure; 2,7 kPa) à 20°C. Le résidu est chromatographié sur une colonne de 50 g de gel de silice Merck (0,06–0,20) (diamètre de la colonne: 2 cm; hauteur: 34 cm). On élue avec un mélange acétate d'éthyle/cyclohexane 25–75 (en volumes) en recueillant des fractions de 50 $cm^3$. On concentre à sec les fractions 4 à 9 sous pression réduite (20 mm de mercure; 2,7 kPa) à 30°C. On obtient ainsi 0,9 g de mélange d'acétonyl-3 benzhydryloxycarbonyl-2 t.butoxycarbonylamino-7 oxo-8 thia-5 aza-1 bicyclo[4.2.0] octène-2 et 3 sous la forme d'une meringue jaune pâle.

Spectre infra-rouge ($CDCl_3$), bandes caractéristiques ($cm^{-1}$) 3440, 1780, 1720, 1505, 1455, 1395, 1370, 1160, 695.

Spectre de RMN du proton (350 MHz, $CDCl_3$, δ en ppm, J en Hz) mélange d'octène-2 pour 65% et d'octène-3 pour 35% 1,45 [s, 9H, $-C(CH_3)_3$]; 1,95 (s, $-CO-CH_3$ octène-3); 2,09 (s, $-CO-CH_3$ octène-2); 3,14 et 3,29 (2d, J = 17, $-CH_2-CO-$ octène-3); 3,27 et 3,57 (2d, J = 18, $-S-CH_2-$ octène-2); 3,56 et 3,68 (2d, J = 17, $-CH_2-CO-$ octène-2); 5,01 (d, J = 4, -H en 6 octène-2); 5,10 (s, -H en 2 octène-3); 5,21 (d, J = 4, -H en 6 octène-3); 5,25 (d, J = 9, -CO-NH- octène-2); 5,30 à 5,50 (mt, -CO-NH- et -H en 7 octène-3); 5,63 (dd, J = 9 et 4, -H en 7 octène-2); 6,09 (s, -H en 4 octène-3); 6,81 [s, $-COO-\underline{CH}(C_6H_5)_2$ octène-3]; 6,88 [s, $-COO-\underline{CH}(C_6H_5)_2$ octène-2]; 7,2 à 7,5 (mt, aromatiques).

A une solution de 0,2 g d'acétonyl-3 benzhydryloxycarbonyl-2 t.butoxycarbonylamino-7 oxo-8 thia-5 aza-1 bicyclo[4.2.0] octène-2 et -3 dans 1,5 $cm^3$ de dichlorométhane à −10°C on ajoute en 10 minutes une solution de 0,8 g d'acide m. chloroperbenzoïque à 85% dans 1,5 $cm^3$ de dichlorométhane. La solution est agitée pendant 15 minutes à 0°C puis lavée avec 5 $cm^3$ de solution aqueuse saturée de bicarbonate de sodium, séchée sur sulfate de sodium et concentrée à sec sous pression réduite (20 mm de mercure; 2,7 kPa) à 20°C. Le résidu est chromatographié sur une colonne de 2 g de gel de silice Merck (0,06–0,2) (diamètre de la colonne: 0,8 cm; hauteur: 8 cm). On élue avec 50 $cm^3$ de mélange acétate d'éthyle/cyclohexane 50–50 (en volumes) en recueillant des fractions de 1 $cm^3$. Les fractions 13 à 17 sont concentrées à sec sous pression réduite (20 mm de mercure; 2,7 kPa) à 20°C. On obtient ainsi 50 mg d'acétonyl-3 benzhydryloxycarbonyl-2 t.butoxycarbonylamino-7 oxo-8 oxyde-5 thia-5 aza-1 bicyclo[4.2.0] octène-2 sous forme d'un solide jaune pâle.

Spectre de RMN du proton (350 MHz, DMSO $d_6$, δ en ppm, J en Hz) 1,43 [s, 9H, $-C(CH_3)_3$]; 2,00 (s, 3H, $CH_3CO-$); 3,53–3,68–3,80–3,83 (4d, J = 19, 4H, $-SCH_2-$ et $-CH_2CO-$); 4,98 (d, J = 4, 1H, $H_6$); 5,75 (dd, J = 4 et 10, 1H, $H_7$); 6,17 (d, J = 10, 1H, -CONH-); 6,85 (s, 1H, -COOCH$<$).

Exemple de référence 4

A une solution de 5,23 g d'acétonyl-3 benzhydryloxycarbonyl-2 t.butoxycarbonylamino-7 oxo-8 thia-5 aza-1 bicyclo[4.2.0] octène-2 dans 50 $cm^3$ d'acétonitrile à 40°C on ajoute en 30 minutes une solution de 3,8 g de monohydrate d'acide p.toluènesulfonique dans 50 $cm^3$ d'acétonitrile. Le mélange est maintenu à 40°C pendant 30 minutes puis concentré sous pression réduite (20 mm de mercure; 2,7 kPa) à 40°C jusqu'à un volume de 30 $cm^3$ environ. On dilue avec 150 $cm^3$ de dichlorométhane, lave la solution avec 50 $cm^3$ de solution aqueuse saturée de bicarbonate de sodium et sèche sur sulfate de magnésium. On obtient ainsi une solution d'acétonyl-3 amino-7 benzhydryloxycarbonyl-2 oxo-8 thia-5 aza-1 bicyclo[4.2.0] octène-2 identique au produit décrit dans l'exemple de référence 2.

La solution ainsi obtenue est refroidie à 4°C et on ajoute 2,51 g d'acide D-α-t.butoxycarbonylaminophénylacétique et 2,27 g de dicyclohexylcarbodiimide. On aigte pendant 2 heures à 4°C puis 10 heures à 20°C. On filtre puis concentre à sec sous pression réduite (20 mm de mercure; 2,7 kPa) à 20°C. Le résidu est chromatographié sur une colonne de 120 g de gel de silice Merck (0,06–0,2) (diamètre de la colonne: 3,5 cm; hauteur: 28 cm). On élue avec 1,5 litre de mélange acétate d'éthyle/cyclohexane 30–70 (en volumes) en recueillant des fractions de 100 $cm^3$. On concentre à sec les fractions 5 à 10 sous pression réduite (20 mm de mercure; 2,7 kPa) à 20°C. On obtient ainsi 2,2 g d'acétonyl-3 benzhydryloxycarbonyl-2 D-α-t.butoxycarbonylaminophénylacétamido-7 oxo-8 thia-5 aza-1 bicyclo[4.2.0] octène-2 sous la forme d'une meringue blanche.

Spectre infra-rouge ($CHCl_3$), bandes caractéristiques ($cm^{-1}$) 3420, 1780, 1720, 1695, 1490, 1450, 1370, 1160, 695.

Spectre de RMN du proton (350 MHz, $CDCl_3$, δ en ppm, J en Hz) 1,46 [s, $-C(CH_3)_3$]; 2,10 (s, $-CO-CH_3$); 3,18 et 3,46 (2d, J = 18, $-S-CH_2-$); 3,58 (s, $-CH_2-CO-$); 4,95 (d, J = 4, -H en 6); 5,22 (mf, $>$NH carbamate); 5,61 (d, J = 7, N-CH-CO-);

5,82 (dd, J = 4 et 9, -H en 7); 6,86 [s, $-COO-\underline{CH}(C_6H_5)_2$]; 7,2 à 7,5 (mt, aromatiques).

On dissout 2,2 g d'acétonyl-3 benzhydryloxycarbonyl-2 D-α-t.butoxycarbonylaminophénylacétamido-7 oxo-8 thia-5 aza-1 bicyclo[4.2.0] octène-2 dans un mélange de 50 cm³ d'acide trifluoroacétique et de 4 cm³ d'anisole. La solution est agitée à 20°C pendant 20 minutes puis évaporée à sec sous pression réduite (1 mm de mercure; 0,13 kPa) à 20°C. On ajoute 50 cm³ d'acétate d'éthyle et concentre à sec sous pression réduite (20 mm de mercure; 2,7 kPa) à 20°C. On répète cette opération 3 fois. Le résidu est repris avec 100 cm³ d'éther diisopropylique. L'insoluble est filtré et séché sous pression réduite (1 mm de mercure; 0,13 kPa) à 20°C. On obtient ainsi 2,0 g de trifluoroacétate d'acétonyl-3 D-α-aminophénylacétamido-7 carboxy-2 oxo-8 thia-5 aza-1 bicyclo[4.2.0] octène-2 que l'on dissout dans 200 cm³ d'eau distillée. La solution est extraite avec 50 cm³ d'acétate d'éthyle. La phase aqueuse est traitée par 20 cm³ de résine humide Amberlite IR-45 ($OH^-$) jusqu'à ce que le pH soit stabilisé vers 5,25 (environ 1 heure). La résine est séparée par filtration. La phase aqueuse est lyophilisée. On obtient ainsi 0,46 g d'acétonyl-3 D-α-aminophénylacétamido-7 carboxy-2 oxo-8 thia-5 aza-1 bicyclo[4.2.0] octène-2 sous la forme d'une poudre beige.

Spectre infra-rouge (KBr), bandes caractéristiques ($cm^{-1}$) 3200, 3100, 1770, 1690, 1660, 1570, 1515, 1500, 1455, 750 et 700.

Spectre de RMN du proton (350 MHz, $CF_3COOD$, δ en ppm, J en Hz) 2,28 (mf, 3H, $-CO-CH_3$); 3,35 à 4 (mt, 4H, $-CH_2CO-$ et $-S-CH_2-$); 5,26 (d, J = 4, 1H, -H en 6); 5,55 (s, 1H, >N-CH-CO); 5,91 (d, J = 4, 1H, -H en 7); 7,5 à 7,8 (mt, aromatiques).

Exemple de référence 5

A une solution de 12,3 g de benzhydryloxycarbonyl-2 D-α-t.butoxycarbonylaminophénylacétamido-7 méthyl-3 oxo-8 thia-5 aza-1 bicyclo[4.2.0] octène-2 dans 80 cm³ de diméthylformamide à 80°C on ajoute goutte à goutte en 35 minutes 8,1 g de diméthylamino-1 méthoxy-1 éthylène puis on maintient la solution à 80°C pendant 20 minutes. On obtient ainsi une solution de benzhydryloxycarbonyl-2 D-α-t.butoxycarbonylaminophénylacétamido-7 (diméthylamino-2 propène-1 yl-1)-3 oxo-8 thia-5 aza-1 bicyclo[4.2.0] octène-2 que l'on verse dans un mélange agité de 150 cm³ d'une solution N d'acide citrique et de 500 cm³ d'acétate d'éthyle. La phase organique est décantée, séchée sur sulfate de sodium et concentrée à sec sous pression réduite (20 mm de mercure; 2,7 kPa) à 20°C. On obtient ainsi 11,5 g de mélange d'acétonyl-3 benzhydryloxycarbonyl-2 D-α-t.butoxycarbonylaminophénylacétamido-7 oxo-8 thia-5 aza-1 bicyclo[4.2.0] octène-2 et -3 sous la forme d'une meringue brune qui est utilisée sans purification ultérieure.

Une solution de 17,4 g du mélange d'acétonyl-3 benzhydryloxycarbonyl-2 D-α-t.butoxycarbonylaminophénylacétamido-7 oxo-8 thia-5 aza-1 bicyclo[4.2.0] octène-2 et -3 dans 300 cm³ de dichlorométhane est refroidie à 0°C. Une solution de 5,4 g d'acide m.chloroperbenzoïque à 85% dans 150 cm³ de dichlorométhane est ajoutée en 40 minutes. On agite la solution à 0°C pendant 2 heures. On lave la solution avec 50 cm³ de solution aqueuse saturée de bicarbonate de sodium puis avec 100 cm³ d'eau distillée. La solution est séchée sur sulfate de sodium et concentrée à sec sous pression réduite (20 mm de mercure; 2,7 kPa) à 20°C. Le résidu est chromatographié sur une colonne de 250 g de gel de silice Merck (0,06-0,2) (diamètre de la colonne: 4,0 cm; hauteur: 40 cm). On élue avec 1,5 litre de mélange acétate d'éthyle/cyclohexane 65-35 (en volumes) en recueillant des fractions de 100 cm³. Les fractions 6 à 10 sont concentrées à sec sous pression réduite (20 mm de mercure; 2,7 kPa) à 20°C. On obtient ainsi 4,2 g d'acétonyl-3 benzhydryloxycarbonyl-2 D-α-t.butoxycarbonylaminophénylacétamido-7 oxo-8 oxyde-5 thia-5 aza-1 bicyclo[4.2.0] octène-2 sous la forme d'une poudre beige.

Spectre infra-rouge (KBr), bandes caractéristiques ($cm^{-1}$) 3340, 1790, 1715, 1690, 1660, 1520, 1495, 1455, 1390, 1370, 1170, 755, 740, 700.

Spectre de RMN du proton (350 MHz, DMSO $D_6$, δ en ppm, J en Hz) 1,42 (s, 9H, $-C(CH_3)_3$); 2,03 (s, 3H, $-CH_3$); 3,12 et 4,33 (2d, J = 18, 2H, $-SCH_2-$); 3,26 (s large, 2H, $-CH_2CO-$); 4,47 (d, J = 4, 1H, $H_6$); 5,24 (large, 1H, -NHCOO-); 5,87 (d, J = 2, 1H,
IH-CH-CO-);
|
N
6,00 (dd, J = 4 et 9, 1H, $H_7$); 6,83 (s, 1H, -COOCH<).

Une solution de 0,67 g d'acétonyl-3 benzhydryloxycarbonyl-2 D-α-t.butoxycarbonylaminophénylacétamido-7 oxo-8 oxyde-5 thia-5 aza-1 bicyclo[4.2.0] octène-2 dans 10 cm³ de dichlorométhane est refroidie à −10°C. On ajoute 0,7 cm³ de N,N-diméthylacéthamide puis 0,18 cm³ de trichlorure de phosphore et agite la solution à −10°C pendant 1 heure. La solution est alors lavée avec 10 cm³ de solution aqueuse saturée de bicarbonate de sodium, séchée sur sulfate de sodium et concentrée à sec sous pression réduite (20 mm de mercure; 2,7 kPa) à 20°C. On obtient ainsi 0,50 g d'acétonyl-3 benzhydryloxycarbonyl-2 D-α-t.butoxycarbonylaminophénylacétamido-7 oxo-8 thia-5 aza-1 bicyclo[4.2.0] octène-2 dont les caractéristiques sont identiques à celles du produit obtenu dans l'exemple de référence 4.

Exemple de référence 6

A une solution de 2,06 g de benzhydryloxycarbonyl-2 méthyl-3 phénoxyacétamido-7 oxo-8 thia-5 aza-1 bicyclo[4.2.0] octène-2 dans 15 cm³ de diméthylformamide à 80°C on ajoute en 45 minutes une solution de 1,62 g de diméthylamino-1 méthoxy-1 éthylène dans 3 cm³ de diméthylformamide. Le mélange est ensuite agité pendant 10 minutes à 80°C. On obtient ainsi une solution de benzhydryloxycarbonyl-2 phénoxyacétamido-7 (diméthylamino-2 propène-1 yl-1)-3 oxo-8 thia-5 aza-1 bicyclo[4.2.0] octène-2, qui est versée dans un mélange agité de 100 cm³ d'acétate d'éthyle,

50 cm³ d'eau distillée et 50 cm³ d'acide citrique 1N à 0°C. La phase organique est séparée par décantation, lavée avec 2 fois 100 cm³ d'eau distillée, séchée sur sulfate de sodium et concentrée à sec sous pression réduite (20 mm de mercure; 2,7 kPa) à 30°C. Le résidu est chromatographié sur une colonne de 20 g de gel de silice Merck (0,06–0,2) (diamètre de la colonne: 2 cm; hauteur: 16 cm). On élue avec 600 cm³ de mélange acétate d'éthyle/cyclohexane 28–72 (en volumes) en recueillant des fractions de 30 cm³. Les fractions 7 à 16 sont concentrées à sec sous pression réduite (20 mm de mercure; 2,7 kPa) à 20°C. On obtient ainsi 0,53 g d'acétonyl-3 benzhydryloxycarbonyl-2 phénoxyacétamido-7 oxo-8 thia-5 aza-1 bicyclo[4.2.0] octène-2 sous la forme d'une meringue jaune.

Spectre infra-rouge ($CHCl_3$), bandes caractéristiques ($cm^{-1}$) 3420, 1785, 1725, 1700, 1660, 1520, 1495, 1440, 690.

Spectre de RMN du proton (80 MHz, $CDCl_3$ δ, en ppm, J en Hz) 2,09 (s, 3H, $-CO-CH_3$); 3,10 à 3,90 (mt, 4H, $-CH_2-CO-$ et $-S-CH_2-$); 4,57 (s, 2H, $-O-CH_2-CO-$); 5,05 (d, J = 5, 1H, -H en 6); 6,93 (dd, J = 9 et 5, 1H, -H en 7); 6,95 (s et d, J = 8, $-COO-\underline{CH}(C_6H_5)_2$ et -H aromatiques en ortho du phénoxyméthyl); 7,05 (t, J = 8, -H aromatique en para du phénoxymétzhyl); 7,15 à 7,65 (mt, aromatiques et -CO-NH-).

A une solution de 0,56 g d'acétonyl-3 benzhydryloxycarbonyl-2 phénoxyacétamido-7 oxo-8 thia-5 aza-1 bicyclo[4.2.0] octène dans 12 cm³ de dichlorométhane refroidie à −10°C, on ajoute en 25 minutes une solution de 0,2 g d'acide m.chloroperbenzoïque à 85% dans 6 cm³ de dichlorométhane, puis le mélange est maintenu à −10°C pendant 10 minutes. La solution est lavée avec 10 cm³ de solution aqueuse saturée de bicarbonate de sodium, séchée sur sulfate de magnésium puis concentrée à sec sous pression réduite (20 mm de mercure; 2,7 kPa) à 20°C. Le résidu est chromatographié sur une colonne de 10 g de gel de silice Merck (0,06–0,2) (diamètre de la colonne: 1,5 cm; hauteur: 12 cm). On élue avec 600 cm³ de mélange acétate d'éthyle/cyclohexane 50–50 (en volumes) en recueillant des fractions de 10 cm³. On concentre à sec les fractions 26 à 32 sous pression réduite (20 mm de mercure; 2,7 kPa) à 20°C. On obtient ainsi 0,12 g d'acétonyl-3 benzhydryloxycarbonyl-2 phénoxyacétamido-7 oxo-8 oxyde-5 thia-5 aza-1 bicyclo[4.2.0] octène-2 sous la forme d'un solide blanc.

Spectre de RMN du proton (360 MHz, DMSO, δ en ppm, J en Hz) 1,97 (s, 3H, $-CH_3$); 3,54 et 3,81 (2d, J = 16, 2H, $-CO-CH_2-$); 3,70 et 3,87 (2d, J = 18, 2H, $-S(O)CH_2-$); 4,72 (s, 2H, $-OCH_2-$); 5,04 (d, J = 5, 1H, $H_6$); 6,12 (dd, J = 5 et 9, 1H, $H_7$); 6,88 (s, 1H, $-COOCH<$); 8,16 (d, J = 9, -CONH-).

Exemple de référence 7

Une suspension constituée par un mélange de 0,21 g de benzhydryloxycarbonyl-2 t.butoxycarbonylamino-7 [α-(β-diméthylaminostyryl)]-3 oxo-8 thia-5 aza-1 bicyclo[4.2.0] octène-2, forme E, de 5 cm³ d'acétate d'éthyle et de 3 cm³ d'acide chlorhydrique 1N est agitée fortement à 20°C pendant 2 heures. La phase organique est décantée, lavée par 3 cm³ d'une solution saturée de bicarbonate de sodium, puis par 2 cm³ d'eau, séchée sur sulfate de magnésium et évaporée à sec sous pression réduite (20 mm de mercure; 2,7 kPa) à 20°C. On obtient ainsi 0,195 g de benzhydryloxycarbonyl-2 t.butoxycarbonylamino-7 oxo-8 phénacyl-3 thia-5 aza-1 bicyclo[4.2.0] octène-2 sous forme d'une meringue jaune pâle.

Spectre infra-rouge ($CHBr_3$), bandes caractéristiques ($cm^{-1}$) 3420, 1785, 1720, 1690, 1595, 1580, 1505, 1495, 1455, 1450, 1390, 1370, 760, 745.

Spectre de RMN du proton (350 MHz, $CDCl_3$, δ en ppm, J en Hz) 1,48 (s, 9H $(CH_3)_3C-$); 3,32 et 3,64 (2d, J = 19, 2H, $-CH_2S-$); 4,09 et 4,47 (2d, J = 17, 2H, $-CH_2CO-$); 5,04 (d, J = 5, 1H, H en 6); 5,31 (d, J = 8, 1H, -CONH-); 5,64 (dd, J = 5 et 8, 1H, H en 7); 6,86 (s, 1H, $-COOCH<$).

On chauffe à 35°C une solution de 3 g de ce produit dans 45 cm³ d'acétonitrile et on ajoute d'un coup une solution de 2,5 g d'acide p.toluènesulfonique monohydraté dans 15 cm³ d'acétonitrile et laisse 2 heures à 35°C. Le mélange réactionnel est alors versé dans 50 cm³ d'une solution saturée de bicarbonate de sodium, extrait par 150 cm³ d'acétate d'éthyle, la phase organique est lavée 2 fois par 100 cm³ d'eau, séchée sur sulfate de magnésium et évaporée à sec sous pression réduite (20 mm de mercure; 2,7 kPa) à 20°C. On obtient ainsi 1,9 g d'amino-7 benzhydryloxycarbonyl-2 oxo-8 phénacyl-3 thia-5 aza-1 bicyclo[4.2.0] octène-2 sous forme de meringue brune.

Spectre infra-rouge ($CHCl_3$), bandes caractéristiques ($cm^{-1}$) 3520, 3440, 1780, 1725, 1675, 1620, 1600, 1580, 1495, 1450, 695.

Spectre de RMN du proton (350 MHz, $CDCl_3$, δ en ppm, J en Hz) 3,28 et 3,63 (2d, J = 18, 2H, $-CH_2S-$); 4,00 et 4,42 (2d, J = 17, 2H, $-CH_2CO-$); 4,70 et 4,99 (2d, J = 4, 2H, $H_6$ et $H_7$); 6,88 (s, 1H, $-COOCH<$).

Exemple de référence 8

A une solution de 7,6 g de benzhydryloxycarbonyl-2 t.butoxycarbonylamino-7 méthyl-3 oxo-8 thia-5 aza-1 bicyclo[4.2.0] octène-2 dans 50 cm³ de dioxanne à 80°C, on ajoute en 8 minutes une solution de 7 g de tris-diméthylaminométhylbenzène dans 50 cm³ de dioxanne et on maintient à 80°C pendant 12 minutes sous agitation. A ce stade on obtient un mélange de benzhydryloxycarbonyl-2 t.butoxycarbonylamino-7 [α-(β-diméthylaminostyryl)]-3 oxo-8 thia-5 aza-1 bicyclo[4.2.0] octène-2 et octène-3, formes Z et E. Le mélange réactionnel est versé dans un mélange agité de 200 cm³ d'acétate d'éthyle, de 20 cm³ d'acide chlorhydrique 1N et de 20 g de glace. L'agitation est continuée à 20°C pendant 22 heures. La phase organique est séparée, lavée par 50 cm³ d'une solution saturée de bicarbonate de sodium, puis 2 fois par 100 cm³ d'eau distillée, séchée sur sulfate de magnésium et évaporée à sec sous pression réduite (20 mm de mercure; 2,7 kPa) à 20°C. Le résidu (11,2 g) est dissous dans 100 cm³ d'un mélange acétate d'éthyle/cyclohexane 30–70 (en volumes). La solution est alors filtrée sur 50 g de gel de silice Merck (0,06–0,2), lavée par 200 $cm^3$ du même mélange et le filtrat évaporé. On obtient 8,3 g d'un mélange de benzhydryloxycarbonyl-2 t.butoxycarbonylamino-7 oxo-8 phénacyl-3 thia-5 aza-1 bicyclo[4.2.0] octène-2 et octène-3 sous forme d'une meringue blonde.

Spectre infra-rouge ($CHBr_3$), bandes caractéristiques ($cm^{-1}$) 3420, 1785, 1720, 1690, 1595, 1580, 1505, 1495, 1455, 1450, 1390, 1370, 760, 745.

Spectre de RMN du proton (350 MHz, $CDCl_3$, δ en ppm, J en Hz) Mélange 50–50 d'octène-2 et d'octène-3.

1,45 (s, 9H, $-C(CH_3)_3$) octène-2 + octène-3); 3,32 et 3,67 (2d, J = 18, 1H, $-SCH_2-$ octène-2); 3,69 et 3,92 (2d, J = 17, 1H, $-CH_2CO-$ octène-3); 4,08 et 4,46 (2D, J = 17, 1H, $-CH_2CO-$ octène-2); 5,07 (d, J = 5, 0,5 H, H en 6 octène-2); 5,24 (d, J = 4, 0,5 H, H en 6 octène-3); 5,30 (s, 0,5 H, H en 2 octène-3); 5,20 à 5,35 (m, 0,5 H, -CONH- octène-2); 5,35 à 5,50 (m, 1H, H en 7 et -CONH- octène-3); 5,64 (dd, J = 5 et 9, 0,5 H, -H en 7 octène-2); 6,14 (s, 0,5 H, H en 4 octène-3); 6,86 (s, 1H, -COOCH octène-2 et octène-3).

A une solution de 4,5 g de mélange de benzhydryloxycarbonyl-2 t.butoxycarbonylamino-7 oxo-8 phénacyl-3 thia-5 aza-1 bicyclo[4.2.0] octène-2 et octène-3 dans 90 $cm^3$ de dichlorométhane à 5°C, on ajoute 1,6 g d'acide m.chloroperbenzoïque à 85% et laisse 30 minutes à 5°C sous agitation. Le mélange réactionnel est versé sur 50 $cm^3$ d'une solution saturée de bicarbonate de sodium; la phase organique est lavée par 50 $cm^3$ d'eau distillée, séchée sur sulfate de magnésium et évaporée à sec sous pression réduite (20 mm de mercure; 2,7 kPa) à 20°C. Le résidu (4,25 g) est fixé sur 20 g de gel de silice Merck (0,06–0,2) et chromatographié sur une colonne de 150 g de gel de silice Merck (0,06–0,2) (diamètre de la colonne: 3,6 cm; hauteur: 40 cm). On élue d'abord par 700 $cm^3$ d'un mélange acétate d'éthyle/cyclohexane 80–20 (en volumes) puis par 1 litre d'un mélange acétate d'éthyle/cyclohexane 70–30 (en volumes), par 500 $cm^3$ d'un mélange 65–35, par 500 $cm^3$ d'un mélange 60–40 et enfin par 1 litre d'un mélange 50–50 en recueillant des fractions de 50 $cm^3$. Les fractions 47 à 56 sont réunies et le solvant est évaporé à sec sous pression réduite (20 mm de mercure; 2,7 kPa) à 20°C. On obtient ainsi 2,5 g de benzhydryloxycarbonyl-2 t.butoxycarbonylamino-7 oxo-8 oxyde-5 phénacyl-3 thia-5 aza-1 bicyclo[4.2.0] octène-2 sous forme d'une meringue brune.

Spectre infra-rouge ($CHCl_3$), bandes caractéristiques ($cm^{-1}$) 3410,1800, 1715, 1685, 1595, 1580, 1500, 1445, 1390, 1370, 1165, 1040.

Spectre de RMN du proton (350 MHz, $CDCl_3$, δ en ppm, J en Hz) 1,50 (s, 9H, $(CH_3)_3C-$); 3,62 (s, 2H, $-CH_2CO-$); 3,65 et 5,28 (2d, J = 18, 2H, $-CH_2S{\rightarrow}O$); 4,65 (d, J = 4, 1H, H en 6); 5,82 (s large, 2H, H en 7 et -CONH-); 6,86 (s, 1H, $-COOCH<$).

A une solution de 8,15 g d'acide p.toluènesulfonique monohydraté dans 170 $cm^3$ d'acétonitrile tiédie à 35°C on ajoute 10,1 g de benzhydryloxycarbonyl-2 t.butoxycarbonylamino-7 oxo-8 oxyde-5 phénacyl-3 thia-5 aza-1 bicyclo[4.2.0] octène-2 et laisse à 35°C sous agitation pendant 1 heure. Le mélange réactionnel est alors versé sur un mélange agité de 150 $cm^3$ d'une solution saturée de bicarbonate de sodium et de 300 $cm^3$ d'acétate d'éthyle. La phase organique est ensuite lavée par 100 $cm^3$ d'eau distillée, séchée sur sulfate de magnésium et évaporée à sec sous pression réduite (20 mm de mercure; 2,7 kPa) à 20°C. On obtient ainsi 7 g d'amino-7 benzhydryloxycarbonyl-2 oxo-8 oxyde-5 phénacyl-3 thia-5 aza-1 bicyclo[4.2.0] octène-2 brut sous forme d'une huile épaisse utilisable sans purification.

A une solution de 2,37 g d'amino-7 benzhydryloxycarbonyl-2 oxo-8 oxyde-5 phénacyl-3 thia-5 aza-1 bicyclo[4.2.0] octène-2, de 1,32 g d'acide D,α-t.butoxycarbonylaminophénylacétique et de 0,01 g de diméthylamino-4 pyridine dans 25 $cm^3$ de dichlorométhane à 20°C, on ajoute sous agitation une solution de 1,08 g de dicyclohexylcarbodiimide dans 10 $cm^3$ de dichlorométhane et laisse agiter pendant 2 heures à 20°C. Le mélange réactionnel est filtré, le filtrat est évaporé à sec sous pression réduite (20 mm de mercure; 2,7 kPa) à 20°C. Le résidu est dissous dans 60 $cm^3$ d'acétate d'éthyle, la solution est lavée par 30 $cm^3$ d'une solution saturée de bicarbonate de sodium, puis par 25 $cm^3$ d'eau distillée, séchée sur sulfate de magnésium et le solvant est évaporé à sec sous pression réduite (20 mm de mercure; 2,7 kPa) à 20°C. On obtient 3,6 g d'un solide qui est mis en suspension dans 50 $cm^3$ d'éther isopropylique. Après filtration, séchage de la solution sur sulfate de sodium et évaporation du solvant on obtient 2,45 g de benzhydryloxycarbonyl-2 (D,α-t.butoxycarbonylamino phénylacétamido)-7 oxo-8 oxyde-5 phénacyl-3 thia-5 aza-1 bicyclo[4.2.0] octène-2 sous forme d'une poudre brun clair.

Spectre infra-rouge ($CHCl_3$), bandes caractéristiques ($cm^{-1}$) 3420, 3380, 1800, 1710, 1690, 1595, 1585, 1575, 1510, 1490, 1480, 1450, 1445, 1390, 1365, 1165, 1045.

Spectre de RMN du proton (350 MHz, $CDCl_3$, δ en ppm, J en Hz) 3,52 (s, 2H, $-CH_2CO-$); 3,63 et 5,21 (2d, J = 18, 2H, $-CH_2S{\rightarrow}O$); 4,58 (d, J = 4, H en 6); 5,56 et 5,80 (2s larges, 2H, -CH(NH-)CO); 6,06 (dd, J = 4 et 7, 1H, H en 7); 6,83 (s, 1H, $-COOCH<$).

A une solution de 3 g de benzhydryloxycarbonyl-2 (D-α-t.butoxycarbonylamino phénylacétamido)-7 oxo-8 oxyde-5 phénacyl-3 thia-5 aza-1 bicyclo[4.2.0] octène-2 dans 30 $cm^3$ de dichlorométhane et 1,65 $cm^3$ de diméthylacétamide refroidie à −7°C, on ajoute 0,71 $cm^3$ de trichlorure de phosphore et maintient le mélange réactionnel sous agitation à −3°C pendant 45 minutes. On verse alors le mélange dans 100 $cm^3$ d'éthyle, lave par 30 $cm^3$ d'une solution saturée de bicarbonate de sodium et par 2 fois 30 $cm^3$ d'eau, sèche sur sulfate de magnésium, filtre et concentre à sec sous pression réduite (20 mm de mercure; 2,7 kPa) à 20°C. Le résidu (3,2 g) est dissous dans 30 $cm^3$ d'un mélange acétate d'éthyle/cyclohexan

25-75 (en volumes) et est chromatographié sur une colonne de 200 g de gel de silice Merck (0,04–0,06) (diamètre de la colonne: 3,6 cm; hauteur: 39 cm). On élue par 1,5 litre d'un mélange acétate d'éthyle/cyclohexane 25-75 (en volumes) sous une pression de 40 kPa en recueillant des fractions de 50 $cm^3$. Les fractions 21 à 29 sont recueillies et évaporées à sec sous pression réduite (20 mm de mercure; 2,7 kPa) à 20°C. On recueille 1 g de benzhydryloxycarbonyl-2 (D,α-t.butoxycarbonylamino phénylacétamido)-7 oxo-8 phénacyl-3 thia-5 aza-1 bicyclo[4.2.0] octène-2 sous forme d'une meringue jaune.

Spectre infra-rouge ($CHCl_3$), bandes caractéristiques ($cm^{-1}$) 3420, 1790, 1720, 1690, 1595, 1580, 1495, 1455, 1450, 1390, 1370, 1160.

Spectre de RMN du proton (350 MHz, $CDCl_3$, δ en ppm, J en Hz) 1,44 (s, 9H, $(CH_3)_3C-$); 3,21 et 3,53 (2d, J = 18, 2H, $-SCH_2-$); 4,09 et 4,34 (2d, J = 18, 2H, $-CH_2CO-$); 5,00 (d, J = 4, 1H, H en 6); 5,22 (m large, -NH- carbamate); 5,64 (d, J = 7, 1H, -NH-CH-CO-);

5,83 (dd, J = 4 et 7, 1H, H en 7); 6,58 (s large, 1H, -CONH- amide); 6,85 (s, 1H, -COOCH<).

1 g de benzhydryloxycarbonyl-2 (D-α-t.butoxycarbonylamino phénylacétamido)-7 oxo-8 phénacyl-3 thia-5 aza-1 bicyclo[4.2.0] octène-2 est dissous dans 13,9 $cm^3$ d'acide trifluoracétique et agité pendant 20 minutes à 20°C. L'acide trifluoracétique est évaporé sous pression réduite (1 mm de mercure; 0,13 kPa). Le résidu est repris 2 fois avec 20 $cm^3$ d'acétate d'éthyle en concentrant à sec à chaque fois sous pression réduite (20 mm de mercure; 2,7 kPa) pour chasser au maximum l'acide trifluoroacétique; le résidu final est dissous dans 5 $cm^3$ d'acétate d'éthyle et la solution ainsi obtenue est ajoutée goutte à goutte dans 30 $cm^3$ d'éther isopropylique en agitant fortement. Le précipité obtenu est filtré, lavé par 5 $cm^3$ d'éther isopropylique et séché sous vide (1 mm de mercure; 0,13 kPa). On obtient ainsi 0,72 g de trifluoroacétate de (D,α-aminophénylacétamido)-7 carboxy-2 oxo-8 phénacyl-3 thia-5 aza-1 bicyclo [4.2.0] octène-2 sous forme d'une poudre blanchâtre.

Spectre infra-rouge (KBr), bandes caractéristiques ($cm^{-1}$) 3300–2150, 1770, 1680, 1600, 1580, 1520, 1495, 1450, 1200, 1130, 800, 755, 720, 700.

Spectre de RMN du proton (350 MHz, $CF_3COOD$, δ en ppm, J en Hz) 3,49 et 3,72 (2d, J = 18, 2H, $-SCH_2-$); 4,48 et 4,69 (2d, J = 17, 2H, $-CH_2CO-$); 5,31 (d, J = 4, 1H, H en 6); 5,91 (d, J = 4, 1H, H en 7); 5,51 (s large, 1H, -CH(N<)CO-); 7,50 à 8,03 (m, 10H, aromatiques).

Exemple de référence 9

Une solution de 1,25 g d'amino-7 benzhydryloxycarbonyl-2 oxo-8 oxyde-5 phénacyl-3 thia-5 aza-1 bicyclo[4.2.0] octène-2 et de 1,05 $cm^3$ de triéthylamine dans 10 $cm^3$ de dichlorométhane refroidie à 0°C est ajoutée en 1 minute à une solution de chlorure de méthoxy-2 (tritylamino-2 thiazolyl-4)-2 acétyle (préparé à partir de 1,21 g d'acide méthoxyimino-2 (tritylamino-2 thiazolyl-4)-2 acétique, 1,3 $cm^3$ d'une solution 2,4 M de phosgène dans du chlorobenzène, 0,23 $cm^3$ de diméthylacétamide dans 15 $cm^3$ de dichlorométhane pendant 16 heures à −7°C) à −10°C. Le mélange réactionnel est agité, pendant 1 heure à −5°C, puis 1 heure en laissant remonter la température de −5°C à 20°C, puis versé sur 5 $cm^3$ d'acide chlorhydrique 0,5 N. La phase organique est lavée par 10 $cm^3$ d'eau, séchée sur sulfate de magnésium et évaporée à sec sous pression réduite (20 mm de mercure; 2,7 kPa) à 20°C. Le résidu est solidifié dans 20 $cm^3$ d'éther isopropylique. On obtient de cette façon 2 g de benzhydrylocycarbonyl-2 [méthoxyimino-2 (tritylamino-2 thiazolyl-4)-2 acétamido]-7 oxo-8 oxyde-5 phénacyl-3 thia-5 aza-1 bicyclo[4.2.0] octène-2, isomère syn sous forme d'un solide orangé.

Spectre infra-rouge ($CHCl_3$), bandes caractéristiques ($cm^{-1}$) 3400, 1800, 1725, 1680, 1590, 1580, 1510, 1490, 1445, 1040, 690.

Spectre de RMN du proton (350 MHz, $CDCl_3$, δ en ppm, J en Hz) [3,59 (s large, 2H) et 3,68 et 5,22 (2d, J = 18, 2H) $-CH_2S-$ et $-CH_2CO-$]; 4,09 (s, 3H, $=NOCH_3$); 4,71 (d, J = 5, 1H, H en 6); 6,16 (dd, J = 5 et 9, 1H, H en 7); 6,74 (s, 1H, H thiazole); 6,85 (s, 1H, -COOCH<); 7,07 (s large, 1H, $-N\underline{H}C(C_6H_5)_3$); 7,50 (d, J = 9, 1H, -CONH-).

A une solution de 4,3 g de benzhydryloxycarbonyl-2 [méthoxyimino-2 (tritylamino-2 thiazolyl-4)-2 acétamido]-7 oxo-8 oxyde-5 phénacyl-3 thia-5 aza-1 bicyclo[4.2.0] octène-2, isomère syn et de 1,88 $cm^3$ de diméthylacétamide dans 43 $cm^3$ de dichlorométhane refroidie à −8°C, on ajoute en 1 minute 0,81 $cm^3$ de trichlorure de phosphore et laisse pendant 1 heure sous agitation à −8°C. Le mélange est alors versé sur 20 $cm^3$ d'une solution saturée de bicarbonate de sodium et 100 $cm^3$ d'acétate d'éthyle. La phase organique est lavée par 30 $cm^3$ d'eau, séchée sur sulfate de magnésium et évaporée à sec sous pression réduite (20 mm de mercure; 2,7 kPa) à 20°C. Le résidu est dissous dans 15 $cm^3$ d'acétate d'éthyle, la solution est filtrée sur 13 g de gel de silice Merck (0,06–0,2) et lavée par 20 $cm^3$ d'acétate d'éthyle. Le filtrat est évaporé à sec sous pression réduite (20 mm de mercure; 2,7 kPa) à 20°C. On obtient ainsi 3,6 g de benzhydryloxycarbonyl-2 [méthoxyimino-2 (tritylamino-2 thiazolyl-4)-2 acétamido]-7 oxo-8 phénacyl-3 thia-5 aza-1 bicyclo[4.2.0] octène-2, isomère syn sous forme d'un solide beige.

Spectre infra-rouge ($CHCl_3$), bandes caractéristiques ($cm^{-1}$) 3400, 2820, 1780, 1725, 1680, 1590, 1580, 1520, 1490, 1450, 1040.

Spectre de RMN du proton (350 MHz, $CDCl_3$, δ en ppm, J en Hz) 3,30 et 3,63 (2d, J = 18, 2H, $-CH_2S-$); 4,07 (s, 3H, $=NOCH_3$); 4,11 et 4,40 (2d, J = 18, 2H, $-CH_2CO-$); 5,11 (d, J = 5, 1H, H en 6); 5,95 (dd, J = 5 et 9, 1H, H en 7); 6,77 (s, 1H, H du thiazole); 6,87 (s, 1H, -COOCH<); 6,91 (d, J = 9, 1H, -CONH-); 7,03 (s, 1H, $-N\underline{H}C(C_6H_5)_3$).

On dissout 2,7 g de benzhydryloxycarbonyl-2 [méthoxyimino-2 (tritylamino-2 thiazolyl-4)-2 acétamido]-7 oxo-8 phénacyl-3 thia-5 aza-1 bicyclo[4.2.0] octène-2, isomère syn dans 55 $cm^3$

d'acide formique à 20°C. On ajoute 11 $cm^3$ d'eau distillée et chauffe le mélange pendant 15 minutes à 45°C. Le mélange est refroidi à 20°C, dilué par 44 $cm^3$ d'eau et filtré. Le filtrat est amené à sec sous pression réduite (5 mm de mercure; 0,67 kPa) à 20°C. Le résidu est repris 3 fois par 50 $cm^3$ d'éthanol en évaporant à chaque fois le solvant sous pression réduite (5 mm de mercure; 0,67 kPa) à 20°C pour éliminer l'eau et l'acide formique. Le résidu final est repris par 50 $cm^3$ d'une solution saturée de bicarbonate de sodium, la phase aqueuse est lavée par 50 $cm^3$ d'acétate d'éthyle et acidifiée à pH 3 par de l'acide chlorhydrique 1N sous agitation en présence de 100 $cm_3$ d'acétate d'éthyle. La phase organique est séchée sur sulfate de magnésium et évaporée à sec sous pression réduite (20 mm de mercure; 2,7 kPa) à 20°C. On obtient ainsi 0,54 g d'[(amino-2 thiazolyl-4)-2 méthoxyimino-2 acétamido]-7 carboxy-2 oxo-8 phénacyl-3 thia-5 aza-1 bicyclo[4.2.0] octène-2, isomère syn sous forme d'un solide beige.

Spectre infra-rouge (KBr), bandes caractéristiques ($cm^{-1}$) 3340, 3250 à 2200, 1770, 1675, 1630, 1595, 1580, 1530, 1445, 1040, 690.

Spectre de RMN du proton (350 MHz, DMSO $d_6$, δ en ppm, J en Hz) 3,45 et 3,63 (2d, J = 18, 2H, $-CH_2S-$); 3,85 (s, 3H, $=N-OCH_3$); 4,20 et 4,45 (2d, J = 18, 2H, $-CH_2CO-$); 5,17 (d, J = 5, 1H, H en 6); 5,75 (dd, J = 5 et 9, 1H, H en 7); 6,75 (s, 1H, H thiazole); 7,2 (s, 2H, $-NH_2$); 9,65 (d, J = 9, 1H, -CONH-).

L'[(amino-2 thiazolyl-4)-2 méthoxyimino-2 acétamido]-7 carboxy-2 oxo-8 phénacyl-3 thia-5 aza-1 bicyclo[4.2.0] octène-2, isomère syn peut être mis en œuvre par analogie avec la méthode décrite à l'exemple de référence 1 pour préparer un produit de formule générale (XVII).

**Revendications**

pour les Etats contractants BE CH DE FR GB IT LI LU NL SE

1. Un dérivé de la céphalosporine caractérisé en ce qu'il répond à la formule générale:

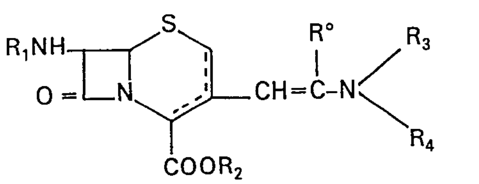

(I)

qui se présente sous forme bicyclooctène-2 ou -3 dans laquelle les symboles $R_3$ et $R_4$, qui sont identiques ou différents, représentent des radicaux alcoyle (éventuellement substitués par un radical alcoyloxy ou dialcoylamino) ou phényle ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle,

a) le symbole $R_1$ représente un radical de formule générale:

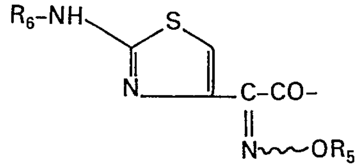

[(dans laquelle $R_5$ est un atome d'hydrogène, un radical alcoyle, vinyle, cyanométhyle, un groupement protecteur d'oxime ou un radical de formule générale:

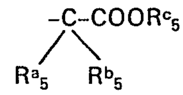

(dans laquelle $R^a_5$ et $R^b_5$ qui sont identiques ou différents sont des atomes d'hydrogène, des radicaux alcoyle, ou forment ensemble un radical alcoylène contenant 2 ou 3 atomes de carbone, et $R^c_5$ est un radical protecteur d'acide) et $R_6$ est un radical protecteur d'amino)], un radical benzhydryle, trityle, un radical acyle de formule générale:

$R_7CO-$

[(dans laquelle $R_7$ est un radical alcoyle substitué par un radical phényle ou phénoxy)], un radical de formule générale:

$R_8OCO-$

[(dans laquelle $R_8$ est un radical alcoyle ramifié non substitué ou alcoyle droit ou ramifié portant un ou plusieurs substituants [choisis parmi phényle et phényle substitué (par un radical alcoyloxy, nitro ou phényle], ou vinyle)], un radical de formule générale:

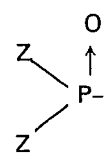

dans laquelle les symboles Z représentent des radicaux phényle ou -OZ' dans lequel Z' représente un radical alcoyle, trichloro-2,2,2 éthyle, phényle ou benzyle (ces deux derniers radicaux pouvant être substitués par un radical alcoyle, alcoyloxy ou nitro), ou bien les symboles Z' des deux substituants Z forment ensemble un radical alcoylène contenant 2 ou 3 atomes de carbone ou bien $R_1NH$ est remplacé par un radical méthylène amino dans lequel le radical méthylène est substitué par un groupement dialcoylamino ou aryle (lui-même éventuellement substitué par 1 ou plusieurs radicaux méthoxy ou nitro),

- le symbole $R_2$ représente un radical protecteur d'acide et
- le symbole R° représente un radical d'un pre-

mier groupe constitué par un radical phényle éventuellement substitué [par un radical alcoyle, trifluorométhyle, dialcoylaminométhyle, alcoyloxy, alcoylthio, dialcoylamino ou par un atome d'halogène choisi parmi le fluor ou le chlore], un radical hétérocyclyle à 5 ou 6 chaînons contenant 1 seul hétéroatome choisi parmi pyridyle-2 ou -3, thiényle-2 ou -3 ou furyle -2 ou -3 et , éventuellement substitué par un radical alcoyle, alcoyloxy ou diméthylaminométhyle, ou bien un radical d'un second groupe constitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons choisi parmi pyridyle-2 ou -3, thiényle-2 ou -3 ou furyle-2 ou -3, un radical cycloalcoyle contenant 3 à 6 chaînons, ou un radical $-CHR_9R_{10}$ dans lequel $R_9$ et $R_{10}$ forment, avec l'atome de carbone auquel ils sont rattachés, un hétérocycle à 5 ou 6 chaînons contenant un atome d'oxygène ou de soufre, ou bien

b) le symbole $R_1$ représente un radical trityle, silyle un radical alcoyloxycarbonyle dont la partie alcoyle contient 1 à 5 atomes de carbone, éventuellement substitué par un ou plusieurs radicaux phényle ou furyle, un radical (biphénylyl-4)-2 propyloxycarbonyle, ou un radical de formule générale:

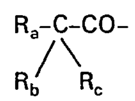

dans laquelle

$R_a$ est un radical phényle, thiényle, furyle, cyclohexadiényle ou cyclohexényle éventuellement substitués, $R_b$ est un atome d'hydrogène et $R_c$ est un atome d'hydrogène, un radical hydroxy éventuellement protégé, un radical amino protégé ou un radical sulfo éventuellement protégé, ou $R_a$ est un radical phénoxy éventuellement substitué ou pyridylthio et $R_b$ et $R_c$ sont des atomes d'hydrogène, ou bien $R_a$ est un radical phényle, thiényle, furyle et $R_b$ et $R_c$ forment ensemble un radical alcoyloxyimino, cycloalcoyloxyimino ou phénylalcoyloxyimino de forme syn

- le symbole $R_2$ représente un radical protecteur d'acide, et
- le symbole R° représente

un radical alcoyle (contenant 1 à 7 atomes de carbone),

un radical cycloalcoyle (contenant 3 à 7 atomes de carbone),

un radical cycloalcoyl-alcoyle (contenant 4 à 7 atomes de carbone)

un radical aryle mono ou bicyclique,

un radical arylalcoyle

un radical hétérocyclyle ou hétérocyclylalcoyle à 5 ou 6 chaînons contenant 1 à 4 atomes d'azote et/ou un atome d'oxygène ou de soufre ces radicaux R° pouvant être éventuellement substitués par des radicaux alcoyle ou alcoyloxy, étant entendu que (sauf mention spéciale) les portions ou radicaux alcoyles ou acyles sont droits ou ramifiés et contiennent 1 à 4 atomes de carbone.

2. Un dérivé de la céphalosporine selon la revendication 1 tel que défini en a) caractérisé en ce que $R_1$ représente un radical de formule générale:

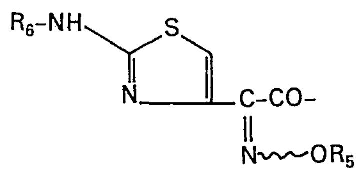

dans laquelle $R_5$ est un atome d'hydrogène, un radical alcoyle, vinyle, cyanométhyle, trityle, tétrahydropyrannyle ou méthoxy-2 propyle-2, ou un radical de formule générale:

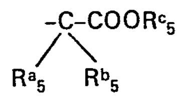

dans laquelle $R^a_5$ et $R^b_5$ sont définis comme dans la revendication 1, et $R^c_5$ est un radical méthoxyméthyle, t.butyle, benzhydryle, benzyle, nitrobenzyle, ou p. méthoxybenzyle.

3. Un dérivé de la céphalosporine selon la revendication 1 tel que défini en a) caractérisé en ce que $R_1$ représente un radical

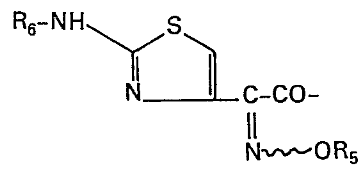

dans laquelle $R_6$ est choisi parmi t.butoxycarbonyle, trityle, benzyle, dibenzyle, benzyloxycarbonyle, p.nitrobenzyloxycarbonyle, p.méthoxybenzyloxycarbonyle ou un radical $Z_2P(O)-$ tel que défini dans la revendication 1.

4. Un dérivé de la céphalosporine selon la revendication 1 tel que défini en a), caractérisé en ce que $R_2$ est choisi parmi méthoxyméthyle, t.butyle, benzhydryle, benzyle, nitrobenzyle ou p.méthoxybenzyle.

5. Un dérivé de la céphalosporine selon la revendication 1 tel que défini en b), caractérisé en ce que $R_2$ est un groupe arlye polysubstitué par des radicaux aliphatiques ou aromatiques, un groupe hétérocyclique aromatique contenant de l'oxygène ou du soufre, un groupe t.alcoyle, diphénylméthyle éventuellement substitué, alcoyloxyphénylalcoyle, diméthoxybenzyle, nitrobenzyle, 4,5-diméthoxy 2-nitrobenzyle, furfuryle, ou un groupe 2-oxa (ou 2-thia) cycloalcoyle, 2-oxa (ou 2-thia) cycloalcényle contenant 5 à 7 chaînons, ou un groupe nitrophényle ou 2,4-dinitrophényle, ou silyle substitué par des radicaux alcoyloxy, cycloalcoyle, phényle ou phénylalcoyle.

6. Un dérivé de la céphalosporine selon la revendication 1, défini comme en b) caractérisé en ce que $R_1$ représente un radical de formule générale:

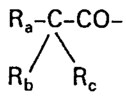

dans laquelle, $R_a$, $R_b$ et $R_c$ étant définis selon la revendication 1, les radicaux phényle et phénoxy que représente $R_a$ peuvent être substitués par des groupes hydroxy, alcoyloxy, acyloxy, benzoyloxy, 4-carbamoyloxy, amino ou alcoylamino protégés, dialcoylamino, alcoylsulfonylamino, aminométhyle, t.butoxycarbonylaminométhyle ou par un atome d'halogène, les radicaux thiényle, furyle, cyclohexadiényle et cyclohexényle que représente $R_a$ peuvent être substitués par des radicaux aminométhyl protégés en position -5 sur les radicaux thiényle et furyle et en position -2 ou -3 sur les radicaux cyclohexadiényle et cyclohexényle.

7. Un dérivé de la céphalosporine selon la revendication 1, caractérisé en ce que le symbole $R_1$ est un radical trityle, un radical de formule générale

$R_7CO-$

dans laquelle $R_7$ est défini comme dans la revendication 1, un radical de formule générale

$R_8OCO-$

dans laquelle $R_8$ est défini comme dans la revendication 1, un radical de formule générale

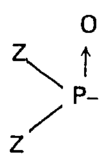

dans laquelle Z est défini comme dans la revendication 1, le symbole $R_2$ représente un radical protecteur, tel que défini en a) dans la revendication 1, ou bien le symbole $R_1$ représente un radical de formule générale

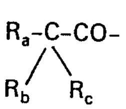

dans laquelle $R_a$, $R_b$ et $R_c$ sont définis comme dans la revendication 1, le symbole $R_2$ est un radical protecteur tel que défini en b) dans la revendication 1, le symbole R° est un radical alcoyle ou phényle et les symboles $R_3$ et $R_4$ représentent des radicaux alcoyle.

8. Un dérivé de la céphalosporine selon la revendication 1, caractérisé en ce que le symbole $R_1$ représente un radical trityle, un radical phénoxyacétyle, un radical de formule générale $R_8OCO-$ dans laquelle $R_8$ est un radical alcoyle ramifié, un radical de formule générale $(Z)_2P(O)-$ dans laquelle Z est un radical alcoyloxy, ou bien un radical α amino α phénylacétamido dont la fonction amine est protégée, le symbole $R_2$ est un radical benzhydryle ou nitrobenzyle, le symbole R° est un radical méthyle ou phényle et les symboles $R_3$ et $R_4$ sont des radicaux méthyle.

9. Un procédé de préparation d'un produit selon l'une des revendications 1 à 8 pour lequel $R_1$, $R_2$, $R_3$ et $R_4$ sont définis comme dans les revendications 1 à 5 et R° est soit un radical du premier groupe défini en a) dans la revendication 1, soit choisi parmi les radicaux aryle ou hétérocyclyle définis en b) dans la revendication 1, caractérisé en ce que l'on fait agir un réactif de formule générale:

$R°-C(NR_3R_4)_3$

dans laquelle R°, $R_3$ et $R_4$ sont définis comme ci-dessus, sur un dérivé de la céphalosporine de formule générale:

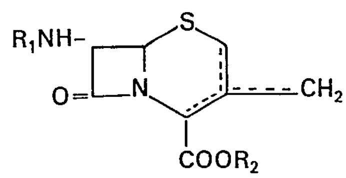

qui se présente sous forme méthyl-3 bicyclooctène-2 ou -3 ou méthylène-3 bicyclooctane et dans laquelle $R_1$ et $R_2$ sont définis selon l'une des revendications 1 à 5.

10. Un procédé de préparation d'un produit selon l'une des revendications 1 à 8 pour lequel $R_1$, $R_2$, $R_3$ et $R_4$ sont définis comme dans les revendications 1 à 5 et R° est soit un radical du second groupe défini précédemment en a) dans la revendication 1, soit choisi parmi les radicaux alcoyle, cycloalcoyle, cycloalcoylalcoyle, arylalcoyle ou hétérocyclylalcoyle définis en b) dans la revendication 1, caractérisé en ce que l'on fait agir un réactif de formule générale:

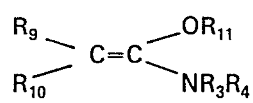

(dans laquelle $R_9$ et $R_{10}$ sont tels que $R_9R_{10}CH-$ représente le radical R° défini ci-dessus, $R_3$ et $R_4$ sont définis comme dans la revendication 1, et $R_{11}$ est un radical alcoyle contenant 1 à 5 atomes de carbone), sur un dérivé de la céphalosporine de formule générale:

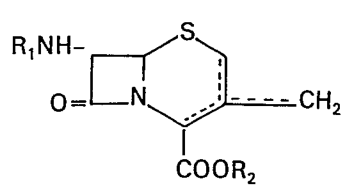

défini comme dans la revendication 9.

**Revendication**
pour l'Etat contractant AT

Procédé de préparation d'un dérivé de la céphalosporine de formule générale:

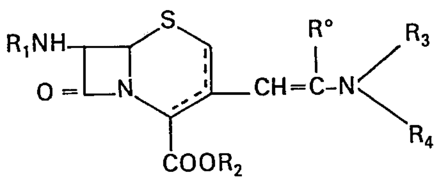

qui se présente sous forme bicyclooctène-2 ou -3 dans laquelle les symboles $R_3$ et $R_4$, qui sont identiques ou différents, représentent des radicaux alcoyle (éventuellement substitués par un radical alcoyloxy ou dialcoylamino) ou phényle ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle, et

a) le symbole $R_1$ représente un radical de formule générale:

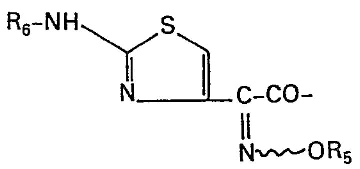

[(dans laquelle $R_5$ est un atome d'hydrogène, un radical alcoyle, vinyle, cyanométhyle, un groupement protecteur d'oxime ou un radical de formule générale:

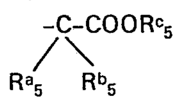

(dans laquelle $R^a{}_5$ et $R^b{}_5$ qui sont identiques ou différents sont des atomes d'hydrogène, des radicaux alcoyle, ou forment ensemble un radical alcoylène contenant 2 ou 3 atomes de carbone, et $R^c{}_5$ est un radical protecteur d'acide) et $R_6$ est un radical protecteur d'amino)],
un radical benzhydryle, trityle,
un radical acyle de formule générale:

$R_7CO-$

[(dans laquelle $R_7$ est un radical alcoyle substitué par un radical phényle ou phénoxy)],
un radical de formule générale:

$R_8OCO-$

[(dans laquelle $R_8$ est un radical alcoyle ramifié non substitué ou alcoyle droit ou ramifié portant un ou plusieurs substituants [choisis parmi phényle et phényle substitué (par un radical alcoyloxy, nitro ou phényle)], ou vinyle)],
un radical de formule générale:

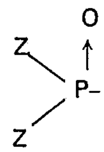

dans laquelle les symboles Z représentent des radicaux phényle ou -OZ' dans lequel Z' représente un radical alcoyle, trichloro-2,2,2 éthyle, phényle ou benzyle (ces deux derniers radicaux pouvant être substitués par un radical alcoyle, alcoyloxy ou nitro), ou bien les symboles Z' des deux substituants Z forment ensemble un radical alcoylène contenant 2 ou 3 atomes de carbone ou bien $R_1NH$ est remplacé par un radical méthylène amino dans lequel le radical méthylène est substitué par un groupement dialcoylamino ou aryle (lui-même éventuellement substitué par 1 ou plusieurs radicaux méthoxy ou nitro),

– le symbole $R_2$ représente un radical protecteur d'acide et

– le symbole R° représente un radical d'un premier groupe constitué par un radical phényle éventuellement substitué [par un radical alcoyle, trifluorométhyle, dialcoylaminométhyle, alcoyloxy, alcoylthio, dialcoylamino ou par un atome d'halogène choisi parmi le fluor ou le chlore], un radical hétérocyclyle à 5 ou 6 chaînons contenant 1 seul hétéroatome choisi parmi pyridyle-2 ou -3, thiényle-2 ou -3 ou furyle -2 ou -3 et éventuellement substitué par un radical alcoyle, alcoyloxy ou diméthylaminométhyle, ou bien un radical d'un second groupe consitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons choisi parmi pyridyle-2 ou-3, thiényle-2 ou -3 ou furyle-2 ou -3, un radical cycloalcoyle contenant 3 à 6 chaînons, ou un radical $-CHR_9R_{10}$ dans lequel $R_9$ et $R_{10}$ forment, avec l'atome de carbone auquel ils sont rattachés un hétérocycle à 5 ou 6 chaînons contenant un atome d'oxygène ou de soufre, ou bien

b) le symbole $R_1$ représente un radical trityle, silyle un radical alcoyloxycarbonyle dont la partie alcoyle contient 1 à 5 atomes de carbone, éventuellement substitué par un ou plusieurs radicaux phényle ou furyle,
un radical (biphénylyl-4)-2 propyloxycarbonyle, ou
un radical de formule générale:

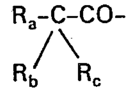

dans laquelle
$R_a$ est un radical phényle, thiényle, furyle, cyclohexadiényle ou cyclohexényle éventuellement substitués, $R_b$ est un atome d'hydrogène et $R_c$ est

un atome d'hydrogène, un radical hydroxy éventuellement protégé, un radical amino protégé ou un radical sulfo éventuellement protégé, ou $R_a$ est un radical phénoxy éventuellement substitué ou pyridylthio et $R_b$ et $R_c$ sont des atomes d'hydrogène, ou bien $R_a$ est un radical phényle, thiényle, furyle et $R_b$ et $R_c$ forment ensemble un radical alcoyloxyimino, cycloalcoyloxyimino ou phénylalcoyloxyimino de forme syn

- le symbole $R_2$ représente un radical protecteur d'acide, et
- le symbole R° représente

un radical alcoyle (contenant 1 à 7 atomes de carbone),
un radical cycloalcoyle (contenant 3 à 7 atomes de carbone),
un radical cycloalcoyl-alcoyle (contenant 4 à 7 atomes de carbone)
un radical aryle mono ou bicyclique,
un radical arylalcoyle
un radical hétérocyclyle ou hétérocyclylalcoyle à 5 ou 6 chaînons contenant 1 à 4 atomes d'azote et/ou un atome d'oxygène ou de soufre ces radicaux R° pouvant être éventuellement substitués par des radicaux alcoyle ou alcoyloxy, étant entendu que (sauf mention spéciale) les portions ou radicaux alcoyles ou acyles sont droits ou ramifiés et contiennent 1 à 4 atomes de carbone, caractérisé en ce que pour la préparation d'un produit pour lequel R° est soit un radical du premier groupe défini en a), soit choisi parmi les radicaux aryle ou hétérocyclyle définis en b) on fait agir un réactif de formule générale:

$R°-C(NR_3R_4)_3$

dans laquelle R°, $R_3$ et $R_4$ sont définis comme ci-dessus, sur un dérivé de la céphalosporine de formule générale:

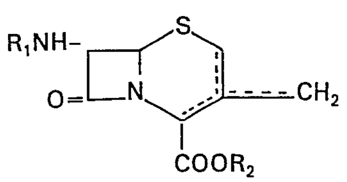

qui se présente sous forme méthyle-3 bicyclooctène-2 ou -3 ou méthylène-3 bicyclooctane et dans laquelle $R_1$ et $R_2$ sont définis comme ci-dessus ou, pour la préparation d'un produit pour lequel R° est soit un radical du second groupe défini précédemment en a), soit choisi parmi les radicaux alcoyle, cycloalcoyle, cycloalcoylalcoyle, cycloalcoylalcoyle, arylalcoyle ou hétérocyclylalcoyle définis en b), on fait agir un réactif de formule générale:

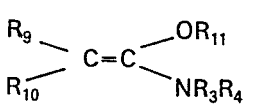

(dans laquelle $R_9$ et $R_{10}$ sont tels que $R_9R_{10}CH-$ représente le radical R° défini ci-dessus, $R_3$ et $R_4$ sont définis comme ci-dessus, et $R_{11}$ est un radical alcoyle contenant 1 à 5 atomes de carbone), sur un dérivé de la céphalosporine de formule générale:

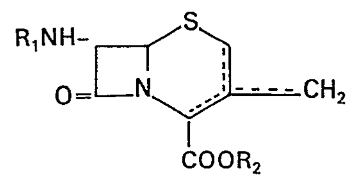

défini comme ci-dessus.

**Patentanspruch**
für den Vertragsstaat AT

Verfahren zur Herstellung eines Cephalosporinderivats der allgemeinen Formel:

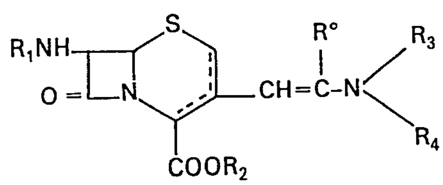

das in 2- oder 3-Bicyclooцten-Form vorliegt, worin die Symbole $R_3$ und $R_4$, die gleich oder voneinander verschieden sind, Alkylreste (gegebenenfalls substituiert durch einen Alkyloxy- oder Dialkylaminorest) oder Phenylreste darstellen oder zusammen mit dem Stickstoffatom, an das sie gebunden sind, einen gesättigten Heterocyclus mit 5 oder 6 Gliedern bilden, der gegebenenfalls ein weiteres Heteroatom, ausgewählt aus Stickstoff, Sauerstoff oder Schwefel, enthält und gegebenenfalls durch einen Alkylrest substituiert ist, und

a) das Symbol $R_1$ einen Rest der allgemeinen Formel:

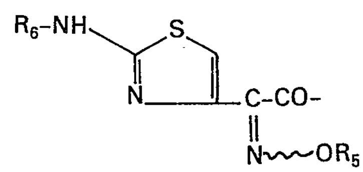

[in welcher $R_5$ ein Wasserstoffatom, ein Alkyl-, Vinyl-, Cyanomethylrest, eine Oximschutzgruppe oder ein Rest der allgemeinen Formel:

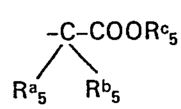

ist (in welcher $R^a_5$ und $R^b_5$, die gleich oder voneinander verschieden sind, Wasserstoffatome oder Alkylreste sind oder zusammen einen Alkylenrest mit 2 oder 3 Kohlenstoffatomen bilden und $R^c_5$

eine Säureschutzgruppe ist) und $R_6$ eine Aminoschutzgruppe ist],
einen Benzhydryl- oder Tritylrest,
einen Acylrest der allgemeinen Formel:

$R_7CO-$

[(in welcher $R_7$ ein Alkylrest, substituiert durch einen Phenyl- oder Phenoxyrest, ist)],
einen Rest der allgemeinen Formel:

$R_8OCO-$

[(in welcher $R_8$ ein verzweigter nicht substituierter Alkylrest oder ein geradkettiger oder verzweigter Alkylrest mit einem oder mehreren Substituenten [ausgewählt aus Phenyl und substituiertem (durch einen Alkyloxy-, Nitro- oder Phenylrest) Phenyl] oder Vinylrest ist)],
einen Rest der allgemeinen Formel:

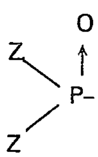

in welcher die Symbole Z Phenylreste oder $-OZ'$ darstellen, worin Z′ einen Alkyl-,2,2,2-Trichloräthyl-, Phenyl- oder Benzylrest darstellt (diese letzten beiden Reste können durch einen Alkyl-, Alkyloxy- oder Nitrorest substituiert sein) oder die Symbole Z′ der beiden Substituenten Z gemeinsam einen Alkylenrest mit 2 oder 3 Kohlenstoffatomen bilden, darstellt oder $R_1NH$ ersetzt ist durch einen Methyleniminorest, in welchem der Methylenrest durch eine Dialkylamino- oder Arylgruppe (selbst gegebenenfalls substituiert durch 1 oder mehrere Methoxy- oder Nitroreste) substituiert ist,
- das Symbol $R_2$ eine Säureschutzgruppe darstellt und
- das Symbol R° einen Rest aus einer ersten Gruppe, bestehend aus einem Phenylrest, gegebenenfalls substituiert [durch einen Alkyl-, Trifluormethyl-, Dialkylaminomethyl-, Alkyloxy-, Alkylthio-, Dialkylaminorest oder durch ein Halogenatom, ausgewählt aus Fluor oder Chlor], einem Heterocyclylrest mit 5 oder 6 Gliedern, enthaltend ein einziges Heteroatom, ausgewählt aus 2- oder 3-Pyridyl, 2- oder 3-Thienyl oder 2- oder 3-Furyl und gegebenenfalls substituiert durch einen Alkyl-, Alkyloxy- oder Dimethylaminomethylrest, oder einen Rest aus einer zweiten Gruppe, bestehend aus einem Alkylrest mit 1 bis 5 Kohlenstoffatomen, einem Benzylrest, gegebenenfalls substituiert (durch ein Halogenatom oder einen Alkyl-, Alkyloxy-, Alkylthio-, Dialkylamino- oder Trifluormethylrest), einem Methylrest, substituiert durch einen aromatischen Heterocyclus mit 5 oder 6 Gliedern, ausgewählt aus 2- oder 3-Pyridyl, 2- oder 3-Thienyl- oder 2- oder 3-Furyl, einem Cycloalkylrest mit 3 bis 6 Gliedern, oder einem Rest $-CHR_9R_{10}$, in welchem $R_9$ und $R_{10}$ mit dem Kohlenstoffatom, an welches sie gebunden sind, einen Heterocyclus mit 5 oder 6 Gliedern bilden, der ein Sauerstoff- oder Schwefelatom enthält, oder
b) das Symbol $R_1$ einen Trityl- oder Silylrest, einen Alkyloxycarbonylrest, dessen Alkylteil 1 bis 5 Kohlenstoffatome enthält, gegebenenfalls substituiert durch einen oder mehrere Phenyl- oder Furylreste,
einen 2-(4-Biphenylyl)-propyloxycarbonylrest oder
einen Rest der allgemeinen Formel:

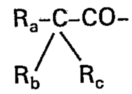

in welcher
$R_a$ ein gegebenenfalls substituierter Phenyl-, Thienyl-, Furyl-, Cyclohexadienyl- oder Cyclohexenylrest ist, $R_b$ ein Wasserstoffatom ist und $R_c$ ein Wasserstoffatom, ein gegebenenfalls geschützter Hydroxyrest, ein geschützter Aminorest oder ein gegebenenfalls geschützter Sulforest ist, oder $R_a$ ein gegebenenfalls substituierter Phenoxyrest oder ein Pyridylthiorest ist, und $R_b$ und $R_c$ Wasserstoffatome sind oder $R_a$ ein Phenyl-, Thienyl- oder Furylrest ist und $R_b$ und $R_c$ zusammen einen Alkyloxyimino-, Cycloalkyloxyimino- oder Phenylalkyloxyiminorest in syn-Form bilden
- das Symbol $R_2$ eine Säureschutzgruppe darstellt und
- das Symbol R°
einen Alkylrest (mit 1 bis 7 Kohlenstoffatomen),
einen Cycloalkylrest (mit 3 bis 7 Kohlenstoffatomen),
einen Cycloalkyl-alkylrest (mit 4 bis 7 Kohlenstoffatomen), einen mono- oder bicyclischen Arylrest,
einen Arylalkylrest,
einen Heterocyclyl- oder Heterocyclylalkylrest mit 5 oder 6 Gliedern, der 1 bis 4 Stickstoffatome und/oder ein Sauerstoff- oder Schwefelatom enthält, darstellt, welche Reste R° gegebenenfalls durch Alkyl- oder Alkyloxyreste substituiert sein können, wobei selbstverständlich die Alkyl- oder Acylteile oder -reste (ohne spezielle Erwähnung) geradkettig oder verzweigt sind und 1 bis 4 Kohlenstoffatome enthalten, dadurch gekennzeichnet, dass man zur Herstellung einer Verbindung in welcher R° entweder ein Rest der ersten unter a) angegebenen Gruppe ist oder ausgewählt ist aus den unter b) angegebenen Aryl- oder Heterocyclylresten
ein Reagens der allgemeinen Formel:

$R°-C(NR_3R_4)_3$

in welcher R°, $R_3$ und $R_4$ die obige Bedeutung haben, mit einem Cephalosporinderivat der allgemeinen Formel:

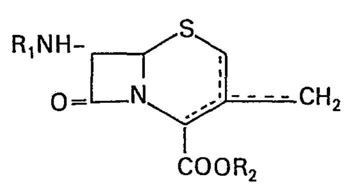

das in 3-Methyl-2- oder -3-bicycloocten-Form oder 3-Methylen-bicyclooctan-Form vorliegt und worin $R_1$ und $R_2$ die obige Bedeutung haben, umsetzt oder zur Herstellung einer Verbindung, in welcher R° entweder ein Rest der zweiten oben unter a) angegebenen Gruppe ist oder ausgewählt ist aus den unter b) angegebenen Alkyl-, Cycloalkyl-, Cycloalkylalkyl-, Arylalkyl- oder Heterocyclylalkylresten, ein Reagens der allgemeinen Formel:

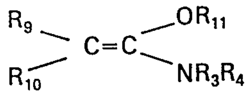

(in welcher $R_9$ und $R_{10}$ derart sind, dass $R_9R_{10}CH$- den oben angegebenen Rest R° darstellt, $R_3$ und $R_4$ die obige Bedeutung haben und $R_{11}$ ein Alkylrest mit 1 bis 5 Kohlenstoffatomen ist) mit einem Cephalosporinderivat der allgemeinen Formel:

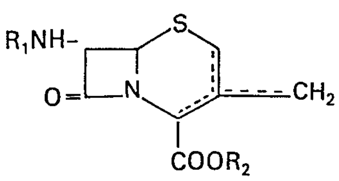

wie oben definiert, umsetzt.

**Patentansprüche**
für die Vertragsstaaten: BE, CH, DE, FR, GB, IT, LI, LU, NL, SE

1. Ein Derivat des Cephalosporins, dadurch gekennzeichnet, dass es der allgemeinen Formel

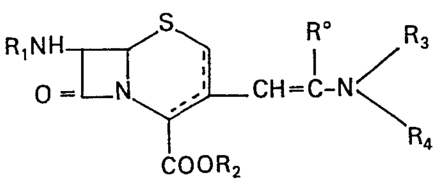

entspricht, welche in Bicycloocten-2- oder -3-Form vorliegt, worin die Symbole $R_3$ und $R_4$, welche identisch oder verschieden sind, Alkylreste (gegebenenfalls substituiert durch einen Alkyloxy- oder Dialkylaminorest) oder Phenyl bedeuten, oder zusammen mit dem Stickstoffatom, an das sie gebunden sind, einen gesättigten Heterocyclus mit 5 oder 6 Kettengliedern bilden, der gegebenenfalls ein anderes Heteroatom ausgewählt unter Stickstoff, Sauerstoff oder Schwefel enthält und gegebenenfalls durch einen Alkylrest substituiert ist,
a) das Symbol $R_1$ einen Rest der allgemeinen Formel

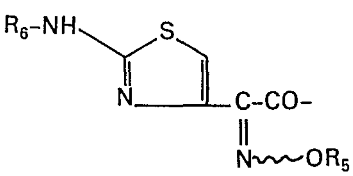

bedeutet [(worin $R_5$ ein Wasserstoffatom, ein Alkyl-, Vinyl-, Cyanomethylrest, eine Oximschutzgruppe oder ein Rest der allgemeinen Formel

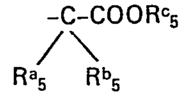

ist (worin $R^a_5$ und $R^b_5$, welche identisch oder verschieden sind, Wasserstoffatome oder Alkylreste sind, oder zusammen einen Alkylenrest mit zwei oder drei Kohlenstoffatomen bilden und $R^c_5$ ein Säureschutzrest ist) und $R_6$ ein Aminoschutzrest ist)],
einen Benzhydryl-, Tritylrest,
einen Acylrest der allgemeinen Formel

$R_7CO-$

[(worin $R_7$ ein Alkylrest substituiert durch einen Phenyl- oder Phenoxyrest ist)],
einen Rest der allgemeinen Formel

$R_8OCO-$

bedeutet [(worin $R_8$ ein nicht substituierter verzweigter Alkylrest oder ein gerader oder verzweigter Alkylrest mit einem oder mehreren Substituenten [ausgewählt unter Phenyl und substituierten Phenyl (durch einen Alkyloxy-, Nitro- oder Phenylrest)] oder Vinyl ist)],
einen Rest der allgemeinen Formel

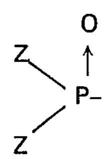

bedeutet, worin die Symbole Z Phenylreste oder -OZ' bedeuten, wobei Z' einen Alkyl-, 2,2,2-Trichlorethyl-, Phenyl- oder Benzylrest bedeutet (wobei diese beiden letzteren Reste durch einen Alkyl-, Alkyloxy- oder Nitrorest substituiert sein können) oder auch die Symbole Z' der beiden Substituenten Z zusammen einen Alkylenrest mit 2 oder 3 Kohlenstoffatomen bilden, oder auch $R_1NH$ durch einen Methylenaminorest ersetzt ist, worin der Methylenrest durch eine Dialkylamino- oder Arylgruppe (die gegebenenfalls ihrerseits durch einen oder mehrere Methoxy- oder Nitroreste substituiert ist) substituiert ist,
- das Symbol $R_2$ einen Säureschutzrest bedeutet und
- das Symbol R° einen Rest einer ersten Gruppe bestehend aus einem gegebenenfalls substituierten Phenylrest [durch einen Alkyl-, Trifluormethyl-, Dialkylaminomethyl-, Alkyloxy-, Alkylthio-, Dialkylaminorest oder durch ein Halogenatom ausgewählt unter Fluor oder Chlor], einen heterocyclischen Rest mit 5 oder 6 Kettengliedern enthaltend ein einziges Heteroatom ausgewählt unter Pyridyl-2 oder -3, Thienyl-2 oder -3 oder Furyl-2 oder -3 und gegebenenfalls substituiert durch einen Alkyl-, Alkyloxy- oder Dimethylaminomethylrest, oder auch einen Rest einer zweiten Gruppe bestehend aus einem Alkylrest mit 1 bis 5 Kohlenstoffatomen, gegebenenfalls substituiertem Benzyl (durch ein Halogenatom oder einen Alkyl-, Alkyloxy-, Alkylthio-, Dialkylamino- oder Trifluormethylrest), einen Methylrest substituiert durch einen aromatischen Heterocyclus mit 5 oder 6 Kettengliedern ausgewählt unter Pyridyl-2 oder -3, Thienyl-2 oder -3 oder Furyl-2 oder -3, einen Cycloalkylrest mit 3 bis 6 Kettengliedern oder einen Rest $-CHR_9R_{10}$, worin $R_9$ und $R_{10}$ mit dem Kohlenstoffatom, an das sie gebunden sind, einen Heterocyclus mit 5 oder 6 Kettengliedern enthaltend ein Sauerstoff- oder Schwefelatom bilden, bedeutet oder

b) das Symbol $R_1$ bedeutet einen Trityl-, Silylrest, einen Alkyloxycarbonylrest, dessen Alkylteil 1 bis 5 Kohlenstoffatome enthält, gegebenenfalls substituiert durch einen oder mehrere Phenyl- oder Furylreste,
einen 2-(Biphenylyl-4)-propyloxycarbonylrest oder einen Rest der allgemeinen Formel

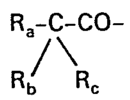

worin $R_a$ ein Phenyl-, Thienyl-, Furyl-, Cyclohexadienyl- oder Cyclohexenylrest, die gegebenenfalls substituiert sind, ist, $R_b$ ein Wasserstoffatom und $R_c$ ein Wasserstoffatom, ein gegebenenfalls geschützter Hydroxyrest, ein geschützter Aminorest oder ein gegebenenfalls geschützter Sulforest ist, oder $R_a$ ein gegebenenfalls substituierter Phenoxyrest oder Pyridylthiorest ist und $R_b$ und $R_c$ Wasserstoffatome sind, oder auch $R_a$ ein Phenyl-, Thienyl-, Furylrest ist und $R_b$ und $R_c$ zusammen einen Alkyloxyimino-, Cycloalkyloxyimino- oder Phenylalkyloxyiminorest der syn-Form bilden,

- das Symbol $R_2$ einen Säureschutzrest bedeutet und
- das Symbol R° bedeutet

einen Alkylrest (mit 1 bis 7 Kohlenstoffatomen),
einen Cycloalkylrest (mit 3 bis 7 Kohlenstoffatomen),
einen Cycloalkylalkylrest (mit 4 bis 7 Kohlenstoffatomen),
einen mono- oder bicyclischen Arylrest,
einen Arylalkylrest,
einen Heterocyclyl- oder Heterocyclylalkylrest mit 5 oder 6 Kettengliedern, enthaltend 1 bis 4 Stickstoffatome und/oder ein Sauerstoff- oder Schwefelatom, wobei diese Reste R° gegebenenfalls durch Alkyl- oder Alkyloxyreste substituiert sein können, wobei die Alkyl- oder Acylreste oder -teile gerade oder verzweigt sind und 1 bis 4 Kohlenstoffatome enthalten, wenn nichts anderes angegeben ist.

2. Ein Derivat des Cephalosporins gemäss Anspruch 1, wie in a) definiert, dadurch gekennzeichnet, dass $R_1$ einen Rest der allgemeinen Formel

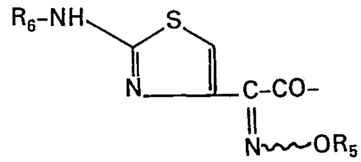

bedeutet, worin $R_5$ ein Wasserstoffatom, ein Alkyl-, Vinyl-, Cyanomethyl-, Trityl-, Tetrahydropyranyl- oder 2-Methoxypropyl-2-Rest ist, oder ein Rest der allgemeinen Formel

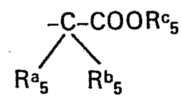

worin $R^a_5$ und $R^b_5$ wie in Anspruch 1 definiert sind und $R^c_5$ ein Methoxymethyl-, tert.-Butyl-, Benzhydryl-, Benzyl-, Nitrobenzyl- oder p-Methoxybenzylrest ist.

3. Ein Derivat des Cephalosporins gemäss Anspruch 1, wie in a) definiert, dadurch gekennzeichnet, dass $R_1$ einen Rest

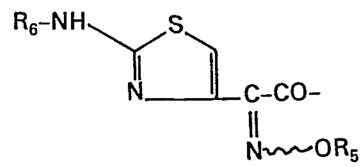

bedeutet, worin $R_6$ ausgewählt ist unter tert.-Butoxycarbonyl, Trityl, Benzyl, Dibenzyl, Benzyloxycarbonyl, p-Nitrobenzyloxycarbonyl, p-Methoxybenzyloxycarbonyl oder einem Rest $Z_2P(O)$- wie in Anspruch 1 definiert.

4. Ein Derivat des Cephalosporins gemäss Anspruch 1, wie in a) definiert, dadurch gekennzeichnet, dass $R_2$ ausgewählt ist unter Methoxymethyl, tert.-Butyl, Benzhydryl, Benzyl, Nitrobenzyl oder p-Methoxybenzyl.

5. Ein Derivat des Cephalosporins gemäss Anspruch 1, wie in b) definiert, dadurch gekennzeichnet, dass $R_2$ eine Arylgruppe ist, polysubstituiert durch aliphatische oder aromatische Reste, eine aromatische heterocyclische Gruppe enthaltend Sauerstoff oder Schwefel ist, eine tert.-Alkylgruppe, Diphenylmethyl gegebenenfalls substituiert, Alkyloxyphenylalkyl, Dimethoxybenzyl, Nitrobenzyl, 4,5-Dimethoxy-2-nitrobenzyl, Furfuryl oder eine 2-Oxa- (oder 2-Thia-) Cycloalkylgruppe, 2-Oxa- (oder 2-Thia-) Cycloalenylgruppe enthaltend 5 bis 7 Kettenglieder oder eine Nitrophenyl- oder 2,4-Dinitrophenylgruppe oder Silyl substituiert durch Alkyloxy-, Cycloalkyl-, Phenyl- oder Phenylalkylreste, ist.

6. Ein Derivat des Cephalosporins gemäss Anspruch 1, wie in b) definiert, dadurch gekennzeichnet, dass $R_1$ einen Rest der allgemeinen Formel

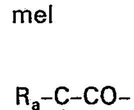

bedeutet, worin $R_a$, $R_b$ und $R_c$ wie in Apruch 1 definiert sind, wobei die Phenyl- oder Phenoxyreste, welche $R_a$ bedeuten, durch Hydroxy-, Alkyloxy-, Axyloxy-, Benzoyloxy-, 4-Carbamoyloxy-, geschützte Amino- oder Alkylaminogruppen, Dialkylamino-, Alkylsulfonylamino-, Aminomethyl-, tert-Butoxycarbonylaminomethyl-gruppen oder durch ein Halogenatom substituiert sein können, die Thienyl-, Furyl-, Cyclohexadienyl- und Cyclohexenylreste, welche $R_a$ bedeuten, in 5-Stellung an den Thienyl- und Furyl-resten und in 2- oder 3-Stellung an den Cyclohexadienyl- und Cyclohexenylresten durch geschützte Aminomethylgruppen substituiert sein können.

7. Ein Derivat des Cephalosporins gemäss Anspruch 1, dadurch gekennzeichnet, dass das Symbol $R_1$ ein Tritylrest, ein Rest der allgemeinen Formel

$R_7CO-$

worin $R_7$ wie in Anspruch 1 definiert ist, ein Rest der allgemeinen Formel

$R_8OCO-$

worin $R_8$ wie in Anspruch 1 definiert ist, ein Rest der allgemeinen Formel

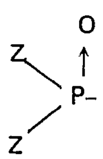

ist, worin Z wie in Anspruch 1 definiert ist, das Symbol $R_2$ eine Schutzgruppe wie in a) in Anspruch 1 definiert bedeutet, oder auch das Symbol $R_1$ einen Rest der allgemeinen Formel

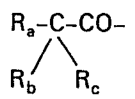

bedeutet, worin $R_a$, $R_b$ und $R_c$ wie in Anspruch 1 definiert sind, das Symbol $R_2$ eine Schutzgruppe wie in b) in Anspruch 1 definiert ist, das Symbol R° ein Alkyl- oder Phenylrest ist und die Symbole $R_3$ und $R_4$ Alkylreste bedeuten.

8. Ein Derivat des Cephalosporins gemäss Anspruch 1, dadurch gekennzeichnet, dass das Symbol $R_1$ einen Tritylrest, einen Phenoxyacetylrest, einen Rest der allgemeinen Formel $R_8OCO-$, worin $R_8$ ein verzweigter Alkylrest ist, einen Rest der allgemeinen Formel $(Z)_2P(O)-$, worin Z ein Alkyloxyrest ist, bedeutet oder auch einen α-Amino-α-phenylacetamidorest, dessen Aminfunktion geschützt ist, das Symbol $R_2$ ein Benzhydryl- oder Nitrobenzylrest ist, das Symbol R° ein Methyl- oder Phenylrest ist und die Symbole $R_3$ und $R_4$ Methylreste sind.

9. Verfahren zur Herstellung eines Produkts gemäss einem der Ansprüche 1 bis 8, worin $R_1$, $R_2$, $R_3$ und $R_4$ wie in den Ansprüchen 1 bis 5 definiert sind und R° entweder ein Rest der ersten Gruppe definiert in a) in Anspruch 1 oder ausgewählt unter den Aryl- oder Heterocyclylresten definiert in b) in Anspruch 1 ist, dadurch gekennzeichnet, dass man einen Reaktanten der allgemeinen Formel

$R°-C(NR_3R_4)_3$

worin R°, $R_3$ und $R_4$ wie vorstehend definiert sind, auf ein Derivat des Cephalosporins der allgemeinen Formel

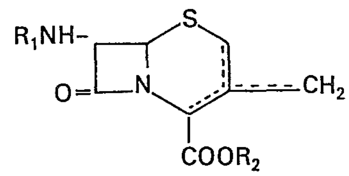

das in 3-Methylbicycloocten-2- oder -3-Form oder 3-Methylenbicyclooctan-Form vorliegt und worin $R_1$ und $R_2$ gemäss einem der Ansprüche 1 bis 5 definiert sind, einwirken lässt.

10. Verfahren zur Herstellung eines Produkts gemäss einem der Ansprüche 1 bis 8, worin $R_1$, $R_2$ $R_3$ und $R_4$ wie in den Ansprüchen 1 bis 5 definiert sind und R° entweder ein Rest der zweiten Gruppe definiert vorstehend in a) in Anspruch 1 oder ausgewählt unter den Alkyl-, Cycloalkyl-, Cycloalkylalkyl-, Arylalkyl- oder Heterocyclylalkylresten definiert in b) in Anspruch 1 ist, dadurch gekennzeichnet, dass man einem Reaktanten der allgemeinen Formel

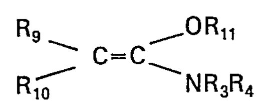

(worin $R_9$ und $R_{10}$ derart sind, dass $R_9R_{10}CH-$ den vorstehend definierten Rest R° bedeutet, $R_3$ und $R_4$ wie in Anspruch 1 definiert sind und $R_{11}$ ein Alkylrest mit 1 bis 5 Kohlenstoffatomen ist) auf ein Derivat des Cephalosporins der allgemeinen Formel

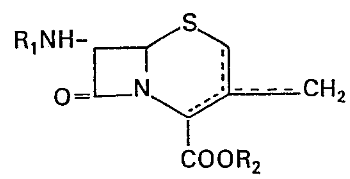

definiert wie in Anspruch 9, einwirken lässt.

**Claims**

for the contracting States BE CH DE FR GB IT LI LU NL SE

1. A derivative of cephalosporin, characterised in that it corresponds to the general formula:

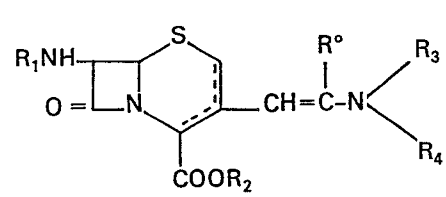

which takes the form of a 2- or 3-bicyclooctene in which the symbols $R_3$ and $R_4$, which are identical or different, denote alkyl radicals (optionally substituted with an alkoxy or dialkylamino radical) or phenyl radicals or form together with the nitrogen atom to which they are attached a saturated 5- or 6-membered heterocycle optionally containing another heteroatom chosen from nitrogen, oxygen or sulphur, and optionally substituted with an alkyl radical,
a) the symbol $R_1$, denotes a radical of general formula:

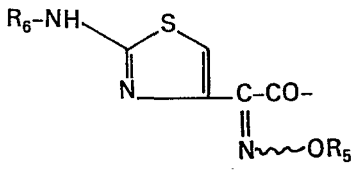

[in which $R_5$ is a hydrogen atom, an alkyl, vinyl or cyanomethyl radical, an oxime-protecting group or a radical of general formula:

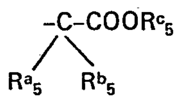

(in which $R^a_5$ and $R^b_5$ which are identical or different are hydrogen atoms or alkyl radicals, or together form an alkylene radical containing two or three carbon atoms, and $R^c_5$ is an acid-protecting radical) and $R_6$ is an amino-protecting radical],
a benzhydryl, trityl radical,
an acyl radical of general formula:

$R_7CO-$

[in which $R_7$ is an alkyl radical substituted with a phenyl or phenoxy radical],
a radical of general formula:

$R_8OCO-$

[in which $R_8$ is an unsubstituted branched alkyl radical or a linear or branched alkyl radical bearing one or more substituents (chosen from unsubstituted phenyl and phenyl substituted with an alkoxy, nitro or phenyl radical), or a vinyl radical],
a radical of general formula:

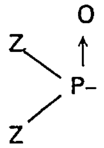

in which the symbols Z denote phenyl or $-OZ'$ radicals in which Z' denotes an alkyl, 2,2,2-trichloroethyl, phenyl or benzyl radical (where the latter two radicals can be substituted with an alkyl, alkoxy or nitro radical), or alternatively symbols Z' of the two substituents Z together form an alkylene radical containing 2 or 3 carbon atoms or alternatively $R_1NH$ is replaced by a methyleneamino radical in which the methylene radical is substituted with a dialkylamino or arylgroup (which can itself be optionally substituted with one or more methoxy or nitro radicals),
- the symbol $R_2$ denotes an acid-protecting radical and
- the symbol R° denotes a radical of a first group consisting of a phenyl radical which is optionally substituted (with an alkyl, trifluoromethyl, dialkylaminomethyl, alkoxy, alkylthio or dialkylamino radical or with a halogen atom chosen from fluorine and chlorine), a 5- or 6-membered heterocyclic radical containing one single heteroatom and chosen from 2- or 3- pyridyl, 2- or 3- thienyl, or 2- or 3-furyl and optionally substituted with an alkyl, alkoxy or dimethylaminomethyl radical or alternatively a radical of a second group consisting of an alkyl radical containing 1 to 5 carbon atoms, a benzyl radical which is optionally substituted (with a halogen atom or an alkyl, alkoxy, alkylthio, dialkylamino or trifluoromethyl radical), a methyl radical substituted with a 5- or 6-membered aromatic heterocycle chosen from 2- or 3-pyridyl, 2- or 3-thienyl or 2- or 3-furyl, a 3- to 6-membered cycloalkyl radical, or a $-CHR_9R_{10}$ radical in which $R_9$ and $R_{10}$ form, with the carbon atom to which they are attached, a 5- or 6-membered heterocycle containing an oxygen or a sulphur atom, or alternatively
b) the symbol $R_1$ denotes a trityl or silyl radical, or an alkoxycarbonyl radical in which the alkyl part contains 1 to 5 carbon atoms, the radical being optionally substituted with one or more phenyl or furyl radicals,
a 2-(4-biphenylyl)propyloxycarbonyl radical, or
a radical of general formula:

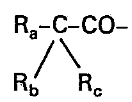

in which
$R_a$ is a phenyl, thienyl, furyl, cyclohexadienyl or cyclohexenyl radical, optionally substituted, $R_b$ is a hydrogen atom and $R_c$ a hydrogen atom, an optionally protected hydroxy radical, a protected amino radical or an optionally protected sulpho radical, or $R_a$ is an optionally substituted phenoxy radical or pyridylthio, and $R_b$ and $R_c$ are hydrogen atoms, or alternatively $R_a$ is a phenyl, thienyl or furyl radical and $R_b$ and $R_c$ together form an alkoxyimino, cycloalkoxyimino or phenylalcoxyimino radical in the syn form
- the symbol $R_2$ denotes an acid-protecting radical, and
- the symbol R° denotes
an alkyl radical (containing 1 to 7 carbon atoms),
a cycloalkyl radical (containing 3 to 7 carbon atoms),
a (cycloalkyl)alkyl radical (containing 4 to 7 carbon atoms),
a mono- or bicyclic aryl radical,
an arylalkyl radical,

a 5- or 6-membered heterocyclic or heterocycle-substituted alkyl radical containing 1 to 4 nitrogen atoms and or an oxygen or a sulphur atom, where these R° radicals can optionally be substituted with alkyl or alkoxy radicals, given that (except where specifically stated) the alkyl or acyl portions or radicals are linear or branched and contain 1 to 4 carbon atoms.

2. A derivative of cephalosporin according to Claim 1 as defined in a), characterised in that $R_1$ denotes a radical of general formula:

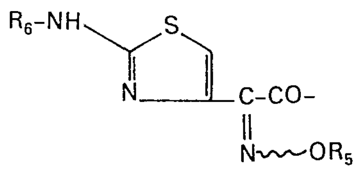

in which $R_5$ is a hydrogen atom or an alkyl, vinyl, cyanomethyl, trityl, tetrahydropyranyl or 2-methoxy-2-propyl radical, or a radical of general formula:

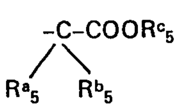

in which $R^a_5$ and $R^b_5$ are defined as in Claim 1, and $R^c_5$ is a methoxymethyl, tert-butyl, benzhydryl, benzyl, nitrobenzyl, or p-methoxybenzyl radical.

3. A derivative of cephalosporin according to Claim 1 as defined in a), characterised in that $R_1$ denotes a radical

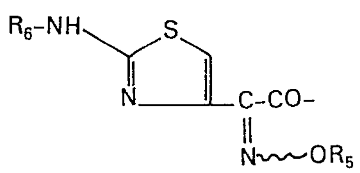

in which $R_6$ is chosen from tert-butoxycarbonyl, trityl, benzyl, dibenzyl, benzyloxycarbonyl, p-nitrobenzyloxycarbonyl, p-methoxybenzyloxycarbonyl or a radical $Z_2P(O)$- as defined in Claim 1.

4. A derivative of cephalosporin according to Claim 1 as defined in a), characterised in that $R_2$ is chosen from methoxymethyl, tert-butyl, benzhydryl, benzyl, nitrobenzyl or p-methoxybenzyl.

5. A derivative of cephalosporin according to Claim 1 as defined in b), characterised in that $R_2$ is an aryl group polysubstituted with aliphatic or aromatic radicals, an aromatic heterocyclic group containing oxygen or sulphur, a tert-alkyl group, an optionally substituted diphenylmethyl group, an alkoxyphenylalkyl, dimethoxybenzyl, nitrobenzyl, 4,5-dimethoxy-2-nitrobenzyl or furfuryl group, a 5- to 7-membered 2-oxa- (or 2-thia-) cycloalkyl or 2-oxa- (or 2-thia-) cycloalkenyl group, a nitrophenyl or 2,4-dinitrophenyl group, or a silyl group substituted with alkoxy, cycloalkyl, phenyl or phenylalkyl radicals.

6. A derivative of cephalosporin according to Claim 1 defined as in b), characterised in that $R_1$ denotes a radical of general formula:

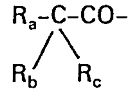

in which ,with $R_a$, $R_b$ and $R_c$ defined according to Claim 1, the phenyl and phenoxy radicals represented by $R_a$ can be substituted with hydroxy, alkoxy, acyloxy, benzoyloxy, or 4-carbamoyloxy groups, protected amino or alkylamino groups, dialkylamino, alkylsulphonylamino, aminomethyl, or tert-butoxycarbonylaminomethyl groups, or with a halogen atom, the thienyl, furyl, cyclohexadienyl and cyclohexenyl radicals represented by $R_a$ can be substituted with protected aminomethyl radicals in the 5-position of the thienyl and furyl radicals and in the 2- or 3-position of the cyclohexadienyl and cyclohexenyl radicals.

7. A derivative of cephalosporin according to Claim 1, characterised in that the symbol $R_1$ is a trityl radical, a radical of general formula:

$R_7CO-$

in which $R_7$ is defined as in Claim 1,
a radical of general formula

$R_8OCO-$

in which $R_8$ is defined as in Claim 1,
a radical of general formula

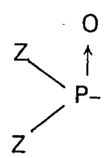

in which Z is defined as in Claim 1 the symbol $R_2$ denotes a protective radical such as defined in a) in Claim 1, or alternatively the symbol $R_1$ denotes a radical of general formula

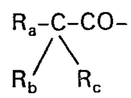

in which $R_a$, $R_b$ and $R_c$ are defined as in Claim 1, the symbol $R_2$ is a protective radical such as defined in b) in Claim 1, the symbol R° is an alkyl or phenyl radical and the symbols $R_3$ and $R_4$ denote alkyl radicals.

8. A derivative of cephalosporin according to Claim 1, characterised in that the symbol $R_1$ denotes a trityl radical, a phenoxyacetyl radical, a radical of general formula $R_8OCO-$ in which $R_8$ is a branched alkyl radical, a radical of general formula $(Z)_2P(O)-$ in which Z is an alkoxy radical, or alternatively an α-amino-α-phenylacetamido radical in which the amino function is protected, the symbol $R_2$ is a benzhydryl or nitrobenzyl radical, the symbol R° is a methyl or phenyl radical and the symbols $R_3$ and $R_4$ are methyl radicals.

9. A process for the preparation of a product according to one of Claims 1 to 8 for which $R_1$, $R_2$, $R_3$ and $R_4$ are defined as in Claims 1 to 5 and R° is either a radical of the first group defined in a) in Claim 1, or chosen from the aryl or heterocyclic radicals defined in b) in Claim 1, characterised in that
a reagent of general formula:

$R°-C(NR_3R_4)_3$

in which R°, $R_3$ and $R_4$ are defined as above, is reacted with a cephalosporin derivative of general formula:

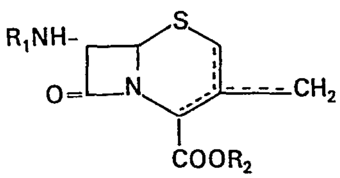

which takes the form of 3-methyl-2-bicyclooctene or 3-methyl-3-bicyclooctene or 3-methylene-bicyclooctane and in which $R_1$ and $R_2$ are defined according to one of Claims 1 to 5.

10. A process for the preparation of a product according to one of Claims 1 to 8 for which $R_1$, $R_2$, $R_3$ and $R_4$ are defined as in Claims 1 to 5 and R° is either a radical of the second group defined above in a) in Claim 1, or chosen from the alkyl, cycloalkyl (cycloalkyl)alkyl, arylalkyl or heterocycle-substituted alkyl radicals defined in b) in Claim 1, characterised in that a reagent of general formula:

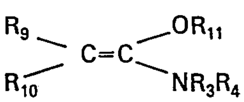

(in which $R_9$ and $R_{10}$ are such that $R_9R_{10}CH-$ denotes the radical R° defined above, $R_3$ and $R_4$ are defined as in Claim 1, and $R_{11}$ is an alkyl radical containing 1 to 5 carbon atoms) is reacted with a cephalosporin derivative of general formula:

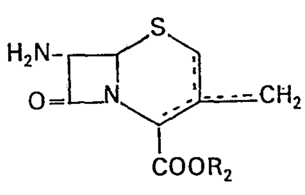

defined as in Claim 9.

**Claim**
for the contracting State AT

Process for the preparation of a derivative of cephalosporin of general formula:

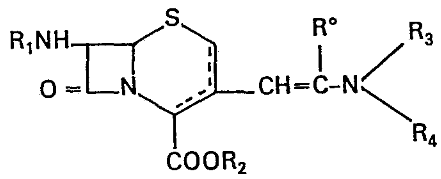

which takes the form of a 2- or 3-bicyclooctene in which the symbols $R_3$ and $R_4$, which are identical or different, denote alkyl radicals (optionally substituted with an alkoxy or dialkylamino radical) or phenyl radicals or form together with the nitrogen atom to which they are attached a saturated 5- or 6-membered heterocycle optionally containing another heteroatom chosen from nitrogen, oxygen or sulphur, and optionally substituted with an alkyl radical, and
a) the symbol $R_1$, denotes a radical of general formula:

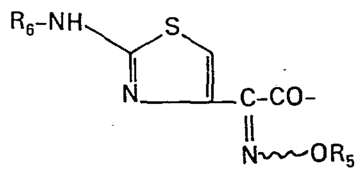

[in which $R_5$ is a hydrogen atom, an alkyl, vinyl or cyanomethyl radical, an oxime-protecting group or a radical of general formula:

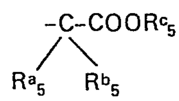

(in which $R^a_5$ and $R^b_5$ which are identical or different are hydrogen atoms or alkyl radicals, or together form an alkylene radical containing two or three carbon atoms, and $R^c_5$ is an acid-protecting radical) and $R_6$ is an amino-protecting radical],
a benzhydryl, trityl radical,
an acyl radical of general formula:

$R_7CO-$

[in which $R_7$ is an alkyl radical substituted with a phenyl or phenoxy radical],
a radical of general formula:

$R_8OCO-$

[in which $R_8$ is an unsubstituted branched alkyl radical or a linear or branched alkyl radical bearing one or more substituents (chosen from unsubstituted phenyl and phenyl substituted with an alkoxy, nitro or phenyl radical), or a vinyl radical],
a radical of general formula:

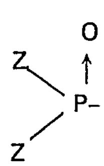

in which the symbols Z denote phenyl or -OZ′ radicals in which Z′ denotes an alkyl, 2,2,2-trichloroethyl, phenyl or benzyl radical (where the latter two radicals can be substituted with an alkyl, alkoxy or nitro radical), or alternatively symbols Z′ of the two substituants Z together form an alkylene radical containing 2 or 3 carbon atoms or alternatively $R_1NH$ is replaced by a methyleneamino radical in which the methylene radical is substituted with a dialkylamino or aryl group (which can itself be optionally substituted with one or more methoxy or nitro radicals),

- the symbol $R_2$ denotes an acid-protecting radical and
- the symbol R° denotes a radical of a first group consisting of a phenyl radical which is optionally substituted (with an alkyl, trifluoromethyl, dialkylaminomethyl, alkoxy, alkylthio or dialkylamino radical or with a halogen atom chosen from fluorine and chlorine), a 5- or 6-membered heterocyclic radical containing one single heteroatom and chosen from 2- or 3-pyridyl, 2- or 3-thienyl, or 2- or 3-furyl and optionally substituted with an alkyl, alkoxy or dimethylaminomethyl radical or alternatively a radical of a second group consisting of an alkyl radical containing 1 to 5 carbon atoms, a benzyl radical which is optionally substitued (with a halogen atom or an alkyl, alkoxy, alkylthio, dialkylamino or trifluoromethyl radical), a methyl radical substituted with a 5- or 6-membered aromatic heterocycle chosen from 2- or 3- pyridyl, 2- or 3-thienyl or 2- or 3-furyl, a 3- to 6-membered cycloalkyl radical, or a $-CHR_9R_{10}$ radical in which $R_9$ and $R_{10}$ form, with the carbon atom to which they are attached, a 5- or 6-membered heterocycle containing an oxygen or a sulphur atom, or alternatively

b) the symbol $R_1$ denotes a trityl or silyl radical, or an alkoxycarbonyl radical in which the alkyl part contains 1 to 5 carbon atoms, the radical being obtionally substituted with one or more phenyl or furyl radicals, a 2-(4-biphenylyl)-propyloxycarbonyl radical, or
a radical of general formula:

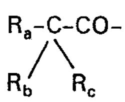

in which
$R_a$ is a phenyl, thienyl, furyl, cyclohexadienyl or cyclohexenyl radical, optionally substituted, $R_b$ is a hydrogen atom and $R_c$ a hydrogen atom, an optionally protected hydroxy radical, a protected amino radical or an optionally protected sulpho radical, or $R_a$ is an optionally subsituted phenoxy radical or pyridylthio, and $R_b$ and $R_c$ are hydrogen atoms, or alternatively $R_a$ is a phenyl, thienyl or furyl radical and $R_b$ and $R_c$ togehter form an alkoxyimino, cycloalkoxyimino or phenylalcoxyimino radical in the syn form

- the symbol $R_2$ denotes an acid-protecting radical, and
- the symbol R° denotes
an alkyl radical (containing 1 to 7 carbon atoms),
a cycloalkyl radical (containing 3 to 7 carbon atoms),
a (cycloalkyl)alkyl radical (containing 4 to 7 carbon atoms),
a mono- or bicyclic aryl radical,
an arylalkyl radical,
a 5- or 6-membered heterocyclic or heterocycle-substituted alkyl radical containing 1 to 4 nitrogen atoms and or an oxygen or a sulphur atom, where these R° radicals can optionally be substituted with alkyl or alkoxy radicals, given that (except where specifically stated) the alkyl or acyl portions or radicals are linear or branched and contain 1 to 4 carbon atoms, characterised in that for the preparation of a product for which R° is either a radical of the first group defined in a), or chosen from the aryl or heterocyclic radicals defined in b) a reagent of general formula:

$R^o-C(NR_3R_4)_3$

in which R°, $R_3$ and $R_4$ are defined as above, is reacted with a cephalosporin derivative of general formula:

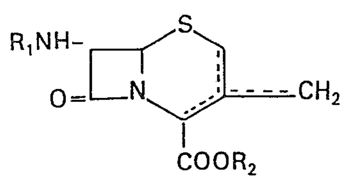

which takes the form of 3-methyl-2-bicyclooctene or 3-methyl-3-bicyclooctene or 3-methylene-bicyclooctane and in which $R_1$ and $R_2$ are defined as above or, for the preparation of a product for which R° is either a radical of the second group defined above in a), or chosen from the alkyl, cycloalkyl, (cycloalkyl)alkyl, arylalkyl or heterocycle-substitued alkyl radicals defined in b), a reagent of general formula:

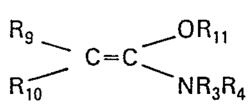

(in which $R_9$ and $R_{10}$ are such that $R_9R_{10}CH-$ denotes the radical R° defined above, $R_3$ and $R_4$ are defined as above, and $R_{11}$ is an alkyl radical containing 1 to 5 carbon atoms) is reacted with a cephalosporin derivative of general formula:

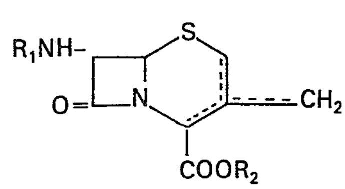

defined as above.